# EUROPÄISCHE PATENTANMELDUNG

(11) **EP 2 719 680 A1**
(43) Veröffentlichungstag der Anmeldung: **16.04.2014**
(21) Anmeldenummer: 13195703.7
(22) Anmeldetag: 28.10.2011
(51) Int. Cl.: C07C 69/157

(54) **Phenylsubstituierte Bicyclooktan-1,3-dion-Derivate**

(30) Priorität: 02.11.2010 US 409185 P; 02.11.2010 EP 10189670
(62) Teilanmeldung aus: 11776193.2
(71) Anmelder: Bayer Intellectual Property GmbH, 40789 Monheim (DE)
(72) Erfinder: Angermann, Alfred, Dr., 65830 Kriftel (DE); Lehr, Stefan, Dr., 69006 Lyon (FR); Bojack, Guido, Dr., 65207 Wiesbaden-Naurod (DE); Fischer, Reiner, Dr., 40789 Monheim (DE); Häuser-Hahn, Isolde, Dr., 51375 Leverkusen (DE); Heinemann, Ines, Dr., 65719 Hofheim (DE); Gatzweiler, Elmar, Dr., 61231 Bad Nauheim (DE); Rosinger, Christopher, Dr., 65719 Hofheim (DE); Voerste, Arnd, Dr., 50674 Köln (DE); Feucht, Dieter, Dr., 65760 Eschborn (DE)
(74) Vertreter: BIP Patents

(57) **Zusammenfassung**

Die Erfindung betrifft neue Verbindungen der Formel (I), 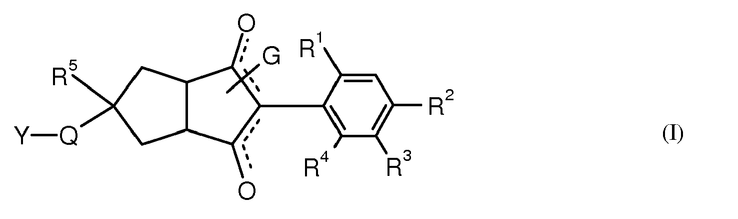 in welcher R¹, R², R³, R⁴, R⁵, Y, Q und G die oben angegebenen Bedeutungen haben,
mehrere Verfahren und Zwischenprodukte zu ihrer Herstellung und ihre Verwendung als Herbizide und/oder Schädlingsbekämpfungsmittel.

Außerdem betrifft die Erfindung selektiv herbizide Mittel, die phenylsubstituierte Bicyclooktan-1,3-dion-Derivate einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere phenylsubstituierte Bicyclooktan-1,3-dion-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Schädlingsbekämpfungsmittel und/oder zur Verhinderung von unerwünschtem Pflanzenwuchs.

## Beschreibung

Die vorliegende Erfindung betrifft neue phenylsubstituierte Bicyclooktan-1,3-dion-Derivate, mehrere Verfahren zu ihrer Herstellung und ihre Verwendung als Herbizide und/oder Schädlingsbekämpfungsmittel.

Die Erfindung betrifft außerdem neue selektiv-herbizide Wirkstoffkombinationen, die phenylsubstituierte Bicyclooktan-1,3-dion-Derivate einerseits und zumindest eine die Kulturpflanzenverträglichkeit verbessernde Verbindung andererseits enthalten und mit besonders gutem Erfolg zur selektiven Unkrautbekämpfung in verschiedenen Nutzpflanzenkulturen verwendet werden können.

Die vorliegende Erfindung betrifft weiterhin die Steigerung der Wirkung von Pflanzenschutzmitteln enthaltend insbesondere phenylsubstituierte Bicyclooktan-1,3-dion-Derivate, durch die Zugabe von Ammonium- oder Phosphoniumsalzen und gegebenenfalls Penetrationsförderern, die entsprechenden Mittel, Verfahren zur ihrer Herstellung und ihre Anwendung im Pflanzenschutz als Schädlingsbekämpfungsmittel und/oder zur Verhinderung von unerwünschtem Pflanzenwuchs.

Es ist bekannt, dass bestimmte substituierte 2-Arylcyclopentandione herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO99/48869, WO 99/55673, WO 01/17972, WO 01/74770, WO 03/062244, WO 04/080962, WO04/111042 WO05/092897, WO06/029799, WO07/080066, WO07/096058, WO 09/019005, WO 09/019015, WO 10/000773, WO 10/081894, WO 10/089210 und WO10/040460). Außerdem sind ähnlich substituierte Verbindungen bekannt: 3-Hydroxy-5,5-dimethyl-2-phenylcyclopent-2-en-1-on aus der Publikation Micklefield et. al, Tetrahedron, (1992), 7519-26 sowie der Naturstoff Involution (-)-cis-5-(3,4-Dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66 (1973), 584 und der Offenlegungsschrift DE-A 2 361 084 (US 4,091,006) bekannt, mit Angabe von herbiziden und akariziden Wirkungen.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 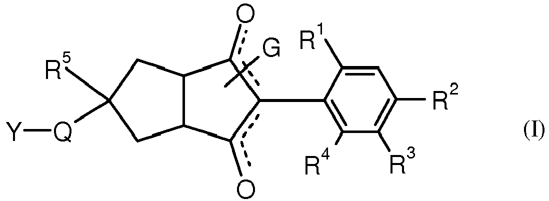 in welcher
wenn
- Q: für eine Bindung, eine C₁-C₃-Alkylen-, eine C₂-C₃-Alkenylen- oder eine C₂-C₃-Alkinylenkette steht,
dann stehen
- Y: für die Gruppen -OR⁶, -S(O)ₚR⁶, -CO₂R⁷, -CH=CH₂, Cyano, -SCN, -CONR⁸R⁹, -SO₂NR⁸R⁹,-CR¹⁰=O, -NR¹¹R¹², -CR¹⁰=N-OR¹³, -CR¹⁰=N-R¹⁴, CR¹⁰=N-NR¹⁵R¹⁶, -CR¹⁰(OR¹⁷OR¹⁸),-CR¹⁰(SR¹⁷OR¹⁸), -CR¹⁰(SR¹⁷SR¹⁸), -CR¹⁰(NHR¹⁷NHR¹⁸), -CR¹⁰(NHR¹⁷OR¹⁸), -CR¹⁰(NHR¹⁷SR¹⁸),-CH(CN)₂, -CH(OH)R⁶, Halogen, -O(C=M)R¹⁰, -S(C=M)R¹⁰, -O(C=M)NR¹¹R¹², -S(C=M)NR¹¹R¹², -NH(C=M)NR¹¹R¹², -O(C=M)OR⁷, -S(C=M)OR⁷, -NH(C=M)OR⁷ oder für die Gruppe W,
- oder Q, Y und R⁵: bilden gemeinsam eine der Gruppen CHCN=, CH(CO₂C₁-C₆-Alkyl)=, 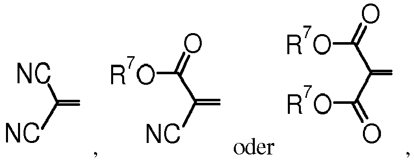
- W: für einen 3 bis 7-gliedrigen gesättigten oder teilgesättigten Heterocyclus, der mindestens ein Heteroatom wie Sauerstoff, Schwefel oder Stickstoff enthält und zusätzlich ein- oder mehrfach, gleich oder verschieden subsituiert sein kann,
- G: für Wasserstoff, Methyl, Ethyl oder Benzyl (a) oder für eine der Gruppen 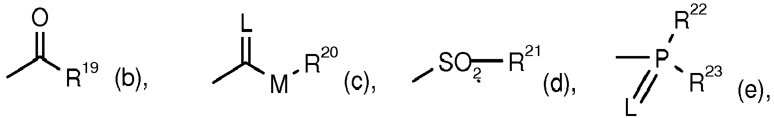 E (f) oder 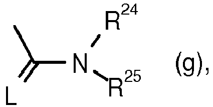
- E: für ein Metallionenäquivalent, ein tertiäres Sulfoniumion oder ein Ammoniumion,
- L: für Sauerstoff oder Schwefel,
- M: für Sauerstoff oder Schwefel,
- R¹: für Wasserstoff, Halogen, Cyano, Nitro, C₁-C₆-Alkyl, Halogen-C₁-C₆-alkyl, C₃-C₆-Cycloalkyl, Halogen-C₃-C₆-cycloalkyl, C₂-C₆-Alkenyl, Halogen-C₂-C₆-alkenyl, C₂-C₆-Alkinyl, Halogen-C₂-C₆-alkinyl, C₁-C₆-Alkoxy, Halogen-C₁-C₆-alkoxy, C₃-C₆-Cycloalkoxy, Halogen-C₃-C₆-cycloalkoxy, C₂-C₆-Alkinyloxy, Halogen-C₂-C₆-alkinyloxy, C₂-C₆-Alkenyloxy, Halogen-C₂-C₆-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl oder Halogen-C₁-C₃-alkylsulfonyl,
- R² und R³: unabhängig voneinander, gleich oder verschieden für Wasserstoff, Halogen, Cyano, Nitro, C₁-C₆-Alkyl, Halogen-C₁-C₆-alkyl, C₂-C₆-Alkenyl, Halogen-C₂-C₆-alkenyl, C₂-C₆-Alkinyl, Halogen-C₂-C₆-alkinyl, C₁-C₆-Alkoxy, Halogen-C₁-C₆-alkoxy, C₃-C₆-Cycloalkyl, C₃-C₆-Cycloalkoxy, Halogen-C₃-C₆-cycloalkoxy, C₂-C₆-Alkinyloxy, Halogen-C₂-C₆-alkinyloxy, C₂-C₆-Alkenyloxy, Halogen-C₂-C₆-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl, Halogen-C₁-C₃-alkylsulfonyl, Phenyl oder gegebenenfalls ein- oder mehrfach, gleich oder verschieden durch Halogen, C₁-C₄-Alkyl, Halogen-C₁-C₄-alkyl, C₃-C₆-Cycloalkyl, Halogen-C₃-C₆-cycloalkyl, C₁-C₆-Alkoxy, Halogen-C₁-C₆-alkoxy, Nitro oder Cyano substituiertes Phenyl,
- R⁴: für Wasserstoff, Halogen, Cyano, Nitro, C₁-C₆-Alkyl, Halogen-C₁-C₆-alkyl, C₃-C₆-Cycloalkyl, Halogen-C₃-C₆-cycloalkyl, C₁-C₆-Alkoxy, Halogen-C₁-C₆-alkoxy, C₃-C₆-Cycloalkoxy, oder Halogen-C₃-C₆-cycloalkoxy,
- R⁵: für Wasserstoff oder C₁-C₄-Alkyl,
- R⁶: für Wasserstoff, C₁-C₆-Alkyl, Halogen-C₁-C₆-alkyl, C₃-C₆-Cycloalkyl, Halogen-C₃-C₆-cycloalkyl, C₂-C₆-Alkenyl, Halogen-C₂-C₆-Alkenyl, C₂-C₆-Alkinyl, Halogen-C₂-C₆-Alkinyl, C₁-C₄-Alkoxy-C₁-C₄-alkyl, Halogen-C₁-C₄-Alkoxy-C₁-C₄-alkyl, C₁-C₆-Cycloalkoxy-C₁-C₄-alkyl, Halogen-C₁-C₄-Cycloalkoxy-C₁-C₄-alkyl, für jeweils gegebenenfalls ein- oder mehrfach, gleich oder verschieden durch Halogen, Cyano, Nitro, C₁-C₆-Alkyl, Halogen-C₁-C₆-alkyl, C₃-C₆-Cycloalkyl, Halogen-C₃-C₆-cycloalkyl, Halogen-C₂-C₆-Alkenyl, C₂-C₆-Alkinyl, Halogen-C₂-C₆-Alkinyl, C₁-C₄-Alkoxyalkyl, Halogen-C₁-C₄-alkoxy-C₁-C₆alkyl, C₃-C₆-Cycloalkoxy-C₁-C₆alkyl oder Halogen-C₃-C₆-cycloalkoxy-C₁-C₆alkyl substituiertes Benzyl, Phenyl, Heteroaryl, -CH₂-Heteroaryl, -CH₂CH₂-Heteroaryl, Pyranyl, Tetrahydrofuranyl, C₁-C₄-Alkanoyl, Halogen-C₁-C₄-Alkanoyl, Benzoyl oder für jeweils gegebenenfalls ein- oder mehrfach, gleich oder verschieden durch Halogen, Cyano, Nitro, C₁-C₆-Alkyl, Halogen-C₁-C₆-alkyl, C₃-C₆-Cycloalkyl oder Halogen-C₃-C₆-cycloalkyl substituiertes Benzoyl,
- R⁷: für Wasserstoff, für jeweils gegebenenfalls substituiertes C₁-C₆-Alkyl, C₃-C₆-Cycloalkyl, C₂-C₆-Alkenyl oder für ein Kation E,
- R⁸ und R⁹: unabhängig voneinander, gleich oder verschieden für Wasserstoff, C₁-C₆-Alkyl, C₂-C₆-Alkenyl, C₂-C₆-Alkinyl, C₃-C₆-Cycloalkyl, Phenyl, Benzyl, für jeweils ein- oder mehrfach, gleich oder verschieden durch Halogen, Nitro, Cyano oder C₁-C₃-Alkyl substituiertes Phenyl oder Benzyl oder
- R⁸ und R⁹: bilden gemeinsam mit dem benachbarten Stickstoffatom eine Morpholino- Piperidino- oder Pyrrolidinogruppe,
- R¹⁰: für Wasserstoff, alogen C₁-C₆-Alkyl, Halogen-C₁-C₄-alkyl, C₂-C₆-Alkenyl, Halogen-C₂-C₆-alkenyl, C₂-C₆-Alkinyl, C₃-C₆-Cycloalkyl, für jeweils gegebenenfalls ein- oder mehrfach, gleich oder verschieden durch Halogen, C₁-C₆-Alkyl oder Halogen-C₁-C₆-alkyl substituiertes Phenyl oder Benzyl,
- R¹¹ und R¹²: unabhängig voneinander, gleich oder verschieden für Wasserstoff, C₁-C₆-Alkyl, C₂-C₆-Alkenyl, C₂-C₆-Alkinyl, C₃-C₆-Cycloalkyl, Phenyl, Benzyl, für jeweils ein- oder mehrfach, gleich oder verschieden durch Halogen, Nitro, Cyano oder C₁-C₃-Alkyl substituiertes Phenyl oder Benzyl oder
- R¹¹ und R¹²: bilden gemeinsam mit dem benachbarten Stickstoffatom eine Morpholino- Piperidino- oder Pyrrolidinogruppe,
- R¹³: für Wasserstoff oder für jeweils gegebenenfalls ein- oder mehrfach durch Sauerstoff oder Schwefel unterbrochenenes, gebenenfalls jeweils ein- oder mehrfach durch Halogen substituiertes C₁-C₆-Alkyl, C₂-C₆-Alkenyl, C₂-C₆-Alkinyl, C₃-C₆-Cycloalkyl, C₃-C₆-Cycloalkyl-C₁-C₃-alkyl, jeweils gegebenenfalls ein- oder mehrfach, gleich oder verschieden durch Halogen, Nitro, Cyano oder C₁-C₃-Alkyl substituiertes Benzyl oder -CH₂-Heterocyclyl,
- R¹⁴: für Wasserstoff, C₁-C₆-Alkyl, Halogen-C₁-C₆-alkyl, C₂-C₆-Alkenyl, Halogen-C₂-C₆-alkenyl, C₂-C₆-Alkinyl, Halogen-C₂-C₆-alkinyl, Phenyl, Benzyl oder für jeweils ein- oder mehrfach, gleich oder verschieden durch Halogen, C₁-C₃-Alkyl, Halogen-C₁-C₃-alkyl, C₃-C₆-Cycloalkyl, Halogen-C₃-C₆-cycloalkyl, Nitro oder Cyano substituiertes Phenyl oder Benzyl,
- R¹⁵ und R¹⁶: unabhängig voneinander, gleich oder verschieden für Wasserstoff, C₁-C₅-Alkyl, C₂-C₆-Alkenyl, C₂-C₆-Alkinyl, C₃-C₆-Cycloalkyl, Phenyl, Benzyl, für jeweils ein- oder mehrfach, gleich oder verschieden durch Halogen, Nitro, Cyano oder C₁-C₃-Alkyl substituiertes Phenyl oder Benzyl oder
- R¹⁵ und R¹⁶: bilden gemeinsam mit dem benachbarten Stickstoffatom eine Morpholino-, Piperidino- oder Pyrrolidinogruppe,
- R¹⁷: für Wasserstoff, für jeweils gegebenenfalls ein- oder mehrfach, gleich oder verschieden durch Sauerstoff oder Schwefel unterbrochenes C₁-C₆-Alkyl, Benzyl oder Halogen-C₁-C₆-alkyl,
- R¹⁸: für Wasserstoff, für jeweils gegebenenfalls ein- oder mehrfach, gleich oder verschieden durch Sauerstoff oder Schwefel unterbrochenes C₁-C₆-Alkyl, Benzyl oder Halogen-C₁-C₆-alkyl,
- R¹⁹: für gegebenenfalls durch Halogen substituiertes C₁-C₆-Alkyl, C₂-C₆-Alkenyl, C₁-C₄-Alkoxy-C₁-C₄-alkyl, C₁-C₃-Alkylthio-C₁-C₄-alkyl, Poly-C₁-C₄-alkoxy-C₁-C₄-alkyl oder gegebenenfalls einoder mehrfach, gleich oder verschieden durch Halogen, C₁-C₄-Alkyl oder C₁-C₄-Alkoxy substituiertes C₃-C₆-Cycloalkyl, das gegebenenfalls durch Sauerstoff oder Schwefel im Ring unterbrochen sein kann, jeweils gegebenenfalls mit Halogen oder C₁-C₄-Alkyl substituiertes Phenyl, Phenyl-C₁-C₄-alkyl, Hetaryl, Phenoxy-C₁-C₄-alkyl oder Hetaryloxy-C₁-C₄-alkyl,
- R²⁰: für jeweils gegebenenfalls ein- oder mehrfach, gleich oder verschieden durch Halogen substituiertes C₁-C₆-Alkyl, C₂-C₆-Alkenyl, C₁-C₄-Alkoxy-C₁-C₄-alkyl, Poly-C₁-C₄-alkoxy C₁-C₄-alkyl oder für C₃-C₆-Cycloalkyl, das gegebenenfalls durch Sauerstoff oder Schwefel im Ring unterbrochen sein kann oder für Benzyl,
- R²¹, R²² und R²³: unabhängig voneinander, gleich oder verschieden für jeweils gegebenenfalls ein-oder mehrfach, gleich oder verschieden durch Halogen substituiertes C₁-C₆-Alkyl, C₁-C₆-Alkoxy, C₁-C₆-Alkylamino, C₁-C₆-Dialkylamino, C₁-C₃Alkylthio, C₂-C₄-Alkenylthio, C₃-C₆-Cycloalkylthio oder für Phenyl, Benzyl, Phenoxy oder Phenylthio,
- R²⁴ und R²⁵: unabhängig voneinander, gleich oder verschieden für Wasserstoff, jeweils gegebenenfalls ein-oder mehrfach, gleich oder verschieden durch Halogen substituiertes C₁-C₆-Alkyl, C₃-C₆-Cycloalkyl, C₂-C₆-Alkenyl, C₁-C₆-Alkoxy, C₁-C₆-AlkoxyC₁-C₄-alkyl, für gegebenenfalls ein- oder mehrfach, gleich oder verschieden durch Halogen oder C₁-C₄-Alkyl substituiertes Phenyl oder Benzyl oder
- R²⁴ und R²⁵: bilden gemeinsam mit dem benachbarten Stickstoffatom eine Morpholino- Piperidino- oder Pyrrolidinogruppe,
- p: für die Zahlen 0, 1 oder 2,
oder wenn
- Q, Y und R⁵: gemeinsam für die Gruppe CH₂= stehen, dann steht
- G: für Methyl, Ethyl oder Benzyl (a).

Der Substituent G kann entsprechend der Formeln (I-A), (I-B) oder (I-C) gebunden sein.

Die Bezeichnung "Halogen" umfaßt in diesem Zusammenhang Fluor, Chlor, Brom und Iod.

Unter den Begriffen "Alkyl", "Alkenyl" und "Alkinyl" sind sowohl geradkettige als auch verzweigte Kohlenwasserstoffreste zu verstehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Die Verbindungen der Formel (I) können in Abhängigkeit von der Stellung des Substituenten G in den zwei isomeren Formen der Formeln (I-A) und (I-B) vorliegen, 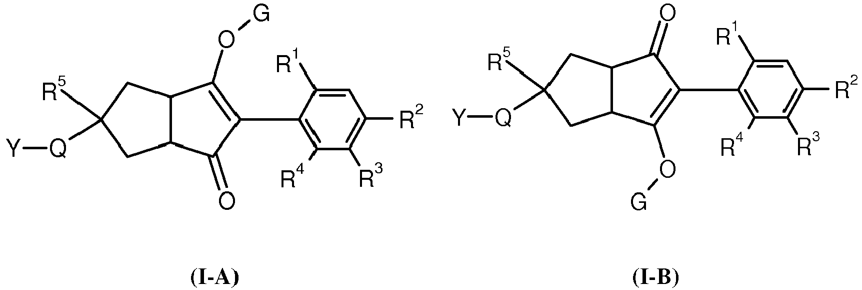 was durch die gestrichelte Linie in der Formel (I) zum Ausdruck gebracht werden soll.

Für den speziellen Fall, dass der Substituent G in der Formel (I) für Wasserstoff steht, kann zusätzlich eine weitere isomere Form mit der Formel (I-C) vorliegen

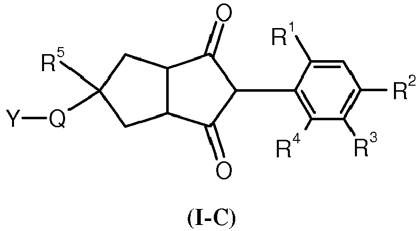

Die Verbindungen der Formeln (I-A), (I-B) und (I-C) können sowohl als Gemische als auch in Form ihrer reinen Isomeren vorliegen. Gemische der Verbindungen der Formeln (I-A), (I-B) und (I-C) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Aus Gründen der Einfachheit ist im Falle der Verbindungen der Formel (I-a) meist nur die isomere Form (I-C) aufgeführt, in der G für Wasserstoff steht. In den Verbindungen der Formel (I-a) kann der G-Substituent aber auch für Methyl, Ethyl oder Benzyl stehen und die Verbindungen liegen dann in den isomeren Formen (I-A) und (I-B) vor.

Unter Einbeziehung der verschiedenen Bedeutungen (a), (b), (c), (d), (e), (f) und (g) der Gruppe G ergeben sich folgende hauptsächlichen Strukturen (I-a) bis (I-g): 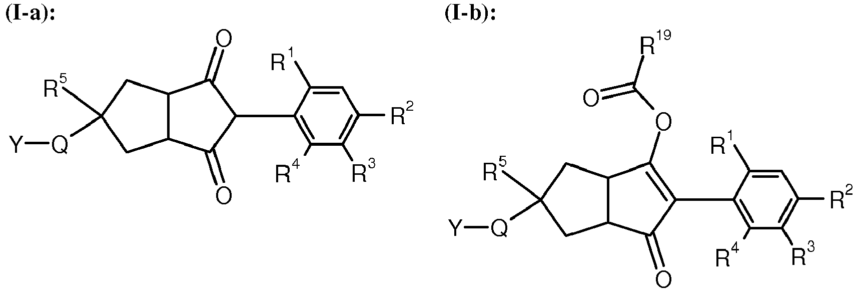 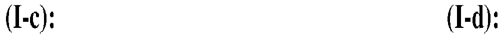 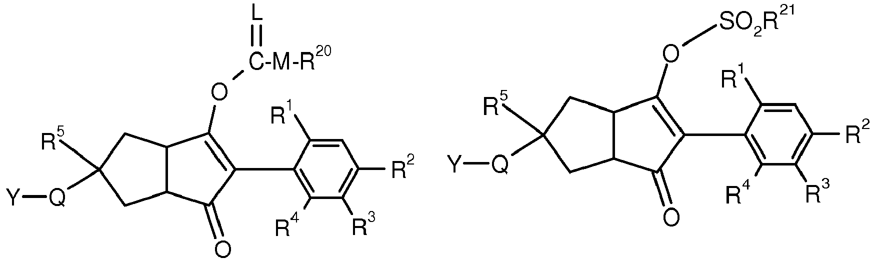 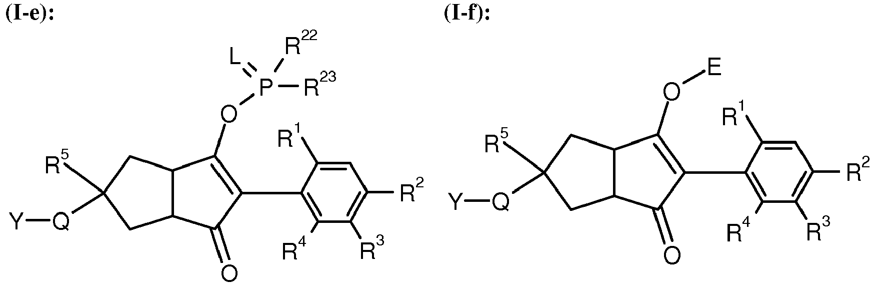 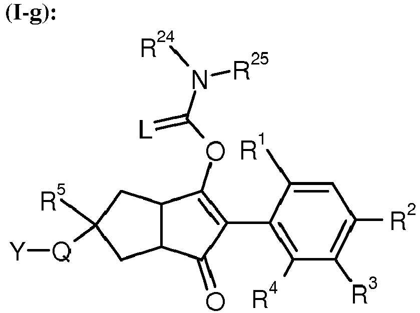 worin
Q, Y, E, L, M, R¹, R², R³, R⁴, R⁵, R¹⁹, R²⁰, R²¹, R²², R²³, R²⁴ und R²⁵ die oben angegebenen Bedeutungen besitzen.

Eine weitere Form von Stereoisomerie ergibt sich aus der cis-Verknüpfung der beiden carbacyclischen Fünfringe. In Abhängigkeit von der räumlichen Anordnung der Gruppierung Q-Y bzw. R⁵ ergeben sich zwei verschiedene räumliche Isomere.

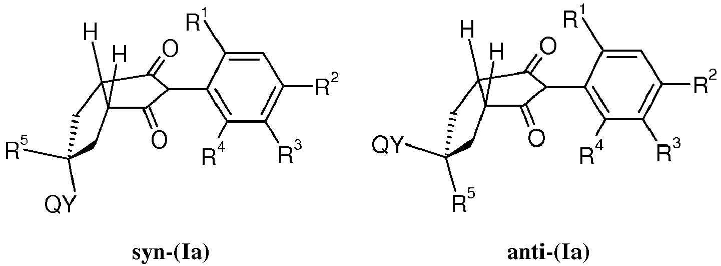

Diese Isomeren werden im Folgenden mit "syn" bzw "anti" bezeichnet, je nachdem ob die Gruppierung Q-Y in den erfindungsgemäßen Verbindungen in anti- oder syn-Position zum Cyclopentandion-Ring steht. Beispielsweise für den Fall, dass R⁵ für Wasserstoff, Q für eine -CH₂-Gruppe, Y für -CO₂CH₃ und R¹, R², R³ und R⁴ die bei der Formel (I-a) angegebene Bedeutung haben, sind syn- und anti-Isomer folgendermaßen definiert:

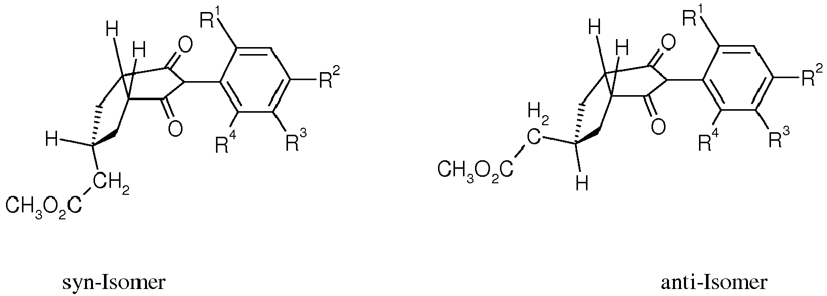

Weiterhin wurde gefunden, dass man die neuen Verbindungen der Formel (I) nach einem der im folgenden beschriebenen Verfahren erhält:
(A) Man erhält Verbindungen der Formel (I-a) 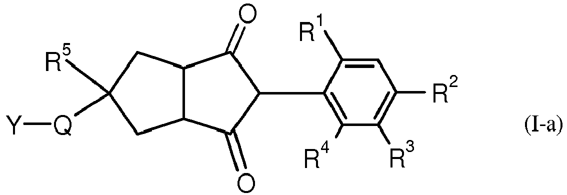 in welcher
   Q, Y, R¹, R², R³, R⁴ und R⁵ die oben angegebene Bedeutung haben,
   wenn man Ketocarbonsäureester der Formel (II) 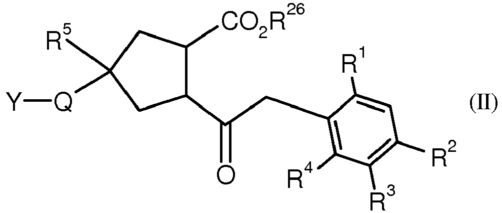 in welcher
   Q, Y, R¹, R², R³, R⁴ und R⁵ die oben angegebene Bedeutung haben, und
   R²⁶ für Alkyl (insbesondere C₁-C₈-Alkyl) steht, gegebenenfalls in Gegenwart eines Verdünnungsmittels in Gegenwart einer Base intramolekular cyclisiert.

Außerdem erhält man Verbindungen der Formel (I-a) durch weitere Funktionalisierung von bereits bekannten Verbindungen, beispielsweise den Synthesebeispielen I-a-4 und I-a-5 aus WO2010/040460, deren Herstellung literaturbekannt ist.

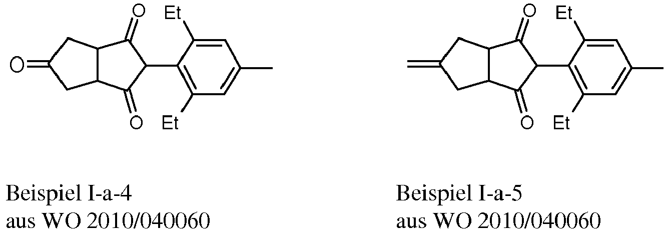

Die synthetische Überführung dieser und analoger Substanzen in die erfindungsgemäßen Verbindungen der Formel (I) kann nach bekannten, dem Fachmann geläufigen Standardverfahren der Organischen Chemie, beispielsweise Hydroborierung, Alkylierung, Acylierung, Oxidation, Reduktion, Acetalisierung, Kodensation, Wittig-Reaktion, Grignard- bzw. Organometall-Addition, Halogenierung oder nukleophile Substitutionsreaktionen erfolgen. In Schema 1 und 2 sind einige dieser Verfahren beispielhaft skizziert. Weitere Details können darüber hinaus auch den ausgeführten Herstellungsbeispielen entnommen werden.

Für den Fall, dass der Substituent G für Methyl, Ethyl oder Benzyl steht, erfolgt die Einführung des Subsituenten G durch Alkylierung oder Benzylierung ausgehend von der Verbindung, in der G für Wasserstoff steht.

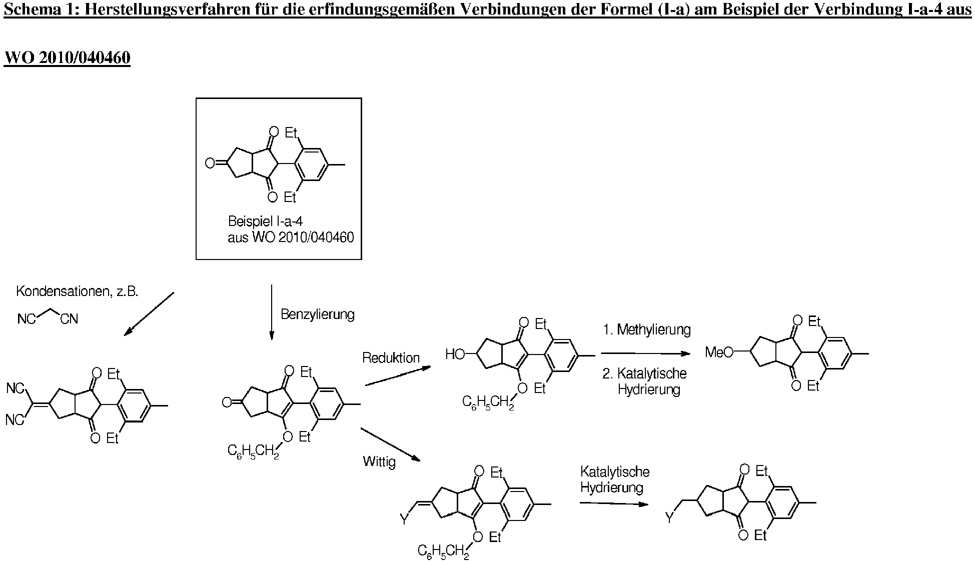

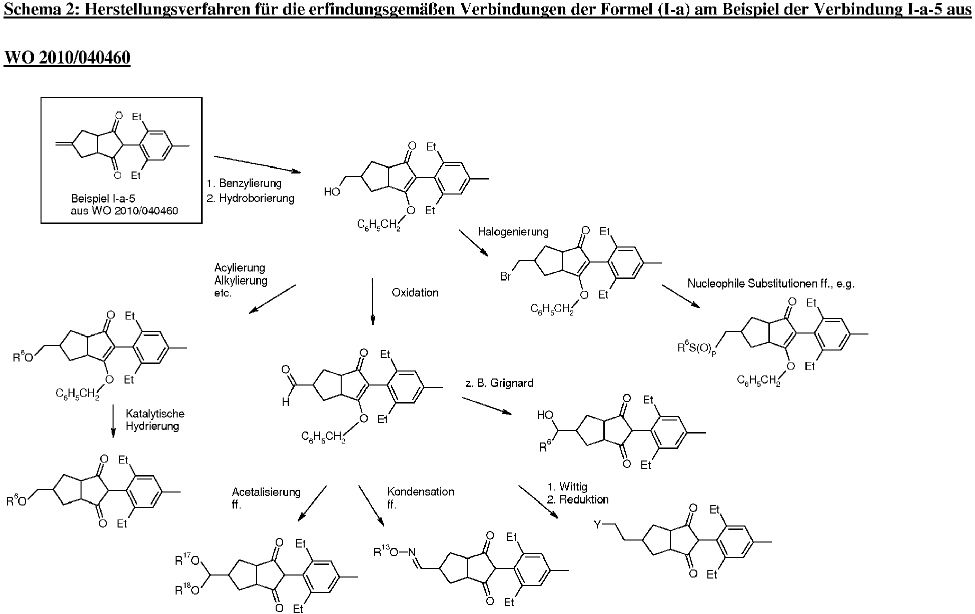

Außerdem wurde gefunden
(B) dass man die Verbindungen der oben gezeigten Formel (I-b), in welcher G, Q, Y, R¹, R², R³, R⁴ und R⁵ die oben angegebene Bedeutung haben, erhält, wenn man Verbindungen der oben gezeigten Formel (I-a), in welcher Q, Y, R¹, R², R³, R⁴ und R⁵ die oben angegebene Bedeutung haben haben, jeweils
(α) mit Säurehalogeniden der Formel (III) 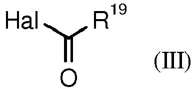 in welcher
   - R¹⁹: die oben angegebene Bedeutung hat und
   - Hal: für Halogen (insbesondere Chlor oder Brom) steht
   oder
(ß) mit Carbonsäureanhydriden der Formel (IV)

   R¹⁹-CO-O-CO-R¹⁹ (IV)

   in welcher
   - R¹⁹: die oben angegebene Bedeutung hat,
   gegebenenfalls in Gegenwart eines Verdünnungsmittels und gegebenenfalls in Gegenwart eines Säurebindemittels umsetzt;
(C) dass man die Verbindungen der oben gezeigten Formel (I-c), in welcher Q, Y, R¹, R², R³, R⁴ und R⁵ die oben angegebene Bedeutung haben haben und L für Sauerstoff steht, erhält, wenn man Verbindungen der oben gezeigten Formel (I-a), in welcher Q, Y, R¹, R², R³, R⁴ und R⁵ die oben angegebene Bedeutung haben, jeweils
   mit Chlorameisensäureestern oder Chlorameisensäurethioestern der Formel (V)

   R²⁰-M-CO-Cl (V)

   in welcher
   - R²⁰: und M die oben angegebenen Bedeutungen haben,
   gegebenenfalls in Gegenwart eines Verdünnungsmittels und gegebenenfalls in Gegenwart eines Säurebindemittels umsetzt;
(D) dass man Verbindungen der oben gezeigten Formel (I-c), in welcher Q, Y, R¹, R², R³, R⁴ und R⁵ die oben angegebene Bedeutung haben haben und L für Schwefel steht, erhält, wenn man Verbindungen der oben gezeigten Formel (I-a), in welchen Q, Y, R¹, R², R³, R⁴ und R⁵ die oben angegebene Bedeutung haben, jeweils
   mit Chlormonothioameisensäureestern oder Chlordithioameisensäureestern der Formel (VI) 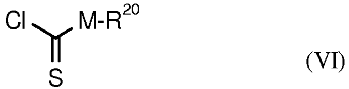 in welcher
   - M und R²⁰: die oben angegebenen Bedeutungen haben,
   gegebenenfalls in Gegenwart eines Verdünnungsmittels und gegebenenfalls in Gegenwart eines Säurebindemittels umsetzt;
(E) dass man Verbindungen der oben gezeigten Formel (I-d), in welcher Q, Y, R¹, R², R³, R⁴ und R⁵ die oben angegebene Bedeutung haben, erhält, wenn man Verbindungen der oben gezeigten Formel (I-a), in welcher Q, Y, R¹, R², R³, R⁴ und R⁵ die oben angegebene Bedeutung haben, jeweils
   mit Sulfonsäurechloriden der Formel (VII)

   R²¹-SO₂-Cl (VII)

   in welcher
   - R²¹: die oben angegebene Bedeutung hat,
   gegebenenfalls in Gegenwart eines Verdünnungsmittels und gegebenenfalls in Gegenwart eines Säurebindemittels umsetzt,
(F) dass man Verbindungen der oben gezeigten Formel (I-e), in welcher Q, Y, R¹, R², R³, R⁴ und R⁵ die oben angegebene Bedeutung haben, erhält, wenn man Verbindungen der oben gezeigten Formel (I-a), in welchen Q, Y, R¹, R², R³, R⁴ und R⁵ die oben angegebene Bedeutung haben, jeweils
   mit Phosphorverbindungen der Formel (VIII) 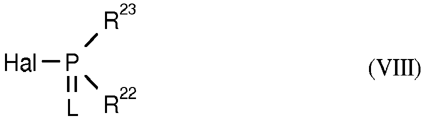 in welcher
   - L, R²² und R²³: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(G) dass man Verbindungen der oben gezeigten Formel (I-f), in welcher Q, Y, R¹, R², R³, R⁴ und R⁵ die oben angegebene Bedeutung haben, erhält, wenn man Verbindungen der Formel (I-a), in welcher Q, Y, R¹, R², R³, R⁴ und R⁵ die oben angegebene Bedeutung haben, jeweils
   mit Metallverbindungen oder Aminen der Formeln (IX) oder (X) 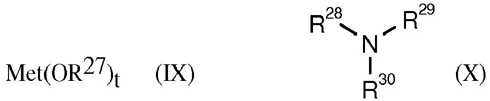 in welchen
   - Met: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R²⁷, R²⁸, R²⁹ und R³⁰: unabhängig voneinander für Wasserstoff oder Alkyl (bevorzugt C₁-C₈-Alkyl) stehen,
   gegebenenfalls in Gegenwart eines Verdünnungsmittels umsetzt,
(H) dass man Verbindungen der oben gezeigten Formel (I-g), in welcher Q, Y, R¹, R², R³, R⁴ R⁵ und R²⁴ die oben angegebene Bedeutung haben und R²⁵ für Wasserstoff steht, erhält, wenn man Verbindungen
   der oben gezeigten Formel (I-a), in welcher Q, Y, R¹, R², R³, R⁴ und R⁵ die oben angegebene Bedeutung haben, jeweils
(α) mit Isocyanaten oder Isothiocyanaten der Formel (XI)

   R²⁴-N=C=L (XI)

   in welcher
   - R²⁴ und L: die oben angegebenen Bedeutungen haben,
   egebenenfalls in Gegenwart eines Verdünnungsmittels und gegebenenfalls in Gegenwart eines Katalysators umsetzt oder
(ß) mit Carbamidsäurechloriden oder Thiocarbamidsäurechloriden der Formel (XII) 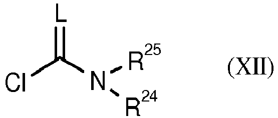 in welcher
   - L, R²⁴ und R²⁵: die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder als Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:

Bevorzugt sind Verbindungen der Formel (I), in denen
wenn
- Q: für eine Bindung, eine C₁-C₃-Alkylen-, eine C₂-C₃-Alkenylen- oder eine C₂-C₃-Alkinylenkette steht,
dann stehen
- Y: für die Gruppen -OR⁶, -S(O)ₚR⁶, -CO₂R⁷, -CH=CH₂, Cyano, -SCN, -CONR⁸R⁹, -SO₂NR⁸R⁹,-CR¹⁰=O, -NR¹¹R¹², -CR¹⁰=N-OR¹³, -CR¹⁰=N-R¹⁴, CR¹⁰=N-NR¹⁵R¹⁶, -CR¹⁰(OR¹⁷OR¹⁸),-CR¹⁰(SR¹⁷OR¹⁸), -CR¹⁰(SR¹⁷SR¹⁸), -CR¹⁰(NHR¹⁷NHR¹⁸), -CR¹⁰(NHR¹⁷OR¹⁸), -CR¹⁰(NHR¹⁷SR¹⁸),-CH(CN)₂, -CH(OH)R⁶, Halogen, -O(C=M)R¹⁰, -S(C=M)R¹⁰, -O(C=M)NR¹¹R¹², -S(C=M)NR¹¹R¹², -NH(C=M)NR¹¹R¹², -O(C=M)OR⁷, -S(C=M)OR⁷, -NH(C=M)OR⁷ oder für die Gruppe W,
- oder Q, Y und R⁵: bilden gemeinsam eine der Gruppen CHCN=, CH(CO₂C₁-C₆-Alkyl)=, 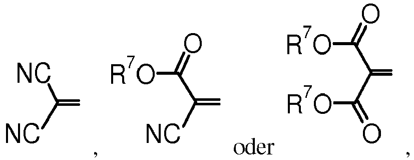
- W: für einen der nachfolgend genannten 3 bis 7-gliedrigen gesättigten oder teilgesättigten Heterocyclen, die unterschiedlich verküpft und ein- oder mehrfach, gleich- oder verschieden durch die Substituenten R³¹ und R³² substituiert sein können 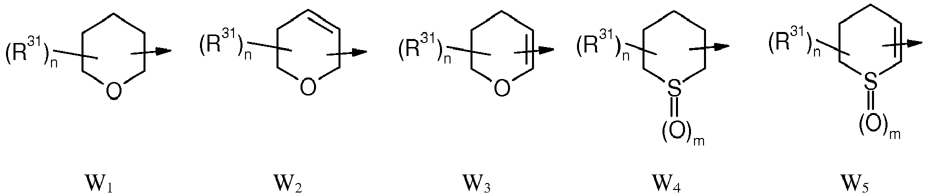 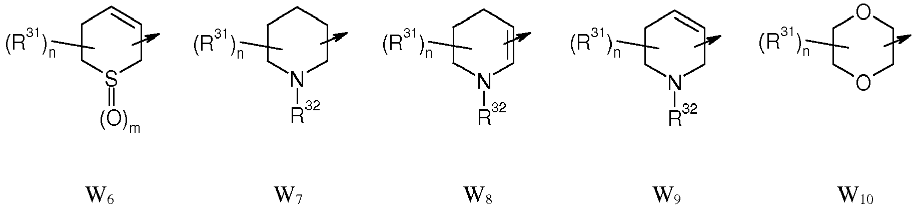 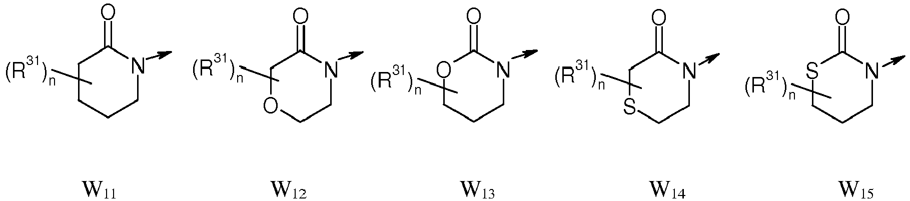 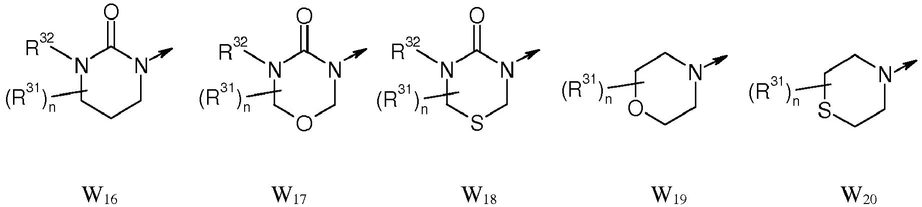 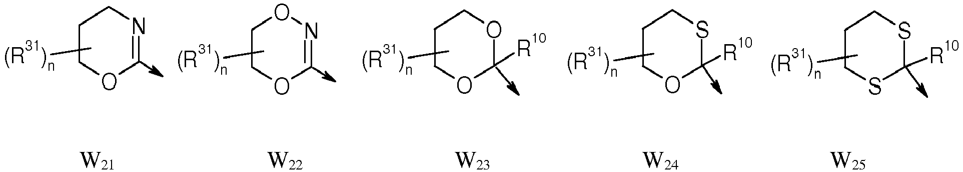 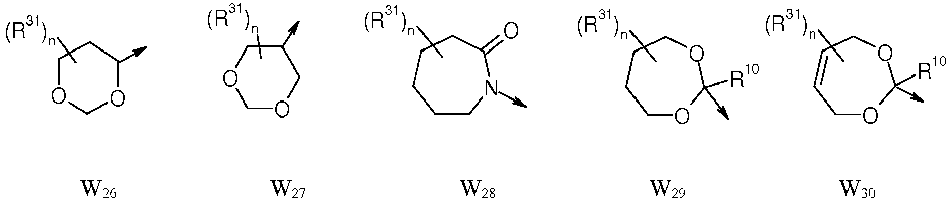 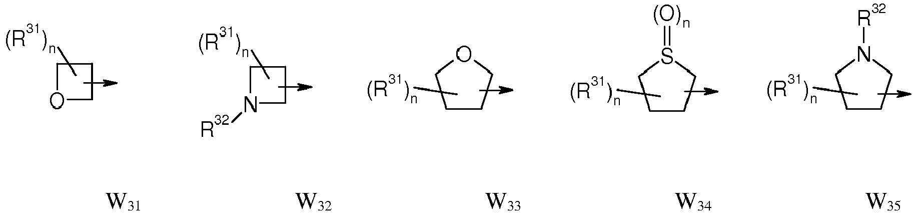 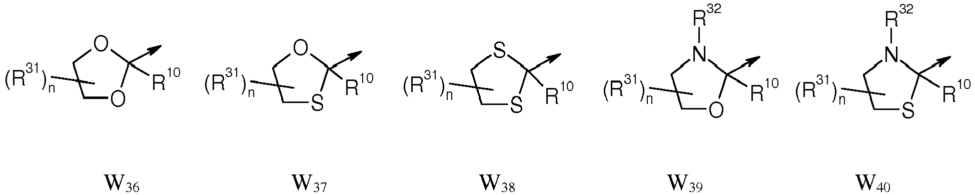 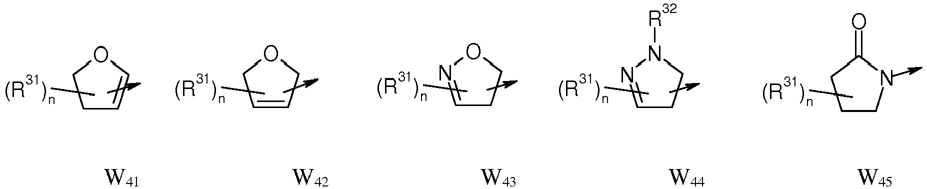 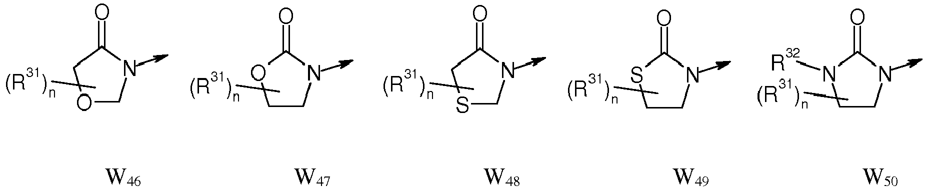 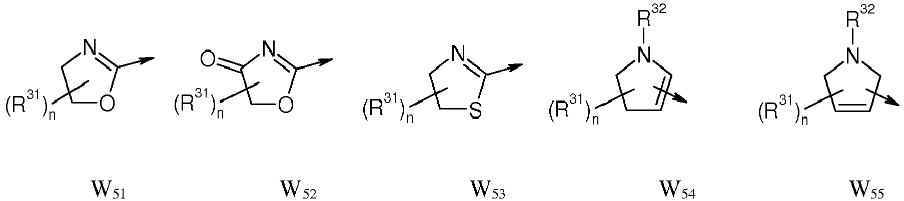 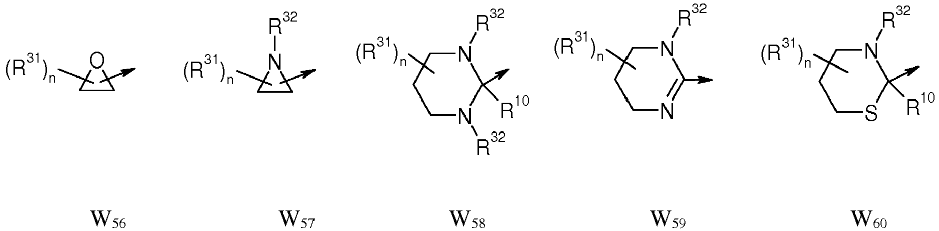 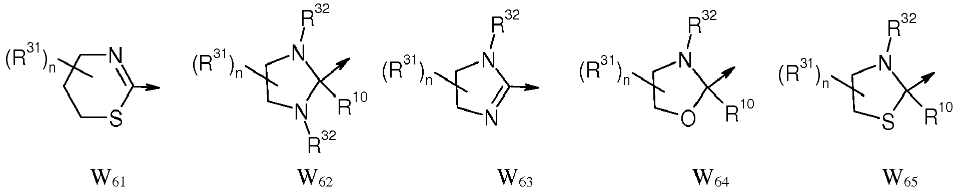 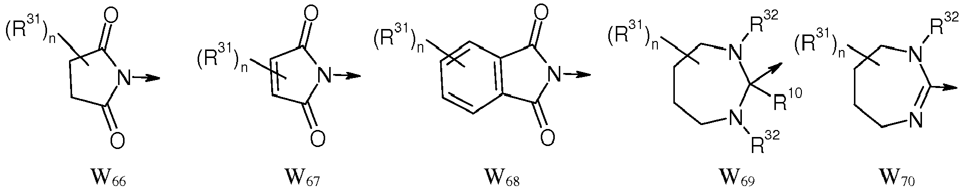 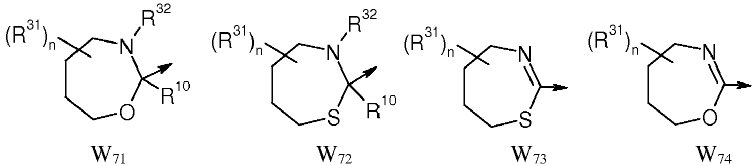
- G: Wasserstoff, Methyl, Ethyl oder Benzyl (a) oder für eine der Gruppen 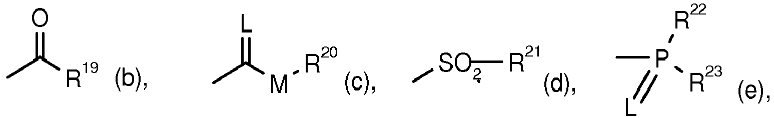 E (f) oder 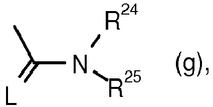
- E: für ein Metallionenäquivalent, ein tertiäres Sulfoniumion oder ein Ammoniumion,
- L: für Sauerstoff oder Schwefel,
- M: für Sauerstoff oder Schwefel,
- R¹: für Wasserstoff, Halogen, C₁-C₄-Alkyl, Halogen-C₁-C₄-alkyl, C₃-C₆-Cycloalkyl, Halogen-C₃-C₆-cycloalkyl, C₂-C₄-Alkenyl, Halogen-C₂-C₄-alkenyl, C₂-C₄-Alkinyl, Halogen-C₂-C₄-alkinyl, C₁-C₄-Alkoxy, Halogen-C₁-C₄-alkoxy, C₂-C₄-Alkinyloxy, Halogen-C₂-C₄-alkinyloxy, C₂-C₄-Alkenyloxy, Halogen-C₂-C₄-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl oder Halogen-C₁-C₃-alkylsulfonyl,
- R² und R³: unabhängig voneinander, gleich oder verschieden für Wasserstoff, Halogen, C₁-C₄-Alkyl, Halogen-C₁-C₄-alkyl, C₂-C₄-Alkenyl, Halogen-C₂-C₄-alkenyl, C₂-C₄-Alkinyl, Halogen-C₂-C₄-alkinyl, C₁-C₄-Alkoxy, Halogen-C₁-C₄-alkoxy, C₃-C₆-Cycloalkyl, C₃-C₆-Cycloalkoxy, Halogen-C₃-C₆-cycloalkoxy, C₂-C₄-Alkinyloxy, Halogen-C₂-C₄-alkinyloxy, C₂-C₄-Alkenyloxy, Halogen-C₂-C₄-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl, Halogen-C₁-C₃-alkylsulfonyl, Phenyl oder gegebenenfalls ein- oder mehrfach, gleich oder verschieden durch Halogen, C₁-C₄-Alkyl, Halogen-C₁-C₄-alkyl substituiertes Phenyl,
- R⁴: für Wasserstoff, Halogen, C₁-C₄-Alkyl, Halogen-C₁-C₄-alkyl, C₃-C₆-Cycloalkyl, Halogen-C₃-C₆-cycloalkyl, C₁-C₄-Alkoxy, Halogen-C₁-C₄-alkoxy, C₃-C₄-Cycloalkoxy, oder Halogen-C₃-C₆-cycloalkoxy,
- R⁵: für Wasserstoff oder C₁-C₄-Alkyl,
- R⁶: für Wasserstoff, C₁-C₄-Alkyl, Halogen-C₁-C₄-alkyl, C₃-C₆-Cycloalkyl, Halogen-C₃-C₆-cycloalkyl, C₂-C₄-Alkenyl, Halogen-C₂-C₄-Alkenyl, C₂-C₄-Alkinyl, Halogen-C₂-C₄-Alkinyl, C₁-C₄-Alkoxy-C₁-C₄-alkyl, Halogen-C₁-C₄-Alkoxy-C₁-C₄-alkyl, C₁-C₆-Cycloalkoxy-C₁-C₄-alkyl, Halogen-C₁-C₄-Cycloalkoxy-C₁-C₄-alkyl, für jeweils gegebenenfalls ein- oder mehrfach, gleich oder verschieden durch Halogen, Cyano, Nitro, C₁-C₄-Alkyl, Halogen-C₁-C₄-alkyl, C₃-C₆-Cycloalkyl, Halogen-C₃-C₆-cycloalkyl, Halogen-C₂-C₄-Alkenyl, C₂-C₄-Alkinyl, Halogen-C₂-C₄-Alkinyl, C₁-C₄-Alkoxyalkyl, Halogen-C₁-C₄-alkoxy-C₁-C₆alkyl, C₃-C₆-Cycloalkoxy-C₁-C₆alkyl oder Halogen-C₃-C₆-cycloalkoxy-C₁-C₆alkyl substituiertes Benzyl, Phenyl, Heteroaryl, -CH₂-Heteroaryl, -CH₂CH₂-Heteroaryl, (beispielsweise Pyranyl, Tetrahydrofuranyl, Pyrazolyl, Thiazolyl, Pyridyl, Pyrimidyl, Furanyl, Thienyl, Benzoxazolyl, Oxazolyl), C₁-C₄-Alkanoyl, Halogen-C₁-C₄-Alkanoyl, Benzoyl oder für jeweils gegebenenfalls ein- oder mehrfach, gleich oder verschieden durch Halogen, Cyano, Nitro, C₁-C₄-Alkyl, Halogen-C₁-C₄-alkyl, C₃-C₆-Cycloalkyl oder Halogen-C₃-C₆-cycloalkyl substituiertes Benzoyl,
- R⁷: für Wasserstoff, für jeweils gegebenenfalls ein-oder mehrfach, gleich oder verschieden durch Halogen oder C₁-C₄-Alkoxy substituiertes C₁-C₄-Alkyl, C₃-C₆-Cycloalkyl, C₂-C₄-Alkenyl oder für ein Kation E,
- R⁸ und R⁹: unabhängig voneinander, gleich oder verschieden für Wasserstoff, C₁-C₄-Alkyl, C₂-C₄-Alkenyl, C₂-C₄-Alkinyl, C₃-C₆-Cycloalkyl, Phenyl, Benzyl, für jeweils ein- oder mehrfach, gleich oder verschieden durch Halogen, Nitro, Cyano oder C₁-C₃-Alkyl substituiertes Phenyl oder Benzyl oder
- R⁸ und R⁹: bilden gemeinsam mit dem benachbarten Stickstoffatom eine Morpholino- Piperidino- oder Pyrrolidinogruppe,
- R¹⁰: für Wasserstoff, alogen C₁-C₄-Alkyl, Halogen-C₁-C₄-alkyl, C₂-C₄-Alkenyl, Halogen-C₂-C₄-alkenyl, C₂-C₄-Alkinyl, C₃-C₆-Cycloalkyl, für jeweils gegebenenfalls ein- oder mehrfach, gleich oder verschieden durch Halogen, C₁-C₄-Alkyl oder Halogen-C₁-C₄-alkyl substituiertes Phenyl oder Benzyl,
- R¹¹ und R¹²: unabhängig voneinander, gleich oder verschieden für Wasserstoff, C₁-C₄-Alkyl, C₂-C₄-Alkenyl, C₂-C₄-Alkinyl, C₃-C₆-Cycloalkyl, Phenyl, Benzyl, für jeweils ein- oder mehrfach, gleich oder verschieden durch Halogen oder C₁-C₃-Alkyl substituiertes Phenyl oder Benzyl oder
- R¹¹ und R¹²: bilden gemeinsam mit dem benachbarten Stickstoffatom eine Morpholino- Piperidino- oder Pyrrolidinogruppe,
- R¹³: für Wasserstoff oder für gebenenfalls jeweils ein- oder mehrfach durch Halogen substituiertes C₁-C₄-Alkyl, C₂-C₄-Alkenyl, C₂-C₄-Alkinyl, C₃-C₄-Cycloalkyl, C₃-C₆-Cycloalkyl-C₁-C₃-alkyl, jeweils gegebenenfalls ein- oder mehrfach, gleich oder verschieden durch Halogen oder C₁-C₃-Alkyl substituiertes Benzyl oder -CH₂-Heterocyclyl,
- R¹⁴: für Wasserstoff, C₁-C₄-Alkyl, Halogen-C₁-C₄-alkyl, C₂-C₄-Alkenyl, C₂-C₄-Alkinyl, Halogen-C₂-C₄-alkinyl, Phenyl, Benzyl oder für jeweils ein- oder mehrfach, gleich oder verschieden durch Halogen, C₁-C₃-Alkyl, Halogen-C₁-C₃-alkyl, C₃-C₆-Cycloalkyl, Halogen-C₃-C₆-cycloalkyl substituiertes Phenyl oder Benzyl,
- R¹⁵ und R¹⁶: unabhängig voneinander, gleich oder verschieden für Wasserstoff, C₁-C₄-Alkyl, C₂-C₄-Alkenyl, C₂-C₄-Alkinyl, C₃-C₆-Cycloalkyl, Phenyl, Benzyl, für jeweils ein- oder mehrfach, gleich oder verschieden durch Halogen oder C₁-C₃-Alkyl substituiertes Phenyl oder Benzyl oder
- R¹⁵ und R¹⁶: bilden gemeinsam mit dem benachbarten Stickstoffatom eine Morpholino-, Piperidino- oder Pyrrolidinogruppe,
- R¹⁷: für Wasserstoff, für jeweils gegebenenfalls ein- oder mehrfach, gleich oder verschieden durch Sauerstoff oder Schwefel unterbrochenes C₁-C₄-Alkyl, Benzyl oder Halogen-C₁-C₄-alkyl,
- R¹⁸: für Wasserstoff, für jeweils gegebenenfalls ein- oder mehrfach, gleich oder verschieden durch Sauerstoff oder Schwefel unterbrochenes C₁-C₄-Alkyl, Benzyl oder Halogen-C₁-C₄-alkyl,
- R¹⁹: für gegebenenfalls durch Halogen substituiertes C₁-C₆-Alkyl, C₂-C₆-Alkenyl, C₁-C₄-Alkoxy-C₁-C₄-alkyl, C₁-C₃-Alkylthio-C₁-C₄-alkyl, Poly-C₁-C₄-alkoxy-C₁-C₄-alkyl oder gegebenenfalls einoder mehrfach, gleich oder verschieden durch Halogen, C₁-C₄-Alkyl oder C₁-C₄-Alkoxy substituiertes C₃-C₆-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind, jeweils gegebenenfalls mit Halogen oder C₁-C₄-Alkyl substituiertes Phenyl, Phenyl-C₁-C₄-alkyl, Hetaryl (beispielsweise Pyrazolyl, Thiazolyl, Pyridyl, Pyrimidyl, Furanyl oder Thienyl), Phenoxy-C₁-C₄-alkyl oder HetaryloxyC₁-C₄-alkyl (beispielsweise Pyridyloxy-C₁-C₄-alkyl, Pyrimidyloxy-C₁-C₄-alkyl oder Thiazolyloxy-C₁-C₄-alkyl),
- R²⁰: für jeweils gegebenenfalls ein- oder mehrfach, gleich oder verschieden durch Halogen substituiertes C₁-C₆-Alkyl, C₂-C₆-Alkenyl, C₁-C₄-Alkoxy C₁-C₄-alkyl, Poly-C₁-C₄-alkoxy C₁-C₄-alkyl oder für C₃-C₆-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind, oder für Benzyl,
- R²¹, R²² und R²³: unabhängig voneinander, gleich oder verschieden für jeweils gegebenenfalls ein-oder mehrfach, gleich oder verschieden durch Halogen substituiertes C₁-C₄-Alkyl, C₁-C₄-Alkoxy, C₁-C₄-Alkylamino, C₁-C₄-Dialkylamino, C₁-C₃-Alkylthio, C₂-C₄-Alkenylthio, C₃-C₆-Cycloalkylthio oder für jeweils gegebenenfalls ein-oder mehrfach, gleich oder verschieden durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Benzyl, Phenoxy oder Phenylthio,
- R²⁴ und R²⁵: unabhängig voneinander, gleich oder verschieden für Wasserstoff, jeweils gegebenenfalls ein-oder mehrfach, gleich oder verschieden durch Halogen substituiertes C₁-C₆-Alkyl, C₃-C₆-Cycloalkyl, C₂-C₆-Alkenyl, C₁-C₆-Alkoxy, C₁-C₆-AlkoxyC₁-C₄-alkyl, für gegebenenfalls ein- oder mehrfach, gleich oder verschieden durch Halogen oder C₁-C₄-Alkyl substituiertes Phenyl oder Benzyl oder
- R²⁴ und R²⁵: bilden gemeinsam mit dem benachbarten Stickstoffatom eine Morpholino- Piperidino- oder Pyrrolidinogruppe bilden,
- R³¹: für Halogen, Cyano, Nitro, C₁-C₆-Alkyl, Halogen-C₁-C₆-alkyl, C₃-C₆-Cycloalkyl, Halogen-C₃-C₆-cycloalkyl, C₂-C₆-Alkenyl, Halogen-C₂-C₆-alkenyl, C₂-C₆-Alkinyl, Halogen-C₂-C₆-alkinyl, C₁-C₆-Alkoxy, Halogen-C₁-C₆-alkoxy, C₃-C₆-Cycloalkoxy, Halogen-C₃-C₆-cycloalkoxy, C₁-C₆-Alkinyloxy, Halogen-C₁-C₆-alkinyloxy, C₂-C₆-Alkenyloxy, Halogen-C₂-C₆-alkenyxloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl, C₁-C₃-Alkylsulfonyl" C₁-C₆-Alkylcarbonyl, C₁-C₆-Alkoxycarbonyl, C₁-C₆-Alkylcarbamoyl, Amino, C₁-C₆-Alkylamino oder C₁-C₆-Dialkylamino,
- R³²: für Wasserstoff, Halogen, Cyano, Nitro, C₁-C₆-Alkyl, Halogen-C₁-C₆-alkyl, C₃-C₆-Cycloalkyl, Halogen-C₃-C₆-cycloalkyl, C₂-C₆-Alkenyl, Halogen-C₂-C₆-alkenyl, C₂-C₆-Alkinyl, Halogen-C₂-C₆-alkinyl, C₁-C₃-Alkylsulfonyl, C₁-C₃-Alkylsulfonyl, C₁-C₆-Alkylcarbonyl, C₁-C₆-Alkoxycarbonyl, C₁-C₆-Alkylcarbamoyl, Amino, C₁-C₆-Alkylamino oder C₁-C₆-Dialkylamino,
- n: für die Zahlen 0, 1,2, 3, 4, 5 oder 6,
- p: für die Zahlen 0, 1 oder 2,
oder wenn
- Q, Y und R⁵: gemeinsam für die Gruppen CH₂= stehen,
dann steht
- G: für Methyl, Ethyl oder Benzyl (a).

Besonders bevorzugt sind Verbindungen der Formel (I), in denen
wenn
- Q: für eine Bindung, -CH₂-, -CH=CH- oder -CH₂CH₂- steht,
dann stehen
- Y: für die Gruppen -OR⁶, -CO₂R⁷, -CH=CH₂, Cyano, -CR¹⁰=O, -CR¹⁰=N-OR¹³, -CH(OR¹⁷OR¹⁸), S(O)ₚR⁶, -SCN, -CONR⁸R⁹, -CH(CN)₂, -CH(OH)R⁶, Halogen, -O(C=M)R¹⁰, -S(C=M)R¹⁰, -O(C=M)NR¹¹R¹², -O(C=M)OR⁷, -NH(C=M)OR⁷ oder für die Gruppe W, oder Q, Y und R⁵ bilden gemeinsam eine der Gruppen 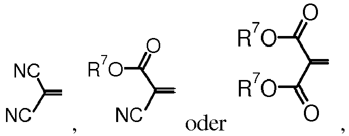
- W: für 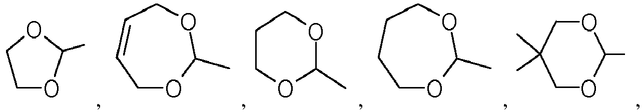 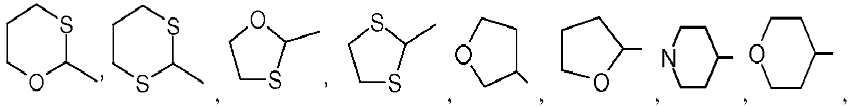 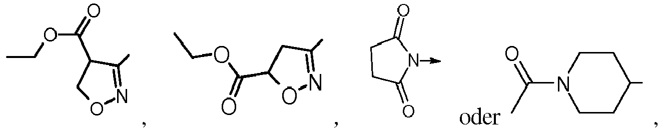
- G: für Wasserstoff, Ethyl oder Benzyl oder für eine der Gruppen 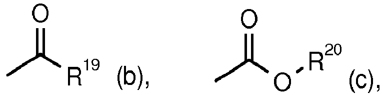
- R¹: für Methyl, Ethyl, Methoxy, Ethoxy, Halogen oder Cyclopropyl,
- R²: für Methyl, Ethyl oder 4-Chlorphenyl,
- R³: für Wasserstoff, Methyl, Ethyl oder Cyclopropyl,
- R⁴: für Wasserstoff, Methyl oder Ethyl,
- R⁵: für Wasserstoff,
- R⁶: für Wasserstoff , Methyl, Ethyl, -CH₂-CH(CH₃)₂, -CH₂-CH=CH₂, Cyano, Trifluormethyl, Methoxymethyl, 2-Benzoxazolyl, 4,5-Dimethylthiazol-2-yl, 2-Oxazolyl, 2-Tetrahydrofuryl oder die 2-Pyranylgruppe,
- R⁷: für Wasserstoff, Methyl, Ethyl, iso-Propyl oder n-Propyl,
- R⁸: für Wasserstoff oder Methyl,
- R⁹: für Wasserstoff oder Methyl,
- oder R⁸ und R⁹: bilden gemeinsam mit dem Stickstoffatom die Gruppe 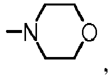
- R¹⁰: für Wasserstoff, Methyl, t-Butyl, Fluormethyl, Difluormethyl oder Trifluormethyl,
- R¹¹: für Wasserstoff oder Methyl,
- R¹²: für Wasserstoff, Methyl, Benzyl oder Phenyl,
- R¹³: für Wasserstoff, Methyl, iso-Propyl, -CH₂CH=CCl₂, -CH₂CH=CH₂, -CH₂C≡CH oder -CH₂C₃H₅,
- R¹⁷: für Methyl, Ethyl oder n-Propyl,
- R¹⁸: für Methyl, Ethyl oder n-Propyl,
- R¹⁹: für C₁-C₄-Alkyl,
- R²⁰: für Metyhl, Ethyl oder iso-Propyl,
- p: für 0 oder 2,
- M: für Sauerstoff oder Schwefel,
oder wenn
- Q, Y und R⁵: gemeinsam für die Gruppe CH₂= stehen,
dann steht
- G: für Methyl, Ethyl oder Benzyl (a).

Ganz besonders bevorzugt sind Verbindungen der Formel (I), in denen
- Q: für eine Bindung, -CH₂-, -CH₂CH₂- oder -CH=CH- steht,
- Y: für die Gruppen -OR⁶, -CO₂R⁷, -CH=CH₂, Cyano, -CR¹⁰=O, -CR¹⁰=N-OR¹³, -CH(OR¹⁷OR¹⁸), S(O)ₚR⁶, -SCN, -CONR⁸R⁹, -CH(CN)₂, -CH(OH)R⁶, Brom, -O(C=O)R¹⁰, -S(C=O)R¹⁰, -O(C=O)NR¹¹R¹² oder für die Gruppe W steht,
- oder Q, Y und R⁵: gemeinsam eine der Gruppen 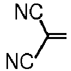 oder 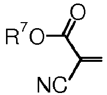 bilden, W für 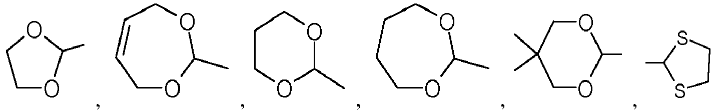 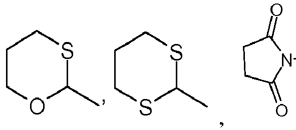 oder 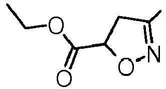 steht,
- G: Wasserstoff, Ethyl, Benzyl (a) oder für eine der Gruppen 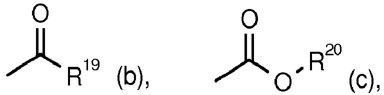 steht,
- R¹: für Methyl oder Ethyl steht, (insbesondere bevorzugt ist Ethyl),
- R²: für Methyl steht,
- R³: für Wasserstoff steht,
- R⁴: für Methyl oder Ethyl steht, (insbesondere bevorzugt ist Ethyl),
- R⁵: für Wasserstoff steht,
- R⁶: für Wasserstoff, Methyl, Ethyl, -CH₂-CH(CH₃)₂, -CH₂-CH=CH₂, Trifluormethyl, Methoxymethyl, 2-Benzoxazolyl, 4,5-Dimethylthiazol-2-yl, 2-Oxazolyl, 2-Tetrahydrofuryl oder die 2-Pyranylgruppe steht,
- R⁷: für Wasserstoff, Methyl oder Ethyl steht,
- R⁸: für Wasserstoff oder Methyl steht,
- R⁹: für Wasserstoff oder Methyl steht,
- oder R⁸ und R⁹: gemeinsam mit dem Stickstoffatom die Gruppe 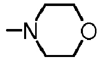 bedeuten,
- R¹⁰: für Wasserstoff, Methyl, t-Butyl oder Trifluormethyl steht,
- R¹¹: für Wasserstoff oder Methyl steht,
- R¹²: für Wasserstoff, Methyl, Benzyl oder Phenyl steht,
- R¹³: für Wasserstoff, Methyl, iso-Propyl, -CH₂CH=CCl₂, -CH₂CH=CH₂, -CH₂C=CH oder-CH₂C₃H₅ steht,
- R¹⁷: für Methyl oder Ethyl steht,
- R¹⁸: für Methyl oder Ethyl steht,
- R¹⁹: für Ethyl, tert.-Butyl oder iso-Propyl steht,
- R²⁰: für Methyl, Ethyl oder iso-Propyl steht,
- p: für 0 oder 2 steht.
Hervorgehoben sind Verbindungen der Formel (I), in denen
- Q, Y und R⁵: gemeinsam für die Gruppe CH₂= stehen,
- G: für Benzyl (a) steht,
- R¹: für Ethyl steht,
- R²: für Methyl steht,
- R³: für Wasserstoff steht,
- R⁴: für Ethyl steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Besonders hervorgehoben steht G für Wasserstoff.

Im einzelnen seien außer den bei den Herstellungsbeispielen genannten Verbindungen die folgenden Verbindungen der Formel (I-1-a) genannt:

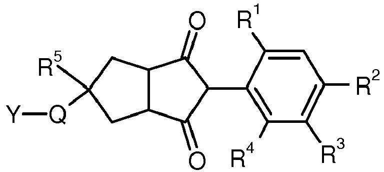

**Tabelle 1: R¹ = CH₃, R² = CH₃, R³ = CH₃, R⁴ = H, R⁵ = H,**

| Y | Q |
|---|---|
| -CO₂H | Bindung |
| -CO₂Me | Bindung |
| -CO₂Et | Bindung |
| -CO₂ⁱPr | Bindung |
| -CONH₂ | Bindung |
| -CONHCH₃ | Bindung |
| -CON(CH₃)₂ | Bindung |
| 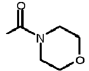 | Bindung |
| -CN | Bindung |
| -SCN | Bindung |
| -CHO | Bindung |
| -CH=N-OH | Bindung |
| -CH=N-OCH₃ | Bindung |
| -CH=N-O-CH₂C=CH₂ | Bindung |
| -CH=N-O-CH₂C=CCl₂ | Bindung |
| -CH=N-O-CH₂C≡CH | Bindung |
| -CH=N-OⁱPr | Bindung |
| CH₃(C=O)- | Bindung |
| 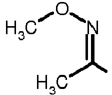 | Bindung |
| -CH=CH₂ | Bindung |
| -CH(CN)₂ | Bindung |
| -C(CN)(CO₂CH₃) | Bindung |
| -C(CO₂CH₃)₂ | Bindung |
| 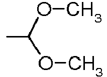 | Bindung |
| 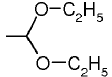 | Bindung |
| 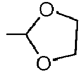 | Bindung |
| 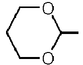 | Bindung |
| 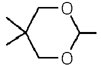 | Bindung |
| 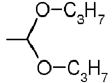 | Bindung |
| 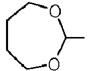 | Bindung |
| 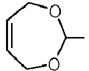 | Bindung |
| 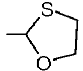 | Bindung |
| 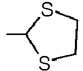 | Bindung |
| 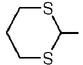 | Bindung |
| 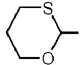 | Bindung |
| 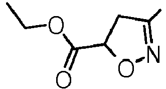 | Bindung |
| -OH | -CH₂- |
| -OCH₃ | -CH₂- |
| -OCF₃ | -CH₂- |
| -OCHF₂ | -CH₂- |
| -OCH₂F | -CH₂- |
| -OCH₂CH₃ | -CH₂- |
| -OCH₂OCH₃ | -CH₂- |
| 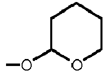 | -CH₂- |
| 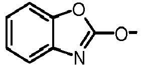 | -CH₂- |
| 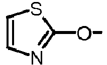 | -CH₂- |
| -O(C=O)-OCH₃ | -CH₂- |
| -O(C=O)-OCF₃ | -CH₂- |
| -O(C=O)-OC₂H₅ | -CH₂- |
| -O(C=O)-N(CH₃)₂ | -CH₂- |
| -O(C=O)-NHC₆H₅ | -CH₂- |
| -O(C=O)-NHCH₂C₆H₅ | -CH₂- |
| -O(C=O)-NHCH₃ | -CH₂- |
| -O(C=S)-NHC₆H₅ | -CH₂- |
| -CHO | -CH₂- |
| -CO₂H | -CH₂- |
| -CO₂Me | -CH₂- |
| -CO₂Et | -CH₂- |
| -CO₂ⁱPr | -CH₂- |
| -CONH₂ | -CH₂- |
| -CONHCH₃ | -CH₂- |
| -CON(CH₃)₂ | -CH₂- |
| 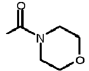 | -CH₂- |
| 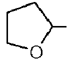 | -CH₂- |
| 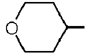 | -CH₂- |
| 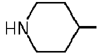 | -CH₂- |
| 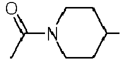 | -CH₂- |
| 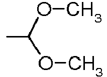 | -CH₂- |
| 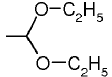 | -CH₂- |
| 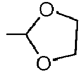 | -CH₂- |
| 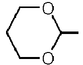 | -CH₂- |
| 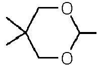 | -CH₂- |
| 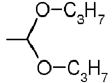 | -CH₂- |
| 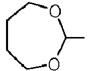 | -CH₂- |
| 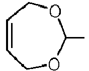 | -CH₂- |
| 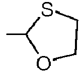 | -CH₂- |
| 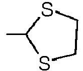 | -CH₂- |
| 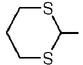 | -CH₂- |
| 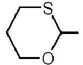 | -CH₂- |
| -CN | -CH₂- |
| -Cl | -CH₂- |
| -Br | -CH₂- |
| -SCN | -CH₂- |
| -CH=N-OH | -CH₂- |
| -CH=N-OCH₃ | -CH₂- |
| -CH=N-O-CH₂C=CH₂ | -CH₂- |
| -CH=N-O-CH₂C=CCl₂ | -CH₂- |
| -CH=N-O-CH₂C≡CH | -CH₂- |
| -CH=N-OⁱPr | -CH₂- |
| 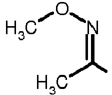 | -CH₂- |
| -SCH₂CH=CH₂ | -CH₂- |
| -SCH₃ | -CH₂- |
| -SO₂CH₃ | -CH₂- |
| CH₃(C=O)S- | -CH₂- |
| (CH₃)₂CHCH₂SO₂- | -CH₂- |
| (CH₃)₂CHCH₂S- | -CH₂- |
| 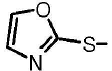 | -CH₂- |
| 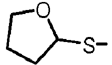 | -CH₂- |
| 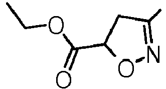 | -CH₂- |
| -OCH₂CH₃ | -CH=CH- |
| -O(C=O)-OCH₃ | -CH=CH- |
| -O(C=O)-OC₂H₅ | -CH=CH- |
| -O(C=O)-N(CH₃)₂ | -CH=CH- |
| -O(C=O)-NHC₆H₅ | -CH=CH- |
| -O(C=O)-NHCH₃ | -CH=CH- |
| -O(C=S)-NHC₆H₅ | -CH=CH- |
| -CO₂H | -CH=CH- |
| -CO₂Me | -CH=CH- |
| -CO₂Et | -CH=CH- |
| -CO₂ⁱPr | -CH=CH- |
| -CONH₂ | -CH=CH- |
| 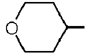 | -CH=CH- |
| 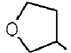 | -CH=CH- |
| 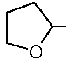 | -CH=CH- |
| 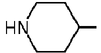 | -CH=CH- |
| 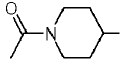 | -CH=CH- |
| 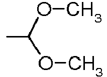 | -CH=CH- |
| 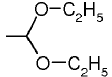 | -CH=CH- |
| 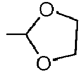 | -CH=CH- |
| 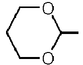 | -CH=CH- |
| 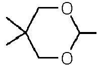 | -CH=CH- |
| 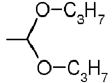 | -CH=CH- |
| 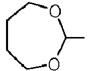 | -CH=CH- |
| 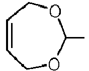 | -CH=CH- |
| 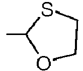 | -CH=CH- |
| 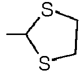 | -CH=CH- |
| 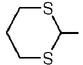 | -CH=CH- |
| 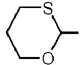 | -CH=CH- |
| -CN | -CH₂CH₂- |
| CH₃(C=O)- | -CH₂CH₂- |
| 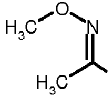 | -CH₂CH₂- |
| -CHO | -CH₂CH₂- |
| -OCH₃ | -CH₂CH₂- |
| -OCH₂CH₃ | -CH₂CH₂- |
| -O(C=O)-OCH₃ | -CH₂CH₂- |
| -O(C=O)-OC₂H₅ | -CH₂CH₂- |
| -O(C=O)-N(CH₃)₂ | -CH₂CH₂- |
| -O(C=O)-NHC₆H₅ | -CH₂CH₂- |
| -O(C=O)-NHCH₃ | -CH₂CH₂- |
| -O(C=S)-NHC₆H₅ | -CH₂CH₂- |
| -CO₂H | -CH₂CH₂- |
| -CO₂Me | -CH₂CH₂- |
| -CO₂Et | -CH₂CH₂- |
| -CO₂ⁱPr | -CH₂CH₂- |
| -CONH₂ | -CH₂CH₂- |
| 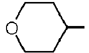 | -CH₂CH₂- |
| 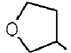 | -CH₂CH₂- |
| 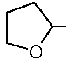 | -CH₂CH₂- |
| 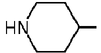 | -CH₂CH₂- |
| 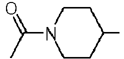 | -CH₂CH₂- |
| 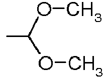 | -CH₂CH₂- |
| 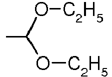 | -CH₂CH₂- |
| 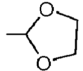 | -CH₂CH₂- |
| 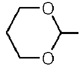 | -CH₂CH₂- |
| 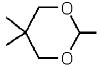 | -CH₂CH₂- |
| 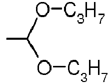 | -CH₂CH₂- |
| 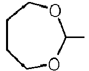 | -CH₂CH₂- |
| 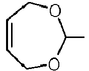 | -CH₂CH₂- |
| 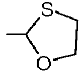 | -CH₂CH₂- |
| 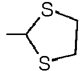 | -CH₂CH₂- |
| 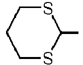 | -CH₂CH₂- |
| 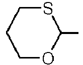 | -CH₂CH₂- |

- Tabelle 2:: Q und Y wie in Tabelle 1 genannt und
R¹ = C₂H₅, R² = CH₃, R³ = CH₃, R⁴ = H, R⁵ = H,
- Tabelle 3:: Q und Y wie in Tabelle 1 genannt und
R¹ = C₂H₅, R² = CH₃, R³ = C₂H₅, R⁴ = H, R⁵ = H,
- Tabelle 4:: Q und Y wie in Tabelle 1 genannt und
R¹ = C₂H₅; R² = C₂H₅; R³ = C₂H₅, R⁴ = H, R⁵ = H,
- Tabelle 5:: Q und Y wie in Tabelle 1 genannt und
R¹ = 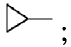 R² = CH₃; R³ = CH₃; R⁴ = H, R⁵ = H,
- Tabelle 6:: Q und Y wie in Tabelle 1 genannt und
R¹ = 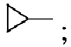 R² = CH₃; R³ = C₂H₅, R⁴ = H, R⁵ = H,
- Tabelle 7:: Q und Y wie in Tabelle 1 genannt und
R¹ = OCH₃; R² = CH₃; R³ = CH₃, R⁴ = H, R⁵ = H,
- Tabelle 8:: Q und Y wie in Tabelle 1 genannt und
R¹ = OC₂H₅; R² = CH₃; R³ = CH₃, R⁴ = H, R⁵ = H,
- Tabelle 9:: Q und Y wie in Tabelle 1 genannt und
R¹ = OCH₃; R² = CH₃; R³ = C₂H₅, R⁴ = H, R⁵ = H,
- Tabelle 10:: Q und Y wie in Tabelle 1 genannt und
R¹ = OC₂H₅; R² = CH₃; R³ = C₂H₅, R⁴ = H, R⁵ = H,
- Tabelle 11:: Q und Y wie in Tabelle 1 genannt und
R¹ = C₂H₅; R² = CH₃; R³ = H, R⁴ = H, R⁵ = H,
- Tabelle 12:: Q und Y wie in Tabelle 1 genannt und
R¹ = 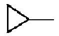 R² = CH₃; R³ = 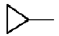 R⁴ = H, R⁵ = H,
- Tabelle 13:: Q und Y wie in Tabelle 1 genannt und
R¹ = C₂H₅; R² = C₂H₅; R³ = CH₃ , R⁴ = H, R⁵ = H,
- Tabelle 14:: Q und Y wie in Tabelle 1 genannt und
R¹ = CH₃; R² = 4-Chlorphenyl; R³ = H , R⁴ = CH₃, R⁵ = H.

Überraschenderweise wurde nun auch gefunden, dass die Verbindungen der Formel (I) bei gemeinsamer Anwendung mit den im weiteren beschriebenen, die Kulturpflanzen-Verträglichkeit verbessernden Verbindungen (Safener/Antidots) ausgesprochen gut die Schädigung der Kulturpflanzen verhindert und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher R¹, R², R³, R⁴, R⁵, Y, Q und G die oben angegebene Bedeutung haben
   und
(b') zumindest eine die Kulturpflanzen-Verträglichkeit verbessernde Verbindung (Safener).

Folgende Gruppen von Verbindungen kommen beispielsweise als Safener in Frage:
S1) Verbindungen der Formel (S1), 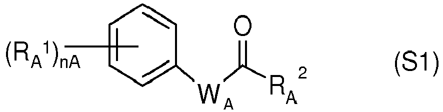 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁴), 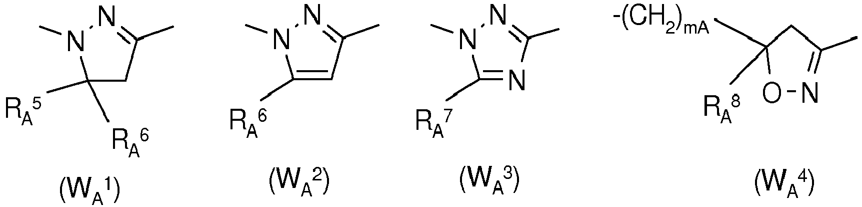
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-ethoxycarbonyl)-5-methyl-2-pyrazolin-3-carbonsäure, 1-(2,4-Dichlorphenyl)-5-ethoxycarbonyl)-5-methyl-2-pyrazolin-3-carbon-säureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-richlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 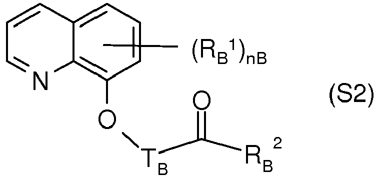 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 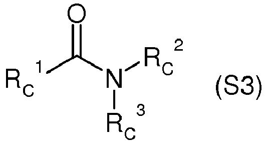 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6),
      "AD-67" oder "MON 4660" (3-Dichloracetyl-1-oxa-3-aza-spiro[4,5]decan) der Firma Nitrokemia bzw. Monsanto (S3-7),
      "TI-35" (1-Dichloracetyl-azepan) der Firma TRI-Chemical RT (S3-8),
      "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
      "Furilazol" oder "MON 13900" ((RS)-3-Dichloracetyl-5-(2-furyl)-2,2-dimethyloxazolidin)
      (S3-10); sowie dessen (R)-Isomer (S3-11).
S4) N-Acylsulfonamide der Formel (S4) und ihre Salze, 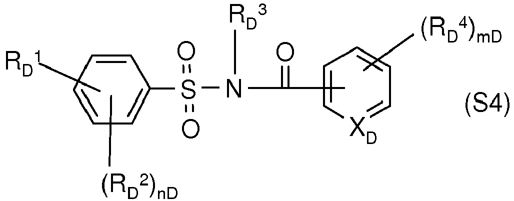 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0, 1, 2 oder 3;
davon bevorzugt sind Verbindungen von Typ der N-Acylsulfonamide, z.B. der nachfolgenden Formel (S4^{a}), die z. B. bekannt sind aus WO-A-97/45016 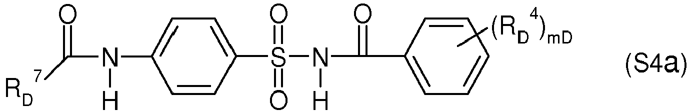 worin
- R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
- R_{D}⁴: Halogen, (C₁-C₄)Alkyl, (C₁-C₄)Alkoxy, CF₃;
- m_{D}: 1 oder 2;
- v_{D}: ist 0, 1, 2 oder 3 bedeutet;
sowie

Acylsulfamoylbenzoesäureamide, z.B. der nachfolgenden Formel (S4^{b}), die z.B. bekannt sind aus WO-A-99/16744, 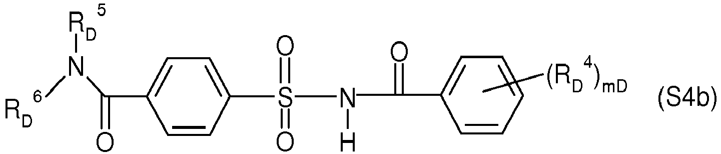 worin
- R_{D}⁵, R_{D}⁶: unabhängig voneinander Wasserstoff, (C₁-C₆)Alkyl, (C₃-C₆)Cycloalkyl, (C₃-C₆)Alkenyl, (C₃-C₆)Alkinyl,
- R_{D}⁴: Halogen, (C₁-C₄)Alkyl, (C₁-C₄)Haloalkyl oder (C₁-C₄)Alkoxy und
- m_{D}: 1 oder 2 bedeuten,
z.B. solche worin
R_{D}⁵ = Cyclopropyl und (R_{D}⁴) = 2-OMe ist ("Cyprosulfamide", S4-1),
R_{D}⁵ = Cyclopropyl und (R_{D}⁴) = 5-Cl-2-OMe ist (S4-2),
R_{D}⁵ = Ethyl und (R_{D}⁴) = 2-OMe ist (S4-3),
R_{D}⁵ = Isopropyl und (R_{D}⁴) = 5-Cl-2-OMe ist (S4-4) und
R_{D}⁵ = Isopropyl und (R_{D}⁴) = 2-OMe ist (S4-5)
sowie

Verbindungen vom Typ der N-Acylsulfamoylphenylharnstoffe der Formel (S4^{c}), die z.B. bekannt sind aus der EP-A-365484, 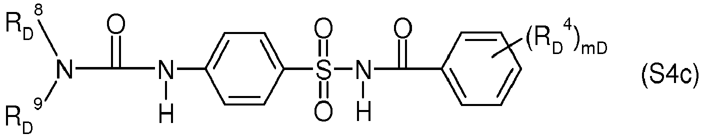 worin
- R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
- R_{D}⁴: Halogen, (C₁-C₄)Alkyl, (C₁-C₄)Alkoxy, CF₃
- m_{D}: 1 oder 2 bedeutet;
beispielsweise
1-[4-(N-2-Methoxybenzoylsulfamoyl)phenyl]-3-methylharnstoff,
1-[4-(N-2-Methoxybenzoylsulfamoyl)phenyl]-3,3-dimethylharnstoff,
1-[4-(N-4,5-Dimethylbenzoylsulfamoyl)phenyl] -3-methylharnstoff.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 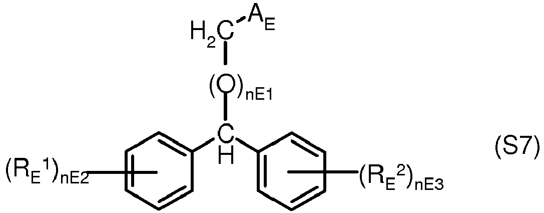 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8),wie sie in der WO-A-98/27049 beschrieben sind 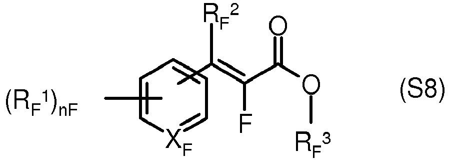 worin
   - X_{F}: CH oder N,
   - n_{F}: für den Fall, dass X_{F}=N ist, eine ganze Zahl von 0 bis 4 und
   für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
   vorzugsweise Verbindungen worin
   - X_{F}: CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 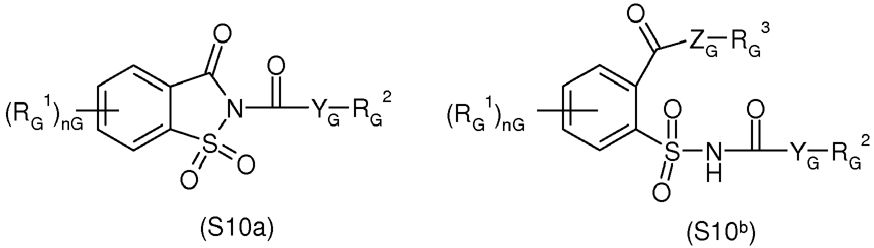 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B. "Oxabetrinil" ((Z)-1,3- Dioxolan--ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg.Nr. 133993-74-5) (2-propenyl 1-oxa-4-azaspiro[4.5]decane-4-carbodithioate) (S 13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 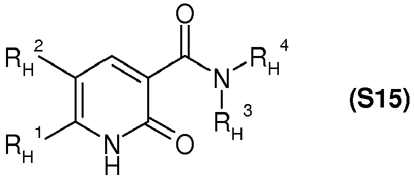 wie sie in der WO-A-2008/131861 und WO-A-2008/131860 beschrieben sind
   worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Als die die Kulturpflanzen-Verträglichkeit verbessernde Verbindung sind Cloquintocet-mexyl, Fenchlorazol-ethylester, Isoxadifen-ethyl, Mefenpyr-diethyl, Fenclorim, Cumyluron, S4-1 und S4-5 am meisten bevorzugt, wobei Cloquintocet-mexyl und Mefenpyr-diethyl besonders hervorgehoben seien.

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die Herstellung der Verbindungen der Formel (I) kann erfindungsgemäß nach den Verfahren A bis H erfolgen.

Verwendet man beispielsweise gemäß Verfahren (A) Methyl-2-[(2,6-diethyl-4-methylphenyl)-acetyl]-4-(1,3-dioxolan-2-yl)cyclopentancarboxylat, so kann der Verlauf des erfindungsgemäßen Verfahrens durch folgendes Reaktionsschema wiedergegeben werden:

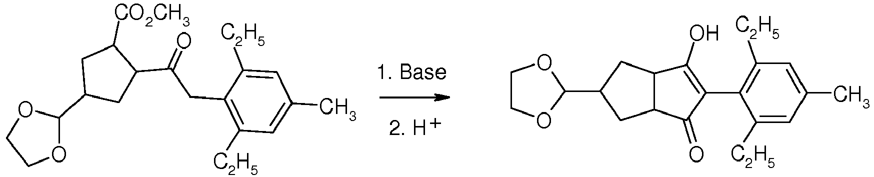

Verwendet man beispielsweise gemäß Verfahren (B) 2-(2,6-Diethyl-4-methylphenyl)-5-(1,3-dioxolan-2-yl)-3-hydroxy-4,5,6,6a-tetrahydropentalen-1(3aH)-on und Pivaloylchlorid als Ausgangsstoffe, so kann der Verlauf des erfindungsgemäßen Verfahrens durch folgendes Reaktionsschema wiedergegeben werden:

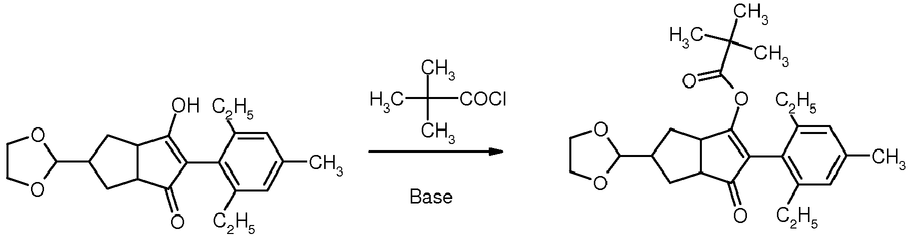

Verwendet man beispielsweise gemäß Verfahren (B) 2-(2,6-Diethyl-4-methylphenyl)-5-(1,3-dioxolan-2-yl)-3-hydroxy-4,5,6,6a-tetrahydropentalen-1(3aH)-on und Acetanhydrid als Ausgangsverbindungen, so kann der Verlauf des erfindungsgemäßen Verfahrens durch folgendes Reaktionsschema wiedergegeben werden:

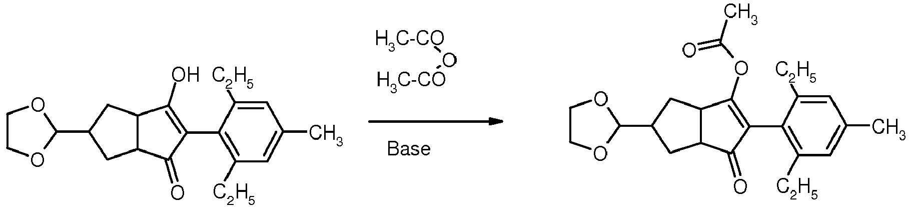

Verwendet man beispielsweise gemäß Verfahren (C) 2-(2,6-Diethyl-4-methylphenyl)-5-(1,3-dioxolan-2-yl)-3-hydroxy-4,5,6,6a-tetrahydropentalen-1(3aH)-on und Chlorameisensäureethyl-ester als Ausgangsverbindungen, so kann der Verlauf des erfindungsgemäßen Verfahrens durch folgendes Reaktionsschema wiedergegeben werden:

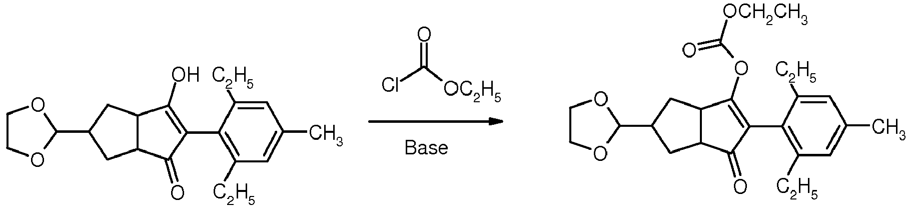

Verwendet man beispielsweise gemäß Verfahren (D) 2-(2,6-Diethyl-4-methylphenyl)-5-(1,3-dioxolan-2-yl)-3-hydroxy-4,5,6,6a-tetrahydropentalen-1(3aH)-on und Chlormonothioameisen-säuremethylester als Ausgangsverbindungen, so kann der Verlauf des erfindungsgemäßen Verfahrens durch folgendes Reaktionsschema wiedergegeben werden:

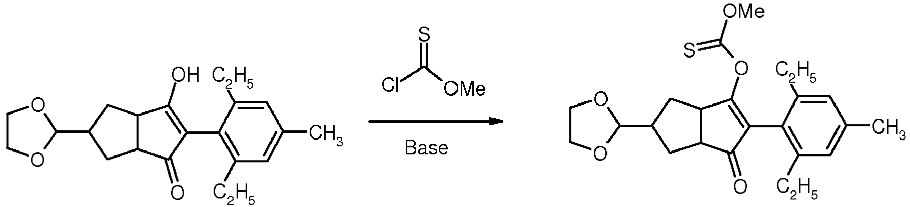

Verwendet man beispielsweise gemäß Verfahren (E) 5'-(2,6-Diethyl-4-methylphenyl)-6'-hydroxy-2,2-diemethyl-1',3',3a',6a'-tetrahydro-4'H-spiro[1,3-dioxolan-4,2'-pentalen]-4'on und Methan-sulfonsäurechlorid als Ausgangsprodukt, so kann der Reaktionsverlauf durch folgendes Reaktionsschema wiedergegeben werden:

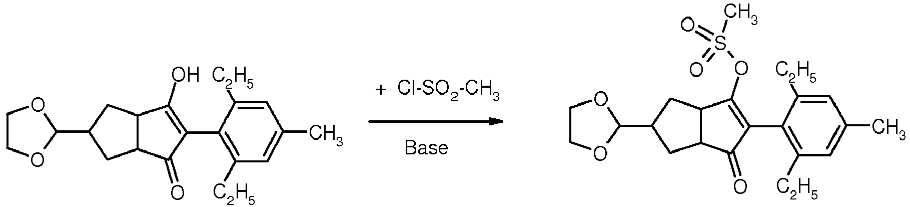

Verwendet man beispielsweise gemäß Verfahren (F) 2-(2,6-Diethyl-4-methylphenyl)-5-(1,3-dioxolan-2-yl)-3-hydroxy-4,5,6,6a-tetrahydropentalen-1(3aH)-on und Methanthiophosphonsäure-chlorid-(2,2,2-trifluorethylester) als Ausgangsprodukte, so kann der Reaktionsverlauf durch folgendes Reaktionsschema wiedergegeben werden:

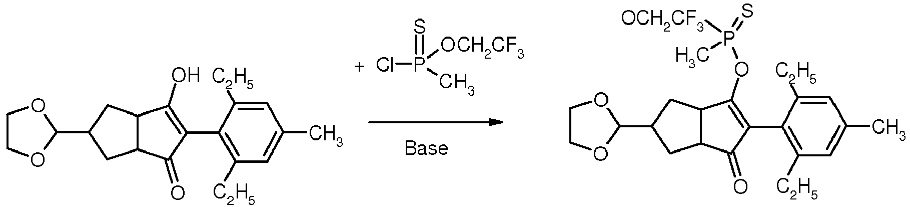

Verwendet man beispielsweise gemäß Verfahren (G) 2-(2,6-Diethyl-4-methylphenyl)-5-(1,3-dioxolan-2-yl)-3-hydroxy-4,5,6,6a-tetrahydropentalen-1(3aH)-on und NaOH als Komponenten, so kann der Verlauf des erfindungsgemäßen Verfahrens durch folgendes Reaktionsschema wiedergegeben werden:

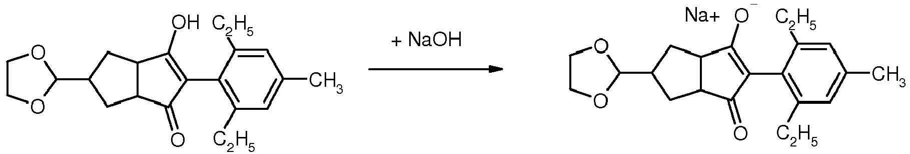

Verwendet man beispielsweise gemäß Verfahren (H) Variante α 2-(2,6-Diethyl-4-methylphenyl)-5-(1,3-dioxolan-2-yl)-3-hydroxy-4,5,6,6a-tetrahydropentalen-1(3aH)-on und Ethylisocyanat als Ausgangsprodukte, so kann der Reaktionsverlauf durch folgendes Reaktionsschema wiedergegeben werden:

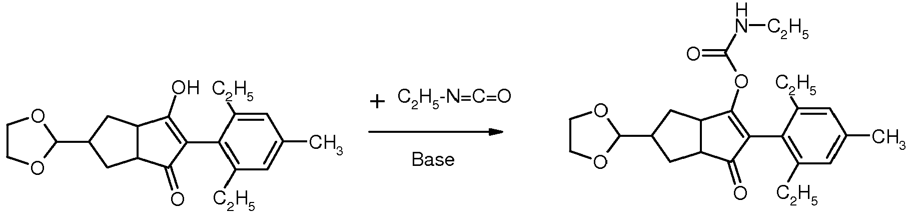

Verwendet man beispielsweise gemäß Verfahren (H) Variante ß 2-(2,6-Diethyl-4-methylphenyl)-5-(1,3-dioxolan-2-yl)-3-hydroxy-4,5,6,6a-tetrahydropentalen-1(3aH)-on und Dimethylcarbamidsäu-rechlorid als Ausgangsprodukte, so kann der Reaktionsverlauf durch folgendes Schema wiedergegeben werden:

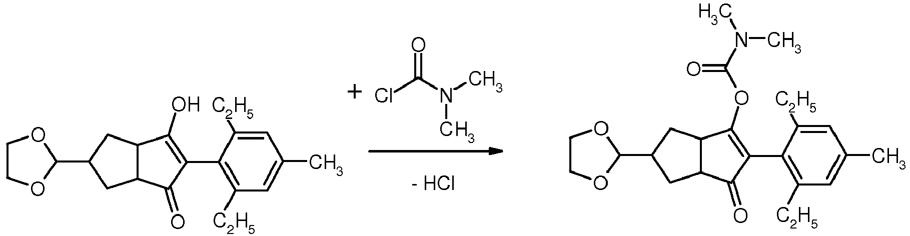

Die bei dem erfindungsgemäßen Verfahren (A) als Ausgangsstoffe benötigten Verbindungen der Formel (II) 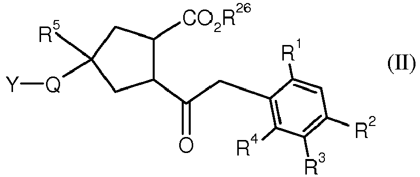 in welcher
- Q, Y, R¹, R², R³, R⁴ und R⁵: die oben angegebene Bedeutung haben, und
- R²⁶: für Alkyl (insbesondere C₁-C₈-Alkyl) steht,
sind neu. Sie lassen sich nach im Prinzip bekannten Methoden herstellen, indem man 5-Aryl-4-ketocarbonsäuren der Formel (XIII) 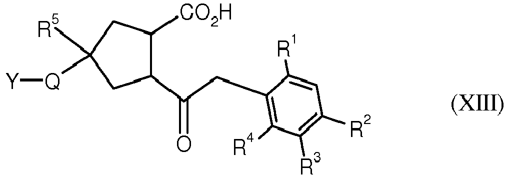 in welcher
- Q, Y, R¹, R², R³, R⁴ und R⁵: die oben angegebene Bedeutung haben,
in üblicher Weise verestert (vgl. z.B. Organikum, 15. Auflage, Berlin, 1977, Seite 499 oder Herstellungsbeispiel).

Verbindungen der Formel (XIII) sind ebenfalls neu, sie lassen sich nach im Prinzip bekannten Methoden herstellen (WO 07/080066, WO 96/01 798, WO 97/14667, WO 98/39281, WO 01/74770), beispielsweise indem man 2-Phenyl-3-oxo-adipinsäureester der Formel (XIV) 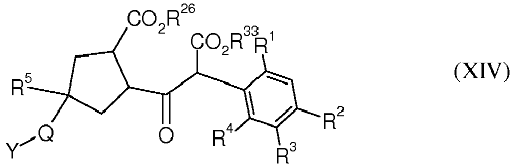 in welcher Q, Y, R¹, R², R³, R⁴ und R⁵ R²⁶ die oben angegebene Bedeutung haben,
- R²⁶: für Alkyl (insbesondere C₁-C₈-Alkyl) oder, im Falle der Herstellung aus den Anhydriden der Formel (XVI) für Wasserstoff steht,
- R³³: für Alkyl (insbesondere C₁-C₈-Alkyl) steht,
gegebenenfalls in Gegenwart eines Verdünnungsmittels und gegebenenfalls in Gegenwart einer Base oder Säure nach üblichen Verfahren decarboxyliert (vgl. z.B. Organikum, 15. Auflage, Berlin, 1977, Seite 519 bis 521).

Die Verbindungen der Formel (XIV) sind im Prinzip ebenfalls neu und können in Anlehnung an bekannte Vewrfahren hergestellt werden. Man erhält die Verbindungen der Formel (XIV) beispielsweise, indem man Dicarbonsäurehalbesterchloride der Formel (XV), 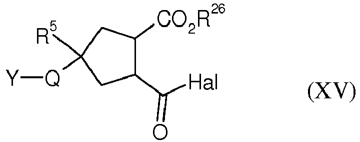 in welcher
Q, Y, R und R⁵ die oben angegebene Bedeutung haben und
Hal für Chlor oder Brom steht,
oder Carbonsäureanhydride der Formel (XVI) 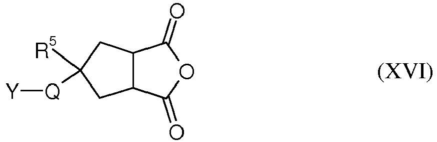 in welcher
Q, Y und R⁵ die oben angegebene Bedeutung haben,
mit einem Phenylessigsäureester der Formel (XVII) 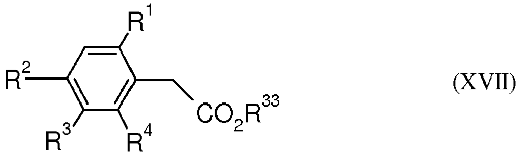 in welcher
R, R¹, R², R³, R⁴, R⁵ und R³³ die oben angegebene Bedeutung haben, in Gegenwart eines Verdünnungsmittels und in Gegenwart einer Base zur Reaktion bringt. (vgl. z.B. M.S. Chambers, E. J. Thomas, D.J. Williams, J. Chem. Soc. Chem. Commun., (1987), 1228.

Eine weitere bewährte Methode zur Herstellung der beim Verfahren (A) als Ausgangsstoffe benötigten Verbindungen der Formel (II),
in welcher
Q, Y, R¹, R², R³, R⁴ und R⁵ die oben angegebene Bedeutung haben,
ist beispielsweise auch die Kupplung von Benzylzinkverbindungen der Formel (XVIII) 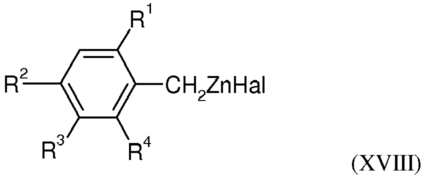 in welcher R¹, R³, R³ und R⁴ die oben angegebene Bedeutung haben und Hal für ein Halogenatom, vorzugsweise Chlor oder Brom, steht,
gegebenenfalls in Gegenwart eines Katalysators, mit einem Dicarbonsäurehalbesterchlorid der Formel (XV) oder einem Carbonsäureanhydrid der Formel (XVI).

Sowohl Herstellung als auch Umsetzung von organischen Zinkverbindungen mit Carbonsäurechloriden bzw. mit Carbonsäureanhydriden sind im Prinzip bekannt und können in enger Anlehnung an die in der Literatur beschriebenen Verfahren durchgeführt werden. Weitere Einzelheiten dazu sind beispielsweise in Chem. Commun. 2008, 5824, WO 2007/113294, WO 2010/040460, Tetrahedron Letters 30, 5069-5072 (1989) oder Chem. Rev. 1993, 93, 2117-2188 beschrieben.

Die zur Durchführung der erfindungsgemäßen Verfahren (B), (C), (D), (E), (F), (G) und (H) außerdem als Ausgangsstoffe benötigten Säurehalogenide der Formel (III), Carbonsäureanhydride der Formel (IV), Chlorameisensäureester oder Chlorameisensäurethioester der Formel (V), Chlormonothioameisensäureester oder Chlordithioameisensäureester der Formel (VI), Sulfonsäurechloride der Formel (VII), Phosphorverbindungen der Formel (VIII) und Metallhydroxide, Metallalkoxide oder Amine der Formel (IX) und (X) und Isocyanate der Formel (XI) und Carbamidsäurechloride der Formel (XII) sind allgemein bekannte Verbindungen der Organischen bzw. Anorganischen Chemie.

Die Verbindungen der Formeln (XV), (XVI), (XVII) und (XVIII) sind bekannte Verbindungen der Organischen Chemie bzw. aus den eingangs zitierten Patentanmeldungen bekannt und/oder lassen sich nach im Prinzip bekannten Methoden in einfacher Weise herstellen bzw. lassen sich nach den in den eingangs zitierten Patentanmeldungen angegebenen Methoden herstellen.

Das Verfahren (A) ist dadurch gekennzeichnet, dass man Verbindungen der Formel (II), in welcher Q, Y, R¹, R², R³, R⁴ und R⁵ die oben angegebene Bedeutung haben, in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetallund Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, molaren Mengen ein. Es ist jedoch auch möglich, die eine oder andere Komponente in einem größeren Überschuss (bis zu 3 Mol) zu verwenden.

Das Verfahren (B-α) ist dadurch gekennzeichnet, dass man Verbindungen der Formel (I-a) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (B-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (B-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (B-α) werden die Ausgangsstoffe der Formel (I-a) und das Carbonsäurehalogenid der Formel (III) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (B-ß) ist dadurch gekennzeichnet, dass man Verbindungen der Formel (I-a) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (B-ß) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (B-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (B-ß) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (B-ß) werden die Ausgangsstoffe der Formel (I-a) und das Carbonsäureanhydrid der Formel (IV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, dass man Verbindungen der Formel (I-a) jeweils mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im Allgemeinen zwischen - 20°C und +100°C, vorzugsweise zwischen 0°C und 50°C.

Das erfindungsgemäße Verfahren (C) wird im Allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethiolester der Formel (V) im Allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (D) ist dadurch gekennzeichnet, dass man Verbindungen der Formel (I-a) jeweils mit Verbindungen der Formel (VI) in Gegenwart eines Verdünnungsmittels und gegebenenfalls in Gegenwart eines Säurebindemittels umsetzt.

Beim Herstellungsverfahren (D) setzt man pro Mol Ausgangsverbindung der Formel (I-a) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (E) ist dadurch gekennzeichnet, dass man Verbindungen der Formel (I-a) jeweils mit Sulfonsäurechloriden der Formel (VII) gegebenenfalls in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formel (I-a) ca. 1 Mol Sulfonsäurechlorid der Formel (VII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formel (I-a) jeweils mit Phosphorverbindungen der Formel (VIII) gegebenenfalls in Gegenwart eines Verdünnungsmittels und gegebenenfalls in Gegenwart eines Säurebindemittels umsetzt.

Beim Herstellungsverfahren (F) setzt man zum Erhalt von Verbindungen der Formel (I-e) auf 1 Mol der Verbindungen der Formel (I-a), 1 bis 2, vorzugsweise 1 bis 1,3 Mol der Phosphorverbindung der Formel (VIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (G) ist dadurch gekennzeichnet, dass man Verbindungen der Formel (I-a) mit Metallhydroxiden bzw. Metallalkoxiden der Formel (IX) oder Aminen der Formel (X), gegebenenfalls in Gegenwart eines Verdünnungsmittels, umsetzt.

Als Verdünnungsmittel können bei dem erfindungsgemäßen Verfahren (G) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (G) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (H) ist dadurch gekennzeichnet, dass man Verbindungen der Formel (I-a) jeweils mit (H-α) Verbindungen der Formel (XI) gegebenenfalls in Gegenwart eines Verdünnungsmittels und gegebenenfalls in Gegenwart eines Katalysators oder (H-ß) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (H-α) setzt man pro Mol Ausgangsverbindung der Formel (I-a) ca. 1 Mol Isocyanat der Formel (XI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (H-ß) setzt man pro Mol Ausgangsverbindung der Formel (I-a) ca. 1 Mol Carbamidsäurechlorid der Formel (XII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, WO 07/068427). Eine entsprechende Wirkungssteigerung bei Insektiziden wurde bereits durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun ebenfalls überraschend gefunden, dass sich die Wirkung von Insektiziden und/oder Akariziden und/oder Herbiziden aus der Klasse der phenylsubstituierten Bicyclooktan-1,3-dion-Derivate der Formel (I) durch den Zusatz von Ammonium- oder Phosphoniumsalzen zur Anwendungslösung oder durch den Einbau dieser Salze in eine Formulierung enthaltend phenylsubstituierte Bicyclooktan-1,3-dion-Derivate der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die herbizid und/oder insektizid und/oder akarizid wirksame phenylsubstituierte Bicyclooktan-1,3-dion-Derivate der Formel (I) als Wirkstoff enthalten. Gegenstand der Erfindung sind ebenfalls Mittel, die herbizid und/oder akarizid und/oder insektizid wirksame phenylsubstituierte Bicyclooktan-1,3-dion-Derivate der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Fettsäure-Biosynthese-Inhibitoren steigern, werden durch Formel (IIIʹ) definiert 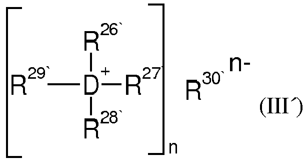 in welcher
- D: für Stickstoff oder Phosphor steht,
- D: bevorzugt für Stickstoff steht,
- R^{26´} , R^{27´} , R^{28´} und R^{29´}: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R^{26`}, R^{27`}, R^{28`} und R^{29`}: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R^{26`}, R^{27`}, R^{28`} und R^{29`}: besonders bevorzugt unabhängig voneinander für Wasserstoff, Methyl, Ethyl, n-Propyl, i-Propyl, n-Butyl, i-Butyl, s-Butyl oder t-Butyl stehen,
- R^{26`}, R^{27`}, R^{28`} und R^{29`}: ganz besonders bevorzugt für Wasserstoff stehen,
- n: für 1, 2, 3 oder 4 steht,
- n: bevorzugt für 1 oder 2 steht,
- R^{30`}: für ein anorganisches oder organisches Anion steht,
- R^{30`}: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R^{30`}: besonders bevorzugt für Laktat, Sulfat, Nitrat, Thiosulfat, Thiocyanat, Oxalat oder Formiat steht,
- R^{30`}: ganz besonders bevorzugt für Sulfat steht.

Die Ammonium- und Phosphoniumsalze der Formel (IIIʹ) können in einem breiten Konzentrationsbereich zur Steigerung der Wirkung von Pflanzenschutzmitteln enthaltend phenylsubstituierten Bicyclooktan-1,3-dion-Derivate der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder herbizid wirksame, phenylsubstituierte Bicyclooktan-1,3-dion-Derivate der Formel (I) als Wirkstoff enthalten. Gegenstand der Erfindung sind ebenfalls Mittel, die herbizid und/oder akarizid und/oder insektizid wirksame phenylsubstituierten Bicyclooktan-1,3-dion-Derivate der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässrigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV´ )

R-O-(-AO)ᵥ-R' (IV`)

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV´ -a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV`-b)

in welcher
- R: die oben angegebene Bedeutung hat,
- R': die oben angegebene Bedeutung hat,
- EO: für -CH₂-CH₂-O- steht,

- PO: für 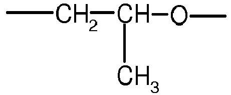 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV´ -c)

in welcher
- R: die oben angegebene Bedeutung hat,
- R': die oben angegebene Bedeutung hat,
- EO: für -CH₂-CH₂-O- steht,
- PO: für 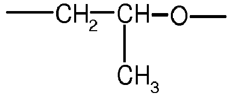 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV´ -d)

in welcher
- R und R': die oben angegebenen Bedeutungen haben,
- EO: für -CH₂-CH₂-O- steht,
- BO: für 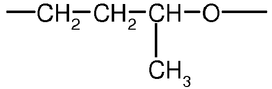 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV`-e)

in welcher
- R und R': die oben angegebenen Bedeutungen haben,
- BO: für 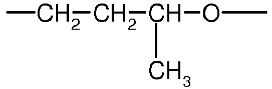 steht,
- EO: für -CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃₋(CH₂)ₜ-CH₂-O-(-CH₂₋CH₂-O-)ᵤ-R' (IV`-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 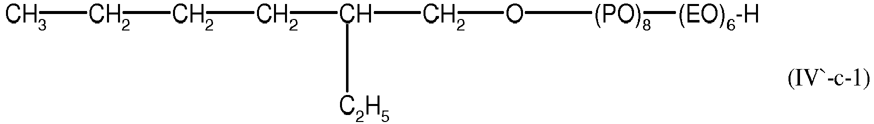 in welcher
- EO: für -CH₂-CH₂-O- steht,
- PO: für 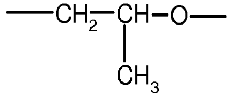 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV`-d-1)

in welcher
- EO: für -CH₂-CH₂-O- steht,
- BO: für 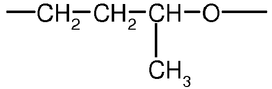 steht und
die Zahlen 10, 6 und 2 Durchschnittswerte darstellen, genannt.

Besonders bevorzugte Alkanol-Alkoxylate der Formel (IV`-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV`-f-1)

CH₃-(CH₂)ₜ-CH₂-O-(-CH₂-CH₂-O-)ᵤ-H (IV´ -f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxidpolypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxypropoxylate, wobei Sorbitan-Derivate, wie Polyethylenoxid-Sorbitan-Fettsäureester und Sorbitan-Fettsäureester, beispielhaft genannt seien.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp.,

Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae,

Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP-POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Bezeichnung "Wirkstoffe" bzw. "Verbindungen" schließt immer auch die hier genannten Wirkstoffkombinationen mit ein.

Die Herstellung und die Verwendung der erfindungsgemäßen Wirkstoffe geht aus den nachfolgenden Beispielen hervor.

### Herstellunssbeispiele

### Beispiele I-a-0 und I-a-1

### 3-(Benzyloxy)-2-(2,6-diethyl-4-methylphenyl)-5-methylen-4,5,6,6a-tetrahydropentalen-1(3aH)-on und 3-(Benzyloxy)-2-(2,6-diethyl-4-methylphenyl)-5-(hydroxymethyl)-4,5,6,6a-tetrahydropentalen-1(3aH)-on

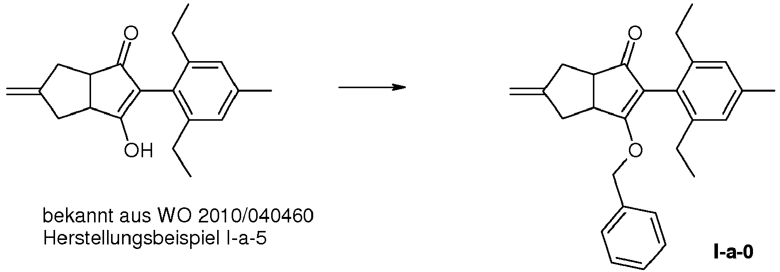

6.80 g (22.94 mMol) 2-(2,6-Diethyl-4-methylphenyl)-3-hydroxy-5-methylen-4,5,6,6a-tetrahydropentalen-1(3aH)-on (Herstellungsbeispiel I-a-5 bekannt aus WO 2010/040460), 8.71 g (68.82 mMol) Benzylchlorid und 9.51 g Kaliumcarbonat werden in 50 ml Aceton 2 h am Rückfluß erhitzt, anschließend das Lösungsmittel abdestilliert und der Rückstand an Kieselgel mit Essigsäureethylester/Hexan 1:4 chromatographiert. Man erhält so 6.40 g (72%) 3-(Benzyloxy)-2-(2,6-diethyl-4-methylphenyl)-5-methylen-4,5,6,6a-tetrahydropentalen-1(3aH)-on in Form farbloser Kristalle mit Fp. 87-88 °C.

¹H-NMR (400 MHz, CDCl₃): δ = 1.03 und 1.12 (je t, je 3H), 3.03 und 3.37 (je mc, je 1H), AB-System: δ_{A} = 4.73, δ_{B} = 4.79, J = 13 Hz (Benzyl-CH₂), 4.90 (mc, 2H), 6.88 und 7.12 (je mc, je 2H), 7.32 (mc, 3H)

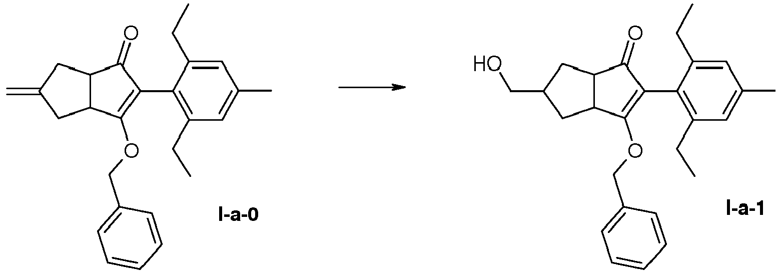

Zu 6.00 g (15.52 mMol) 3-(Benzyloxy)-2-(2,6-diethyl-4-methylphenyl)-5-methylen-4,5,6,6a-tetrahydropentalen-1(3aH)-on in 100 ml THF (Tetrahydrofuran) werden innerhalb von 30 Min. 17 ml Boran-THF-Komplex (1 molare Lösung, 17 mMol) zugetropft und anschließend noch 1h bei Raumtemperatur weitergerührt. Zu dieser Mischung werden 6 ml Wasser und 3 ml 3 molare Natronlauge zugegeben und anschließend 2.3 ml 35%iges Wasserstoffperoxid so zudosiert, dass die Innentemperatur zwischen 30 und 50 °C bleibt. Es wird noch 1h bei Raumtemperatur nachgerührt, danach einrotiert, in Dichlormethan aufgenommen, zweimal mit Wasser gewaschen, getrocknet (Magnesiumsulfat) und das Lösemittel abdestilliert. Chromatographie an Kieselgel (Essigsäureethylester/Hexan 1:4) ergibt 4.39 g (70%) des gewünschten Produktes (I-a-1) als viskoses, farbloses Öl. Das Isomerenverhältnis syn-/anti beträgt lt. 1H-NMR ca. 85:15.

¹H-NMR (400 MHz, CDCl₃): δ = 1.10 (mc, 6H), 3.02-3.15 (m, 1H), 3.40 (mc, 1H), 3.55-3.69 (m, 2H), AB-System: δ_{A} = 4.78, δ_{B} = 4.82, J = 13 Hz (Benzyl-CH₂ anti-Isomer), 4.85 (s, Benzyl-CH₂ syn-Isomer, 6.88 (mc, 2H), 7.12 (mc, 2H), 7.30 (mc, 3H).

### Beispiel I-a-2

### 6-(Benzyloxy)-5-(2,6-diethyl-4-methylphenyl)-4-oxo-1,2,3,3a,4,6a-hexahydropentalen-2-carbaldehyd

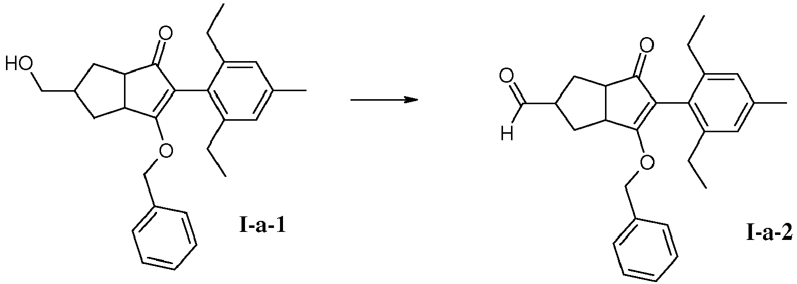

Zu 1.067 g (8.4 mMol) Oxalylchlorid in 40 ml Dichlormethan werden bei -78 °C langsam 1.31 g (16.8 mMol) Dimethylsulfoxid zugetropft und 20 Min. bei dieser Temperatur nachgerührt. Danach werden 1.70 g (4.20 mMol) 3-(Benzyloxy)-2-(2,6-diethyl-4-methylphenyl)-5-(hydroxymethyl)-4,5,6,6a-tetrahydro-pentalen-1(3aH)-on (Beispiel I-a-1), gelöst in 30 ml Dichlormethan, langsam zugetropft und eine weitere Stunde nachgerührt. Dann werden langsam 2.13 g (21 mMol) Triethylamin zugegeben und der Ansatz auf Raumtemperatur gebracht. Zugabe von 50 ml gesättigter Ammoniumchloridlösung, Abtrennen, Waschen der organischen Phase mit Wasser, Trocknen (Magnesiumsulfat), Einrotieren und chromatographische Reinigung des erhaltenen Rohprodukts an Kieselgel (Laufmittel Essigsäureethylester/Hexan 1:5) ergibt 1.24 g (73%) der Titelverbindung als farbloses Öl.

¹H-NMR (400 MHz, CDCl₃): δ = 1.08 und 1.12 (je t, je 3H), 2.30 (s, 3H), 2.90 mc, 1H), 3.12 (mc, 1H), 3.41 (mc, 1H), 4.72 (s, 2H), 6.89 (mc, 2H), 9.70 (mc, 1H).

### Beispiel I-a-3

### 5-(2,6-Diethyl-4-methylphenyl)-4,6-dioxooctahydropentalen-2-carbaldehyd

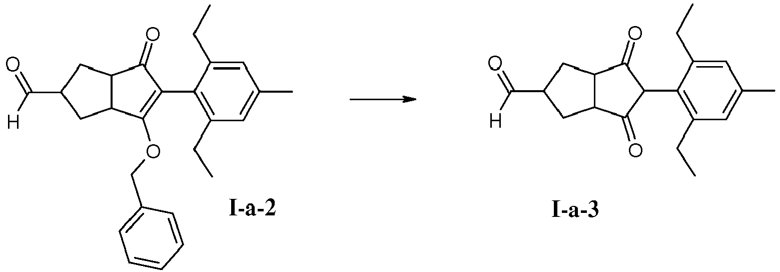

1.10 g (2.72 mMol) 6-(Benzyloxy)-5-(2,6-diethyl-4-methylphenyl)-4-oxo-1,2,3,3a,4,6a-hexahydropentalen-2-carbaldehyd in 20 ml Methanol werden mit 20 mg Palladium auf Aktivkohle (10%ig) versetzt und 20 h hydriert (15 bar, Raumtemperatur). Nach Abfiltrieren und einrotieren erhält man 0.82 g (97%) der Titelverbindung (I-a-3, Isomerenverhältnis lt. ¹H-NMR 4:1).

¹H-NMR (400 MHz, CDCl₃): δ = 1.05 (mc, 6H), 2.90 (mc, 1H), 3.09-3.40 (m, br, 2H), 6.91 (mc, 2H), 9.62 (mc, CHO Isomer 1), 9.68 (mc, CHO Isomer 2).

### Beispiel I-a-4

### 5-(2,6-Diethyl-4-methylphenyl)-4,6-dioxooctahydropentalen-2-carbonitril

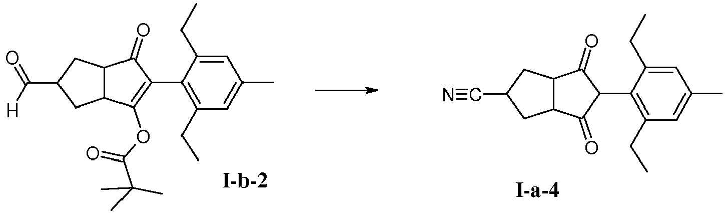

0.91 g (2.30 mMol) 2-(2,6-Diethyl-4-methylphenyl)-5-formyl-3-oxooctahydropentalen-1-y1-2,2-dimethylpropanoat (Beispiel I-b-2), 0.78 g (11.48 mMol) Natriumformiat und 0,80 g (11.48 mMol) Hydroxylamin-hydrochlorid werden in 30 ml Ameisensäure 72 h am Rückfluß erhitzt. Danach wird der Ansatz mit 50 ml Wasser versetzt, 1 h bei Raumtemperatur gerührt, mit Essigsäureethylester aufgenommen, zweimal mit Wasser gewaschen, getrocknet (Magnesiumsulfat) und einrotiert. Der Rückstand wird an Kieselgel (Laufmittel Essigsäure-ethylester/Hexan 1:6) chromatographiert. Man erhält 0.31 g (44%) der gewünschten Verbindung als farbloses Öl.

¹H-NMR (400 MHz, CDCl₃): δ = 1.08 und 1.11 (je t, je 3H), 2.00 (mc, 2H), 2.48 (mc, 2H), 2.99 (mc, 1H), 6.95 (mc, 2H).

### Beispiel I-a-5

### 2-(2,6-Diethyl-4-methylphenyl)-5-(1,3-dioxan-2-yl)tetrahydropentalen-1,3(2H,3aH)-dion

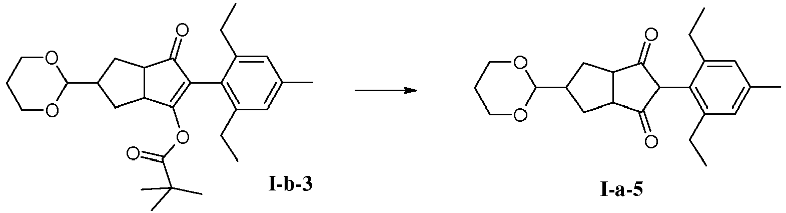

0.100 g (0.22 mMol) 2-(2,6-Diethyl-4-methylphenyl)-5-(1,3-dioxan-2-yl)-3-oxo-3,3a,4,5,6,6a-hexahydropentalen-1-yl-2,2-dimethylpropanoat in 10 ml Methanol und 4 ml 1N Natronlauge 2 h bei Raumtemperatur gerührt. Danach wird eingeengt, in Wasser aufgenommen, mit 2N HCl auf pH 4 angesäuert, mit Ethylacetat extrahiert, getrocknet (Magnesiumsulfat) und das Lösemittel abdestilliert. Chromatographische Reinigung des Rückstandes an Kieselgel (Laufmittel Essigsäure-ethylester/Hexan 1:6) liefert 70 mg (86%) der gewünschten Verbindung in Form farbloser Nadeln mit Fp. 100-101 °C.

### Beispiel I-a-82

### 2-(2,6-Diethyl-4-methyl-phenyl)-5-methoxymethyl-tetrahydro-pentalene-1,3-dion

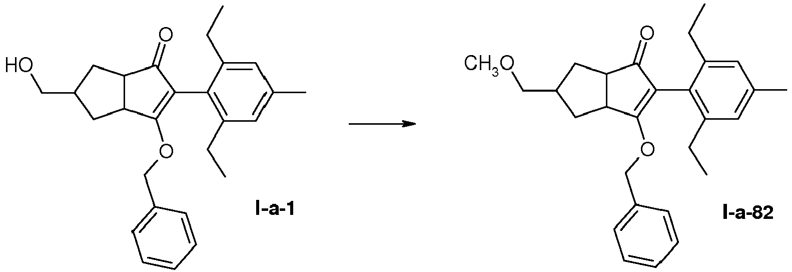

Zu 93 mg (2.32 mMol) Natriumhydrid in 20 ml Tetrahydrofuran werden 0.762 g (1.88 mMol) der Verbindung I-a-1 (3-(Benzyloxy)-2-(2,6-diethyl-4-methylphenyl)-5-(hydroxymethyl)-4,5,6,6a-tetrahydro-pentalen-1(3aH)-on) in 5 ml Tetrahydrofuran getropft und noch 30 Min. bei Raumtemperatur nachgerührt. Danach werden 309 mg (2.45 mMol) Dimethylsulfat, gelöst in 2 ml Tetrahydrofuran, langsam zugetropft und 2 h am Rückfluß erhitzt. Man nimmt in Essigsäureethylester auf, schüttelt zweimal mit Wasser aus, trocknet (Magnesiumsulfat) und destilliert das Lösungsmittel ab. Chromatographie an Kieselgel (Laufmittel Essigsäure-ethylester/Hexan 1:5) liefert 0.50 g (64%) der gewünschten Verbindung in Form farbloser Kristalle mit Fp. 75-76 °C.

### Beispiel I-a-29

### 2-(2,6-Diethyl-4-methyl-phenyl)-5-methoxymethyl-tetrahydro-pentalene-1,3-dion

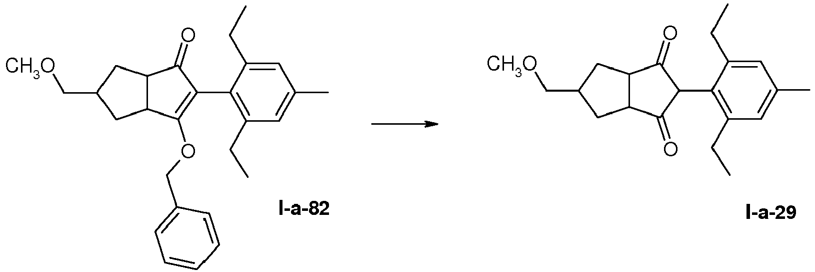

80 mg (0.19 mMol) der Verbindung I-a-82 werden in 10 ml Methanol nach Zugabe von 5 mg Katalysator (Pd/C, 5%ig) bei Raumtemperatur und einem Druck von 5 bar hydriert. Nach Filtration und Abdestillieren des Lösungsmittels erhält man 58 mg (92%) der gewünschten Verbindung I-a-29 als gelbliches Öl.

¹H-NMR (400 MHz, CDCl₃): δ = 1.08 und 1.11 (je t, je 3H), 2.00 (mc, 2H), 2.48 (mc, 2H), 2.99 (mc, 1H), 6.95 (mc, 2H).

### Beispiel I-a-39

### 2-(2,6-Diethyl-4-methyl-phenyl)-5-(3-oxo-butyl)-tetrahydropentalene-1,3-dion

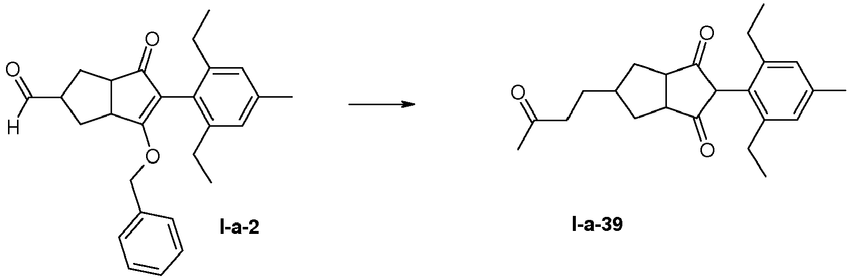

1.87 g (4.65 mMol) 6-(Benzyloxy)-5-(2,6-diethyl-4-methylphenyl)-4-oxo-1,2,3,3a,4,6a-hexahydropentalen-2-carbaldehyd (Verbindung I-a-2) und 1.63 g (5.11 mMol) Acetonylidentriphenylphosphoran werden in 40 ml Trichlormethan 12 h bei Raumtemperatur gerührt. Danach wird das Lösemittel entfernt und der Rückstand an Kieselgel (Laufmittel Essigsäure-ethylester/Hexan 1:5) chromatographisch gereinigt. Man erhält 1.37 g (66%) 3-Benzyloxy-2-(2,6-diethyl-4-methyl-phenyl)-5-(3-oxo-but-1-enyl)-4,5,6,6a-tetrahydro-3aH-pentalen-1-on als farbloses Öl.

¹H-NMR (400 MHz, CDCl₃): δ = 1.10 (mc, 6H), 2.25 (s, 3H), 2.31s, 3H), 2.93, 3.19 und 3.44 (je mc, je 1H), 4.80 (s, H), 6.12 (d, 1H), 6.74 (dd, 1H), 6.90 (mc, 2H)

1.30 g (2.94 mMol) dieser Zwischenverbindung werden in 40 ml Methanol mit 20 mg 5%igem Pd/C Katalysator versetzt und 10 h bei Raumtemperatur und einem Druck von 5 bar katalytisch hydriert. Filtration und Abdestillieren des Lösungsmittels ergibt 1.040 g (99%) der gewünschten Zielverbindung I-a-39 in Form gelbstichiger Kristalle mit Fp. 60 °C

### Beispiel I-a-42

### 2-(2,6-Diethyl-4-methyl-phenyl)-5-[3-methoxyiminobutyl]-tetrahydro-pentalen-1,3-dion

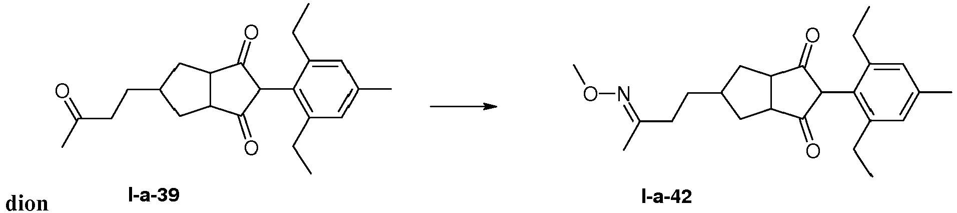

267 mg (0.82 mMol) 2-(2,6-Diethyl-4-methyl-phenyl)-5-(3-oxo-butyl)-tetrahydro-pentalene-1,3-dion (Beispiel I-a-39), 102 mg (1.23 mMol) O-Methylhydroxylamin-Hydrochlorid und 0.5 ml Triethylamin werden in 10 ml Acetonitril bei Raumtemperatur über Nacht gerührt. Danach wird in Ethylacetat aufgenommen, mit Wasser gewaschen, getrocknet (Magnesiumsulfat) und das Lösemittel abdestilliert. Chromatographie an Kieselgel (Laufmittel Essigsäure-ethylester/Hexan 1:4) ergibt 225 mg (77%) der Zielverbindung in Form farbloser Kristalle mit Fp. 79-80 °C.

### Beispiel I-a-40

### 2-(2,6-Diethyl-4-methyl-phenyl)-5-methoxymethoxymethyl-tetrahydropentalene-1,3-dion

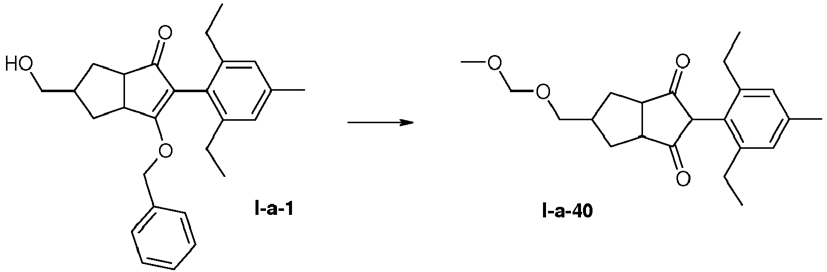

200 mg (0.49 mMol) der Verbindung aus Beispiel I-a-1, 1.12 g (14.8 mMol) Dimethoxyethan werden zusammen mit 10 mg Scandium-trifluormethansulfonat in 10 ml Trichlormethan 8 h am Rückfluß erhitzt. Der Ansatz wird mit Wasser verdünnt, abgetrennt und die organische Phase mit Wasser und 0.5 N Natronlauge gewaschen, getrocknet (Magnesiumsulfat) und einrotiert. Nach Chromatographie an Kieselgel (Laufmittel Essigsäure-ethylester/Hexan 1:5) erhält man 130 mg (59%) 3-Benzyloxy-2-(2,6-diethyl-4-methylphenyl)-5-methoxymethoxymethyl-4,5,6,6a-tetrahydro-3aH-pentalen-1-on als farbloses Öl.

¹H-NMR (400 MHz, CDCl₃): δ = 1.10 (mc, 6H), 2.30 (s, 3H), 3.15 (mc, 1H), 3.35 (s, 3H), 4.60 (s, 2H), 4.85 (s, 2H), 6.89 (mc, 2H)

123 mg (274 mMol) dieser Verbindung werden, wie bei Beispiel I-a-29 beschrieben, katalytisch hydriert. Man erhält 95 mg (96%) der Titelverbindung in Form farbloser Kristalle mit Fp. 155-156 °C.

### Beispiel I-a-77

### 2-(2,6-Diethyl-4-methylphenyl)-5-{[(4,5-dimethyl-1,3-thiazol-2-yl)oxy]methyl}tetrahydropentalen-1,3(2H,3aH)-dion

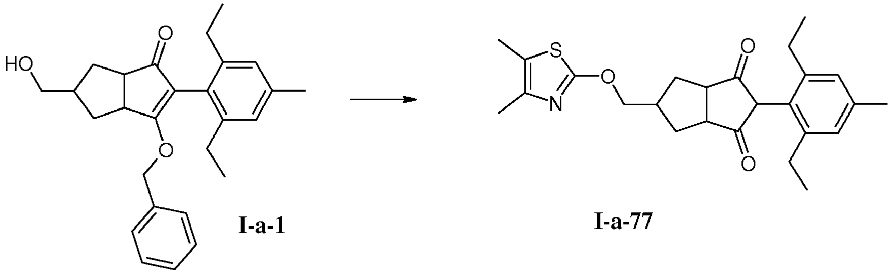

0.83 g (2.05 mMol) der Verbindung I-a-1 und 0.123 g (3.07 mMol) Natriumhydrid werden in 30 ml THF 30 Minuten am Rückfluß erhitzt. Anschließend werden bei Raumtemperatur 0.30 g (2 mMol) 2-Chlor-4,5-Dimethyl-1,3-thiazol zugetropft und 2 h am Rückfluß erhitzt. Nach Abkühlen werden vorsichtig 5 ml 2N Salzsäure zugegeben, in Essigsäureethylester aufgenommen, mit Wasser gewaschen, getrocknet (Magnesiumsulfat) und einrotiert. Chromatographie an Kieselgel (Laufmittel Essigsäureethylester/Hexan 1:6) liefert 0.31 g (30%) 3-Benzyloxy-2-(2,6-diethyl-4-methylphenyl)-5-(4,5-dimethyl-thiazol-2-yloxymethyl)-4,5,6,6a-tetrahydro-3aH-pentalen-1-one als farbloses Öl.

¹H-NMR (400 MHz, CDCl₃): δ = 1.10 (mc, 6H), 2.13, 2.20 und 2.32 (je s, je 3H), 3.15 und 3.40 (je mc, je 1H), 4.25-4.35 (m, 2H), 4.82 (s, 2H), 4.60 (s, 2H), 6.90 und 7.12 (je mc, je 2H), 7.30 (mc, 3H)

0,170 g (0.33 mMol) dieser Verbindung werden, wie in Beispiel I-a-29 beschrieben, katalytisch hydriert (Methanol, 5%iges Pd/C, 5 bar). Man erhält so 0.140 g (99%) der gewünschten Titelverbindung I-a-77 in Form farbloser Kristalle mit Fp. 73-74 °C

¹H-NMR (400 MHz, CDCl₃): δ = 1.05 (mc, 6H), 2.11, 2.19 und 2.31 (je s, je 3H), 2.62 (mc, 1H), 3.15-3.32 (m, 2H), 4.27 (d, 2H), 6.91 (mc, 2H)

### Beispiel I-a-83

### 2-[5-(2,6-Diethyl-4-methyl-phenyl)-4,6-dioxo-hexahydro-pentalen-2-ylidene]-malononitrile

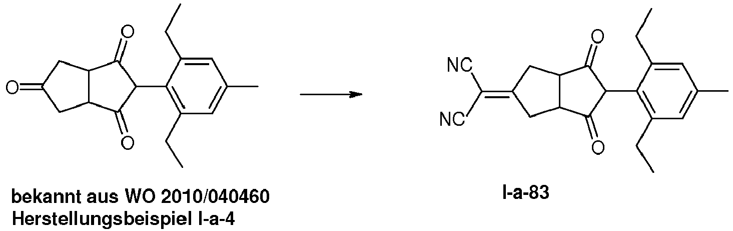

0.500 g (1.70 mmol) 2-(2,6-Diethyl-4-methyl-phenyl)-tetrahydro-pentalene-1,3,5-trion (Beispiel I-a-4 aus WO 2010/040460), 250 mg Malonsäuredinitril, 150 mg Ammoniumacetat und 0.3 ml Eisessig werden in 30 ml Toluol 2 h am Rückfluß erhitzt. Nach Abkühlen wird das Lösungsmittel abdestilliert, in Essigsäureethylester aufgenommen und zweimal mit Wasser gewaschen, anschließend getrocknet (Magnesiumsulfat) und einrotiert. Es verbleibt ein gelbes Öl, das an Kieselgel mit Essigsäureethylester/Hexan 1:6) chromatographiert wird. Man erhält 0.32 g (56%) farbloses Öl.

¹H-NMR (400 MHz, CDCl₃): δ = 1.02 und 1.08 (je t, je 3H), 2.21 und 2.38 (je mc, je 2H), 2.31 (s, 3H), 3.12 und 3.30 (je mc, je 2H), 3.40 und 3.58 (je mc, je 1H), 6.95 (mc, 2H).

### Beispiel I-a-35

### 2-[(3aR,6aS)-5-(2,6-Diethyl-4-methyl-phenyl)-4,6-dioxooctahydropentalen-2-yl]-malononitril

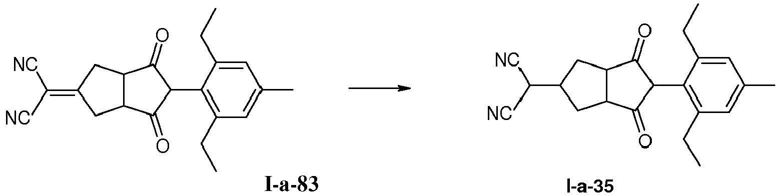

Zu 230 mg (0.66 mmol) 2-[5-(2,6-Diethyl-4-methyl-phenyl)-4,6-dioxo-hexahydro-pentalen-2-ylidene]-malononitril in einem Gemisch aus 10 ml Methanol und 10 ml Tetrahydrofuran werden 0.75 g (1.98 mmol) Natriumboranat gegeben und 2 h bei Raumtemp. gerührt. Das Lösemittel wird abdestilliert, in Ethylacetat aufgenommen, mit Wasser gewaschen, getrocknet (Magnesiumsulfat) und einrotiert. Nach chromatographischer Aufreinigung des Rückstandes erhält man 140 mg (60%) der gewünschten Verbindung in Form farbloser Kristalle mit Fp. 116-117°C.

¹H-NMR (400 MHz, CDCl₃): δ = 1.06 (mc, 6H), 1.61 (mc 2H), 2.31 (s, 3H), 2.33 (mc, 4H), 2.50 (mc, 2H), 2.77 (mc, 1H), 3.82 (d, 1H), 6.95 (mc, 2H).

### Beispiel I-a-84

### Cyano-[5-(2,6-diethyl-4-methyl-phenyl)-4,6-dioxohexahydropentalen-(2Z)-ylidene]-essigsäureethylester

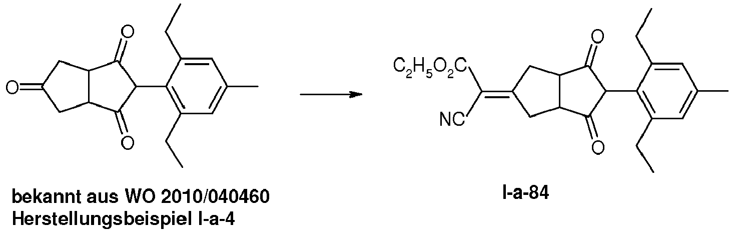

3.37 g (11.29 mmol) 2-(2,6-Diethyl-4-methyl-phenyl)-tetrahydro-pentalene-1,3,5-trion, 2.55 g (22.59 mmol) Cyanessigsäureethylester, 870 mg (11.3 mmol) Ammoniumacetat und 2.5 ml Eisessig in 70 ml Toluol werden analog zu Beispiel I-a-82 umgesetzt. Man erhält nach Chromatographie an Kieselgel (Essigsäureethylester/Hexan 1:6) 0.99 g (22%) der gewünschten Verbindung als farbloses Öl.

¹H-NMR (400 MHz, CDCl₃): δ = 0.94 und 1.08 (je t, je 3H), 1.32 (t, 3H), 2.20 (mc, 2H), 2.30 (s, 3H), 2.36 (mc, 2H), 3.10-3.20 (mc, 2H), 4.28 (q, 2H), 6.90 (mc, 2H).

In Analogie zu den Beispielen (I-a-1) bis (I-a-5) und gemäß den allgemeinen Angaben zur Herstellung erhält man folgende Verbindungen der Formel (I-a):

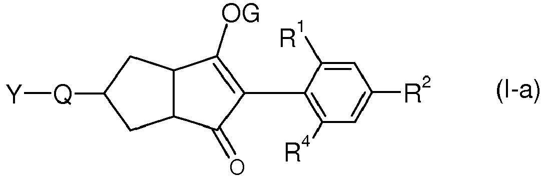

| **Bsp.- Nr.** | **R1** | **R2** | **R4** | **G** | **Y-Q** | **Fp. [°C] oder 1H-NMR (400 MHz, CDCl3, δ in ppm)** | **Bemerkung** |
|---|---|---|---|---|---|---|---|
| I-a-6 | C₂H₅ | CH₃ | C₂H₅ | H | 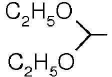 | δ = 1.07 und 1.20 (je mc, je 6H), 3.18 (mc, 2H), 3.50 und 3.65 (je mc, je 2H), 4.27 und 4.31 (je d, ∑ 1H), 6.92 (mc, 2H) | syn-/anti-Gemisch |
| I-a-7 | C₂H₅ | CH₃ | C₂H₅ | H | HOCH₂- | δ = 1.05-1.10 (m, 6H), 3.10-3.22 (m, 1H), 3.39 (mc, 1H), 3.55-3.68 (m, 2H, CH₂OH), 6.94 (mc, 2H) | syn-Isomer |
| I-a-8 | C₂H₅ | CH₃ | C₂H₅ | H | HO₂C- | 124-125 °C | anti-Isomer |
| I-a-9 | C₂H₅ | CH₃ | C₂H₅ | H | CH₃O₂C- | 72-73 | anti-Isomer |
| I-a-10 | C₂H₅ | CH₃ | C₂H₅ | H | 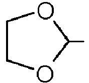 | 86-87 °C | anti-Isomer |
| I-a-11 | CH₃ | CH₃ | CH₃ | H | OHC- | 131-132 °C | syn-/anti-Gemisch |
| I-a-12 | C₂H₅ | CH₃ | C₂H₅ | H | 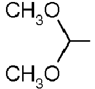 | 71-72 °C | anti-Isomer |
| I-a-13 | C₂H₅ | CH₃ | C₂H₅ | H | CH₃O-N=CH- | δ = 1.08 (t, 6H), 2.93 (mc, 1H), 3.35 (mc, 2H), 3.71 (s, 3H), 6.51 und 7.30 (je d, ∑ 1H) | E-/Z-Gemisch syn-/anti-Gemisch |
| I-a-14 | C₂H₅ | CH₃ | C₂H₅ | H | C₃H₅CH₂O-N=CH- | δ = 0.25 und 0.52 (je mc, je 2H), 2.95 (mc, 1H), 3.10-3.42 (s, br, 2H), 3.50 (mc, 1H), 3.82 und 3.88 (je d, ∑ 2H), 6.53 und 7.38 (je d, ∑ 1H) | E-/Z-Gemisch syn-/anti-Gemisch |
| I-a-15 | C₂H₅ | CH₃ | C₂H₅ | H | *ⁱ*PrO-N=CH- | 68-69 °C | E-/Z-Gemisch syn-/anti-Gemisch |
| I-a-16 | C₂H₅ | CH₃ | C₂H₅ | H | 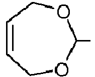 | δ = 1.03 (mc, 6H), 3.05 und 3.33 (je mc, je 1H), 4.10-4.18 und 4.33-4.39 (je m, je 2H), 4.52 und 4.59 (je d, je 1H), 5.70 (s, 2H) | syn-/anti-Gemisch |
| I-a-17 | C₂H₅ | CH₃ | C₂H₅ | H | Cl₂C=CHCH₂O-N=CH- | δ = 1.08 (t, 6H), 2.32 (s, 3H), 2.95 (mc, 1H), 4.58 und 4.66 (je d, ∑ 2H), 6.10 (mc, 1H), 6.60 und 7.37 (je d, ∑ 1H) | E-/Z-Gemisch syn-/anti-Gemisch |
| I-a-18 | C₂H₅ | CH₃ | C₂H₅ | H | HC≡CCH₂O-N=CH- | 2.43 und 2.46 (je t, ∑ 1H), 4.60 und 4.65 (je mc, ∑ 2H), 6.62 und 7.38 (je d, ∑ 1H) | E-/Z-Gemisch syn-/anti-Gemisch |
| I-a-19 | C₂H₅ | CH₃ | C₂H₅ | H | 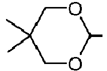 | δ = 0.70 und 1.15 (je s, je 3H), 1.08 (mc, 6H), 1.50 und 2.20 (je mc, je 2H), 3.38 und 3.55 (je d, je 2H), 4.24 (d, 1H), | anti-Isomer |
| I-a-20 | C₂H₅ | CH₃ | C₂H₅ | H | 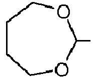 | δ = 1.05 (mc, 6H), 1.15 (s, 9H), 2.02 und 3.30 (je mc, je 1H), 3.60 (mc, 3H), 4.08 (mc, 1H), 4.45 und 4.50 (je d, ∑ 1H), 6.92 (mc, 2H) | syn-/anti-Gemisch |
| I-a-21 | C₂H₅ | CH₃ | C₂H₅ | H | HO-N=CH- | δ = 1.08 (mc, 6H), 2.32 (s, 3H), 3.20 und 3.41 (je mc, je 1H), 6.64 und 7.40 (je d, ∑ 1H), 6.95 (mc, 2H) | E-/Z-Gemisch syn-/anti-Gemisch |
| I-a-22 | C₂H₅ | CH₃ | C₂H₅ | H | CH₃O | δ = 1.05-1.10 (m, 6H), 2.30 (s, 3H), 3.05-3.30 (s, breit, 2H), 3.18 (s, 3H), 3.87 (mc, 1H). | syn-Isomer |
| I-a-23 | C₂H₅ | CH₃ | C₂H₅ | H | OH | 114-115 °C | syn-Isomer |
| I-a-24 | C₂H₅ | CH₃ | C₂H₅ | H | 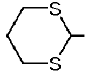 | 127-128 °C | syn-/anti-Gemisch |
| I-a-25 | C₂H₅ | CH₃ | C₂H₅ | H | 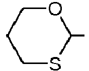 | δ = 1.05 (t, 6H), 2.32 (s, 3H), 3.59 (mc, 1H), 4.14 (mc, 1H), 4.66 und 4.70 (je d, ∑ 1H), 6.91 (s, 2H) | syn-/anti-Gemisch |
| I-a-26 | C₂H₅ | CH₃ | C₂H₅ | H | 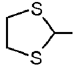 | 103-104°C | syn-/anti-Gemisch |
| I-a-27 | CH₃ | CH₃ | CH₃ | H | CH₃O-N=CH- | 204-205 °C | E-/Z-Gemisch syn-/anti-Gemisch |
| I-a-28 | C₂H₅ | CH₃ | C₂H₅ | H | CH₃(C=O)OCH₂- | 101-103 °C | syn-Isomer |
| I-a-29 | C₂H₅ | CH₃ | C₂H₅ | H | CH₃OCH₂- | 159-160 °C | syn-Isomer |
| I-a-30 | CH₃ | CH₃ | CH₃ | H | 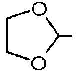 | 97-98 °C | syn-/anti-Gemisch |
| I-a-31 | CH₃ | CH₃ | CH₃ | H | iPrO-N=CH- | δ =1.18 und 1.20 (je d, je 3H), 2.05 (s, 6H), 2.28 (s, 3H), 4.25 (mc, 1H), 6.51 und 7.30 (je d, ∑ 1H), 6.88 (mc, 2H) | E-/Z-Gemisch syn-/anti-Gemisch |
| I-a-32 | CH₃ | CH₃ | CH₃ | H | 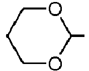 | 135-136°C | syn-/anti-Gemisch |
| I-a-33 | C₂H₅ | CH₃ | C₂H₅ | H | CH₃C=O- | 90-91 °C | syn-/anti-Gemisch |
| I-a-34 | C₂H₅ | CH₃ | C₂H₅ | H | CH₃C(=NOCH₃)- | 195-196 °C | E-/Z-Gemisch syn-/anti-Gemisch |
| I-a-35 | C₂H₅ | CH₃ | C₂H₅ | H | (NC)₂CH- | 116-117°C | syn-/anti-Gemisch |
| I-a-36 | C₂H₅ | CH₃ | C₂H₅ | H | -CH₂CH₂CO₂CH₃ | 59-60 °C | syn-/anti-Gemisch |
| I-a-37 | C₂H₅ | CH₃ | C₂H₅ | H | -CH₂CH₂CN | 142-145 °C | syn-/anti-Gemisch |
| I-a-38 | C₂H₅ | CH₃ | C₂H₅ | H | 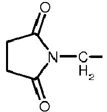 | 142-145 °C | syn-/anti-Gemisch |
| I-a-39 | C₂H₅ | CH₃ | C₂H₅ | H | CH₃(C=O)CH₂CH₂- | δ = 1.08 (mc, 6H), 2.12 (s, 3H), 2.30 (s, 3H), 3.28 (mc, 2H), 6.92 (mc, 2H) | syn-/anti-Gemisch |
| I-a-40 | C₂H₅ | CH₃ | C₂H₅ | H | CH₃OCH₂OCH₂- | δ = 1.08 (mc, 6H), 2.31 (s, 3H), 3.23 (mc, 2H), 3,34 (s, 3H), 3,50 (d, 2H), 4.60 (s, 2H), 6.92 (mc, 2H) | syn-/anti-Gemisch |
| I-a-41 | C₂H₅ | CH₃ | C₂H₅ | H | CH₃CH(OH)- | 71-72 °C | syn-/anti-Gemisch Racemat |
| I-a-42 | C₂H₅ | CH₃ | C₂H₅ | H | CH₃C(=NOCH₃)CH₂CH₂- | 79-80 °C | E-/Z-Gemisch syn-/anti-Gemisch |
| I-a-43 | C₂H₅ | CH₃ | C₂H₅ | H | C₂H₅OCH₂- | δ = 1.08 (t, 6H), 1.19 (t, 3H), 3.15 (mc, 1H), 3,47 (mc, 3H), 3,48 (q, 2H), 6.92 (mc, 2H) | syn-/anti-Gemisch |
| I-a-44 | C₂H₅ | CH₃ | C₂H₅ | H | C₆H₅NH(C=O)OCH₂- | 101-102 °C | syn-/anti-Gemisch |
| I-a-45 | C₂H₅ | CH₃ | C₂H₅ | H | C₂H₅O₂C- | δ = 1.08 (mc, 6H), 1.25 (mc, 3H), 1.88 und 2.00 (je mc, ∑ 2H), 2.99 (mc, 1H), 4.12 (q, 2H), 6.90 (mc, 2H) | syn-/anti-Gemisch |
| I-a-46 | C₂H₅ | CH₃ | C₂H₅ | H | CH₃(C=O)SCH₂- | δ = 1.08 (t, 6H), 2.30 (s, 3H), 2.34 (s, 3H), 2.95, d, 2H), 3.22 (mc, 2H), 6.91 (mc, 2H) | syn-/anti-Gemisch |
| I-a-47 | C₂H₅ | CH₃ | C₂H₅ | H | C₆H₅CH₂NH(C=O)OCH₂- | 101-102 °C | syn-/anti-Gemisch |
| I-a-48 | C₂H₅ | CH₃ | C₂H₅ | H | CH₃SO₂CH₂- | 180-181 °C | syn-/anti-Gemisch |
| I-a-49 | C₂H₅ | CH₃ | C₂H₅ | H | NCCH₂- | 66-67 °C | syn-/anti-Gemisch |
| I-a-50 | C₂H₅ | CH₃ | C₂H₅ | H | H₂N(C=O)- | 161-162 °C | syn-/anti-Gemisch |
| I-a-51 | C₂H₅ | CH₃ | C₂H₅ | H | H₂N(C=O)OCH₂ | 90-91 °C | syn-/anti-Gemisch |
| I-a-52 | C₂H₅ | CH₃ | C₂H₅ | H | (CH₃)₂N(C=O)OCH₂- | 151-152 °C | syn-/anti-Gemisch |
| I-a-53 | C₂H₅ | CH₃ | C₂H₅ | H | CH₃NH(C=O)- | δ = 1.10 (mc, 6H), 2.32 (s, 3H), 2.82 (mc, 1H), 3.49 (d, 3H), 6.95 (mc, 2H) | syn-/anti-Gemisch |
| I-a-54 | C₂H₅ | CH₃ | C₂H₅ | H | 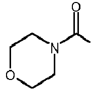 | 131-132 °C | syn-/anti-Gemisch |
| I-a-55 | C₂H₅ | CH₃ | C₂H₅ | H | (CH₃)₂N(C=O)- | 131-132 °C | syn-/anti-Gemisch |
| I-a-56 | C₂H₅ | CH₃ | C₂H₅ | Benzyl | -CHO | δ = 1.08 und 1.12 (je t, je 3H), 2.72 und 2.90 (je mc, ∑ 1H), 3.11 und 3.91 (je mc, je 1H), 4.71 und 4.77 (s und mc, ∑ 1H), 6.90 (mc, 2H), 7.10-7.35 (m, 5H) | syn-/anti-Gemisch |
| I-a-57 | C₂H₅ | CH₃ | C₂H₅ | Benzyl | CH₃C=O- | δ = 1.09 und 1.12 (je t, je 3H), 1.90 un 2.03 (je mc, je 1H), 2.80, 3.08 und 3.41 (je mc, je 1H), 4,78 und 4.80 (AB-System, J=12 Hz, 2H), | syn-/anti-Gemisch |
| I-a-58 | C₂H₅ | CH₃ | C₂H₅ | Benzyl | CH₃O₂CCH=CH- | 90-91 °C | syn-/anti-Gemisch E-/Z-Gemisch |
| I-a-59 | C₂H₅ | CH₃ | C₂H₅ | Benzyl | -CH=CHCN | δ = 1.10 (mc, 6H), 3.10-3.50 (m, 3H), 4.75-4.85 (m, 2H), 5.30 und 5.33, (je d, ∑ 1H), 6.88 und 6.70 (je d, ∑ 1H), 6.90 (mc, 2H) | syn-/anti-Gemisch E-/Z-Gemisch |
| I-a-60 | C₂H₅ | CH₃ | C₂H₅ | H | CH₃SCH₂- | δ = 1.08 (t, 6H), 2.10 (s, 3H), 2.29 (s, 3H), 2.20 -2.42 (m, 4H), 3.25 (mc, 2H), 6.90 (mc, 2H) | syn-/anti-Gemisch |
| I-a-61 | C₂H₅ | CH₃ | C₂H₅ | Benzyl | BrCH₂- | δ = 1.09 (mc, 6H), 1.51-1.70 (m, 2H), 3.02 (mc, 1H), 3.40 (mc, 2H), 3.48 (mc, 1H), 4.79 (mc, 2H), 6.90 (mc, 2H) | syn-/anti-Gemisch |
| I-a-62 | C₂H₅ | CH₃ | C₂H₅ | Benzyl | H₂N(C=O)OCH₂- | δ = 1.09 (mc, 6H), 1.38-1.65 (m, 2H), 3.12 und 3.40 (je mc, je 1H), 4.05 (mc, 2H), 4.60 (s, br), 4.80 und 4.82 (je mc, ∑ 2H), 6.90 (mc, 2H), 7.10-7.35 (m, 5H) | syn-/anti-Gemisch |
| I-a-63 | C₂H₅ | CH₃ | C₂H₅ | Benzyl | C₆H₅NH(C=O)OCH₂- | 60-61 °C | syn-/anti-Gemisch |
| I-a-64 | C₂H₅ | CH₃ | C₂H₅ | Benzyl | CF₃(C=O)OCH₂- | δ = 1.09 (mc, 6H), 1.38-1.62 (m, 2H), 2.33 (s, 3H), 3.10-3.70 (m, 3H), 4.80-4.91 (m, 2H), 6.90 (mc, 2H), 7.12-7.35 (m, 5H) | syn-/anti-Gemisch |
| I-a-65 | C₂H₅ | CH₃ | C₂H₅ | Benzyl | (CH₃)₂N(C=O)- | 111-112 °C | syn-/anti-Gemisch |
| I-a-66 | C₂H₅ | CH₃ | C₂H₅ | H | (CH₃)₃C(C=O)SCH₂- | δ = 1.08 (mc, 6H), 1.21 (s, 9H), 2.30 (s, 3H), 2.92 (d, 2H), 3.12 und 3.35 (je mc, je 1H), 6.93 (mc, 2H), | syn-/anti-Gemisch |
| I-a-67 | C₂H₅ | CH₃ | C₂H₅ | H | CF₃(C=O)OCH₂- | 214-215 °C | syn-/anti-Gemisch |
| I-a-68 | C₂H₅ | CH₃ | C₂H₅ | H | CF₃CH(OH)- | 185-186°C | syn-/anti-Gemisch |
| I-a-69 | C₂H₅ | CH₃ | C₂H₅ | Benzyl | CH₂=CH- | δ = 1.11 (mc, 6H), 1.40-1.69 (m, 2H), 3.00-3.15 (m, 1H), 3.38 (mc, 1H), 4.75-4.85 (m, 2H), 4.95-5.10 (m, 2H), 5.80 (mc, 1H), 6.90 (mc, 2H), 7.12-7.33 (m, 5H) | syn-/anti-Gemisch |
| I-a-70 | C₂H₅ | CH₃ | C₂H₅ | H | BrCH₂- | δ = 1.09 (mc, 6H), 1.40-1.55 (m, 2H), 2.30 (s, 3H), 2.60 (mc, 1H), 3.25 (mc, 1H), 3.48 (mc, 2H), 3.71 (mc, 1H), 6.90 (mc, 2H) | syn-/anti-Gemisch |
| I-a-71 | C₂H₅ | CH₃ | C₂H₅ | H | CH₂=CHCH₂-SCH₂- | δ = 1.07 (mc, 6H), 2.31 (s, 3H), 3.12 (d, 2H), 3.15-3.35 (m, 2H), 5.05-5.15 (mc, 2H), 5.55-5.62 (m, 1H), 6.90 (mc, 2H) | syn-/anti-Gemisch |
| I-a-72 | C₂H₅ | CH₃ | C₂H₅ | H | (CH₃)₂CHCH₂-SCH₂- | δ = 0.99 (mc, 6H), 1.07 (mc, 6H), 1.79 (hept, 1H), 2.40 (d, 2H), 2.55 (mc, 2H), 3.15-3.35 (m, 2H), 6.90 (mc, 2H) | syn-/anti-Gemisch |
| I-a-73 | C₂H₅ | CH₃ | C₂H₅ | H | 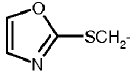 | δ = 1.06 (mc, 6H), 1.31-1.55 (m, 2H), 2.30 (s, 3H), 2.55 (mc, 1H), 3.15-3.55 (m, 3H), 6.90 (d, 1H), 6.92 (mc, 2H), 7.10 (d, 1H) | syn-/anti-Gemisch |
| I-a-74 | C₂H₅ | CH₃ | C₂H₅ | H | (CH₃)₂CHCH₂-SO₂CH₂- | δ = 1.07 (mc, 6H), 1.12 (d, 6H), 2.75 (mc, 1H), 2.88 (d, 2H), 3.00 (mc, 1H), 3.15 (mc, 1H), 3.38 (mc, 1H), 6.92 (mc, 2H) | syn-/anti-Gemisch |
| I-a-75 | C₂H₅ | CH₃ | C₂H₅ | Benzyl | CF₃CH(OH)- | 87-88 °C | syn-/anti-Gemisch |
| I-a-76 | C₂H₅ | CH₃ | C₂H₅ | H | 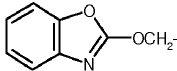 | δ = 1.05 (mc, 6H), 2.50-2.85 (m, ∑ 1H), 3.15-3.41 (m, 2H), 4.51, d, 2H), 6.93 (mc, 2H), 7.15-7.45 (m, 4H) | syn-/anti-Gemisch |
| I-a-77 | C₂H₅ | CH₃ | C₂H₅ | H | 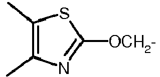 | 73-74 °C | syn-/anti-Gemisch |
| I-a-78 | C₂H₅ | CH₃ | C₂H₅ | H | NCSCH₂- | 87-88 °C | syn-/anti-Gemisch |
| I-a-79 | C₂H₅ | CH₃ | C₂H₅ | H | 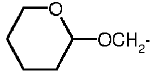 | 74-75 °C | syn-/anti-Gemisch |
| I-a-80 | C₂H₅ | CH₃ | C₂H₅ | H | 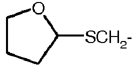 | δ = 1.05 (mc, 6H), 1.88 (mc, 2H), 2.05 (mc, 1H), 2.60-2.71 (m, 4H), 3.05-3.36 (m, 2H), 3.60-4.00 (m, 4H), 6.93 (mc, 2H) | syn-/anti-Gemisch |
| I-a-81 | C₂H₅ | CH₃ | C₂H₅ | C₂H₅ | -CH₂CO₂C₂H₅ | δ = 1.05 (mc, 6H), 1.22 (t, 3H), 2.51 (mc, 1H), 3.20 (mc, 2H), 4.12 (q, 2H), 6.91 (mc, 2H) | syn-/anti-Gemisch |
| I-a-82 | C₂H₅ | CH₃ | C₂H₅ | Benzyl | CH₃OCH₂- | | |

### Beispiel I-b-1

### 2-(2,6-Diethyl-4-methylphenyl)-5-(methoxyimino)methyl]-3-oxo-3,3a,4,5,6,6a-hexahydropentalen-1-yl-2,2-dimethylpropanoat

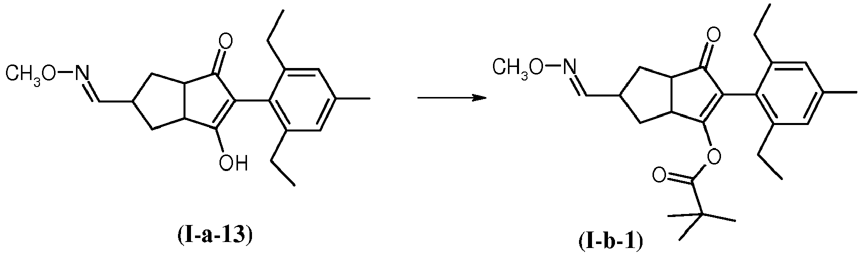

0.104 g (0.31 mmol) 5-(2,6-Diethyl-4-methylphenyl)-6-hydroxy-4-oxo-1,2,3,3a,4,6a-hexahydro-pentalen-2-carbaldehyd-O-methyloxim (erfindungsgemäße Verbindung I-a-13), 0.040 g (0.34 mmol) Pivaloylchlorid und 92 mg (0.91 mmol) Triethylamin in 8 ml Dichlormethan werden 2 h bei Raumtemperatur gerührt. Der Ansatz wird auf Eis gegeben, in Dichlormethan aufgenommen, mit Wasser gewaschen, getrocknet (Magnesiumsulfat) und einrotiert. Nach Chromatographie an Kieselgel (Laufmittel Essigsäureethylester/Hexan v:v= 25:75) erhält man 107 mg der Titelverbindung als farbloses Öl.

¹H-NMR (400 MHz, CDCl₃): δ = 1.04 und 1.09 (je t, je 3 H), 1.08 (s, 9 H), 2.98 (mc, 3 H), 3.30 (mc, 1H), 3.80 (s, 3H), 4.00 (mc, 1H), 6.88 (s, 1H), 7.31 (d, J = 7 Hz, -CH=NOCH₃).

In Analogie zu Beispiel (I-b-1) und gemäß den allgemeinen Angaben zur Herstellung erhält man folgende Verbindungen der Formel (I-b):

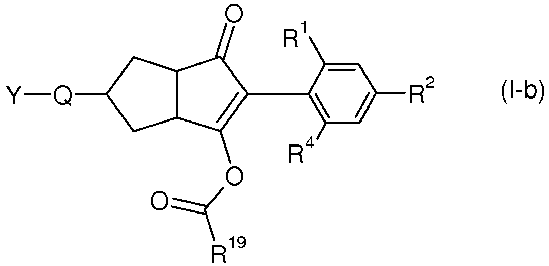

| **Bsp.- Nr.** | **R¹** | **R²** | **R⁴** | **R¹⁹** | **Y-Q** | **Fp. [°C] oder ¹H-NMR (400 MHz, CDCl₃, δ in ppm)** | **Bemerkung** |
|---|---|---|---|---|---|---|---|
| I-b-2 | C₂H₅ | CH₃ | C₂H₅ | tBu | -CHO | δ = 1.10 (s, 9H), 2.98, 3.37 und 4.04 (je mc, je 1H), 9.66 (d, 1H, CHO) | syn-/anti-Gemisch |
| I-b-3 | C₂H₅ | CH₃ | C₂H₅ | tBu | 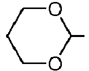 | 127 °C | syn-/anti-Gemisch |
| I-b-4 | C₂H₅ | CH₃ | C₂H₅ | tBu | 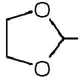 | δ = 1.05 (s, 9H), 1.42-1.58 (m, 3H), 3.22 (mc, 1H), 3.81-3.98 (m, 5H), 4.75 (d, 1H) | syn-/anti-Gemisch |
| I-b-5 | C₂H₅ | CH₃ | C₂H₅ | tBu | 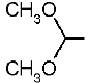 | δ = 1.05 (s, 9H), 1.33-1.50 (m, 2H), 3.34 und 3.35 (je s, ∑ 3H), 3.95 (mc, 1H), 4.18 (d, 1H), 6.86 (s, 2H) | syn-/anti-Gemisch |
| I-b-6 | C₂H₅ | CH₃ | C₂H₅ | tBu | C₃H₅CH₂O-N=CH- | δ = 0.25 und 0.52 (je mc, je 2H), 1.08 (s, 9H), 3.28 (mc, 1H), 3.82 und 3.89 (je d, ∑2H, CH₂C₃H₅), 4.00 (mc, 1H), 6.59 und 7.38 (je d, ∑2H, CH=NO) | E-/Z-Gemisch syn-/anti-Gemisch |
| I-b-7 | C₂H₅ | CH₃ | C₂H₅ | tBu | Cl₂C=CHCH₂O-N=CH- | δ = 1.04 (s, 9H), 2.30 (s, 3H), 3.28 und 4.02 (je mc, je 1H), 4.60 und 4.67 (je d, ∑ 2H), 6.10 (mc, 1H), 6.88 (s, 2H) | E-/Z-Gemisch syn-/anti-Gemisch |
| I-b-8 | C₂H₅ | CH₃ | C₂H₅ | tBu | CH₂=CHCH₂O-N=CH- | δ = 1.05 (s, 9H), 3.28 und 4.00 (je mc, je 1H), 4.50-4.61 (m, 2H), 5.18-5.32 (m, 2H), 5.96 (mc, 1H), 6.51 und 7.39 (je d, ∑ 1H) | E-/Z-Gemisch syn-/anti-Gemisch |
| I-b-9 | C₂H₅ | CH₃ | C₂H₅ | tBu | HC≡CCH₂O-N=CH- | δ = 1.00-1.11 (m, 15H), 2.45 (mc, 1H), 3.00 und 3.52 (je mc, ∑ 1H), 3.28 und 4.00 (je mc, je 1H), 4.60 und 4.67 (je d, ∑ 2H), 6.70 und 7.40 (je d, ∑ 1H) | E-/Z-Gemisch syn-/anti-Gemisch |
| I-b-10 | C₂H₅ | CH₃ | C₂H₅ | iPr | 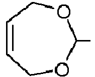 | δ = 1.00-1.11 (m, 12H), 2.56 (hept, 1H), 3.15 und 3.92 (je mc, je 1H), 4.18 und 4.40 (je mc, je 2H), 4.55 und 4.58 (je d, ∑ 1H) | syn-/anti-Gemisch |
| I-b-11 | C₂H₅ | CH₃ | C₂H₅ | tBu | 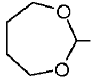 | δ = 1.00 (t, 6H), 1.15 (s, 9H), 2.28 (s, 3H), 3.61 und 4.09 (je s, je 2H), 4.48 und 4.51 (je d, ∑ 1H) | syn-/anti-Gemisch |
| I-b-12 | C₂H₅ | CH₃ | C₂H₅ | tBu | 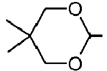 | 99-100°C | syn-/anti-Gemisch |
| I-b-13 | C₂H₅ | CH₃ | C₂H₅ | iPr | CH₃O-N=CH- | δ = 1.00-1.12 (m, 12H), 2.31 (s, 3H), 3.81 und 3.87 (je s, ∑ 1H), 4.00 (mc, 1H), 6.59 und 7.32 (je s, ∑ 1H), | E-/Z-Gemisch syn-/anti-Gemisch |
| I-b-14 | C₂H₅ | CH₃ | C₂H₅ | C₂H₅ | CH₃O-N=CH- | δ = 1.02-1.11 (m, 9H), 2.33 (s, 3H), 3.18-3.30 (m, 1H), 3.80 und 3.86 (je s, ∑ 1H), 3.98-4.08 (m, 1H), 6.59 und 7.31 (je s, ∑ 1H), 6.90 (s, 2H) | E-/Z-Gemisch syn-/anti-Gemisch |
| I-b-15 | C₂H₅ | CH₃ | C₂H₅ | iPr | 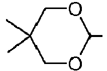 | δ = 0.70 und 1.12 (je s, je 3H), 1.02 und 1.08 (je mc, je 6H), 2.57 (hept, 1H), 3,40 und 3.58 (je mc, je 2H), 4.30 und 4.35 (je d, ∑ 1H), 6.89 (mc, 2H) | syn-/anti-Gemisch |
| I-b-16 | C₂H₅ | CH₃ | C₂H₅ | iPr | 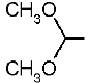 | δ = 1.02 und 1.08 (je mc, je 6H), 2.58 (hept, 1H), 3.33 (mc, 6H), 4.18 und 4.20 (je d, ∑ 1H), 6.99 (s, 2H) | syn-/anti-Gemisch |
| I-b-17 | C₂H₅ | CH₃ | C₂H₅ | C₂H₅ | 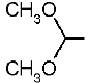 | δ = 1.03-1.12 (m, 9H), 2.31 (s, 3H), 2.52 (mc, 1H), 3.30-3.35 (m, 6H), 3.92-4.04 (m, 1H), 4.19 (mc, 1H), 6.90 (m, 2H) | syn-/anti-Gemisch |
| I-b-18 | C₂H₅ | CH₃ | C₂H₅ | tBu | 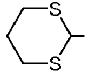 | δ = 1.00 (t, 6H), 1.21 (s, 9H), 2.28 (s, 3H), 3.21 und 3.92 (je mc, je 1H), 4.03 und 4.09 (je d, ∑ 1H), 6.85 (mc, 2H) | syn-/anti-Gemisch |
| I-b-19 | C₂H₅ | CH₃ | C₂H₅ | tBu | 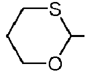 | δ = 1.00 und 1.08 (je t, ∑ 6H), 1.09 (s, 9H), 1.91 (mc, 2H), 3.05-3.19 (m, 1H), 3.35-4.47 (m, 1H), 4.12 (mc, 2H) | syn-/anti-Gemisch |
| I-b-20 | C₂H₅ | CH₃ | C₂H₅ | iPr | 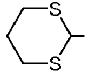 | 133-134°C | syn-/anti-Gemisch |
| I-b-21 | C₂H₅ | CH₃ | C₂H₅ | iPr | 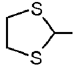 | δ = 1.00-1.11 (m, 9H), 2.52 (hept, 1H), 3.15-3-25 (m, 5H), 3.90-4.01 (m, 1H), 4.48 und 4.50 (je d, ∑1H), 6.87 (s, 2H) | syn-/anti-Gemisch |
| I-b-22 | C₂H₅ | CH₃ | C₂H₅ | tBu | 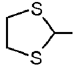 | δ = 1.03 (t, 6H), 1.06 (s, 9H), 3.15-3.26 (m, 5H), 3.90-4.00 (m, 1H), 4.48-4.51 (m, 1H), 6.86 (s, 2H) | syn-/anti-Gemisch |
| I-b-23 | C₂H₅ | CH₃ | C₂H₅ | iPr | CH₃(C=O)OCH₂- | δ = 1.03 (mc, 6H), 1.10 (mc, 6H), 2.05 (s, 3H), 2.30 (s, 3H), 3.15-3.29 (m, 1H), 3.93-4.10 (m, 3H), 6.89 (s, 2H) | syn-/anti-Gemisch |
| I-b-24 | C₂H₅ | CH₃ | C₂H₅ | tBu | BrCH₂- | 106-107 °C | syn-/anti-Gemisch |
| I-b-25 | C₂H₅ | CH₃ | C₂H₅ | tBu | 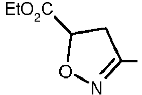 | δ = 1.04 (mc, 6H), 1.07 (s, 9H), 1.30 (t, 3H), 3.12 (mc, 1H), 3.21 (d, 2H), 3.30 (mc, 1H), 4.03 (mc, 1H), 4.23 (q, 2H), 6.88 (mc, 2H) | syn-/anti-Gemisch |
| I-a-26 | C₂H₅ | CH₃ | C₂H₅ | iPr | C₆H₅NH(C=O)OCH₂- | δ = 1.00-1.11 (mc, 12H), 1.30-1.62 (m, 2H), 2.59 (mc, 2H), 3.26 (mc, 1H), 3.95-4.04 (m, 1H), 4.16 (d, 2H), 6.89 (mc, 2H), 7.08 (mc, 1H), 7.25-7.40 (m, 4H) | syn-/anti-Gemisch |
| I-b-27 | C₂H₅ | CH₃ | C₂H₅ | iPr | C₆H₅CH₂NH(C=O)OCH₂ - | δ = 1.01-1.11 (mc, 12H), 1.22-1.60 (m, 2H), 2.48-2.60 (m, 2H), 3.15-3.29 (m, 1H), 3.90-4.04 (m, 1H), 4.11 (d, 2H), 4.38 (mc, 2H), 6.89 (mc, 2H), 7.08 (mc, 1H), 7.25-7.40 (m, 4H) | syn-/anti-Gemisch |
| I-b-28 | C₂H₅ | CH₃ | C₂H₅ | tBu | 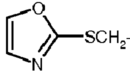 | δ = 1.05 (mc, 6H), 1.07 (s, 9H), 2.28 (s, 3H), 2.61 (mc, 1H), 3.20-3.33 (m, 3H), 6.89 (mc, 2H), 7.25-7.38 (m, 5H) | syn-/anti-Gemisch |

### Beispiel I-c-1

### 2-(2,6-Diethyl-4-methylphenyl)-5-(dimethoxymethyl)-3-oxo-3,3a,4,5,6,6a-hexahydropentalen-1-yl-ethylcarbonat

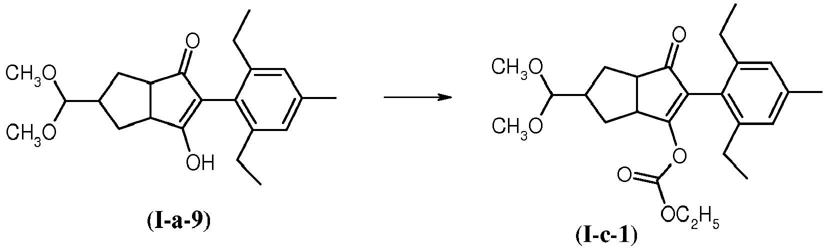

Zu 0.090 g (0.25 mmol) 2-(2,6-Diethyl-4-methylphenyl)-5-(dimethoxymethyl)-3-hydroxy-4,5,6,6a-tetrahydropentalen-1(3aH)-on (erfindungsgemäße Verbindung I-a-9) und 30 mg (0.28 mmol) Chlorameisensäureethylester in 15 ml Dichlormethan werden bei Raumtemperatur 80 mg (0.80 mmol) Triethylamin getropft und noch 1 h nachgerührt. Der Ansatz wird auf Eiswasser gegeben, in Dichlormethan aufgenommen, mit Wasser gewaschen, getrocknet (Magnesiumsulfat) und das Lösemittel abdestilliert.

Chromatographische Aufreinigung an Kieselgel (Essigsäureethylester/Hexan = 1:4) ergibt 88 mg (81%) der gewünschten Verbindung in Form farbloser Kristalle mit Schmelzpunkt 95-96 °C.

¹H-NMR (400 MHz, CDCl₃): δ=1.06, 1.10 und 1.25 (je t, je 3H), 1.40-1.50 (m, 2H), 2.18 (mc, 1H), 2.53 (mc, 1H), 3.21 (mc, 1H), 3.32 (s, 6H), 4.02 (mc, 1H), 4.18 (q, 2H), 6.90 (mc, 1H) ppm

In Analogie zu den Beispielen (I-c-1) und gemäß den allgemeinen Angaben zur Herstellung erhält man folgende Verbindungen der Formel (I-c):

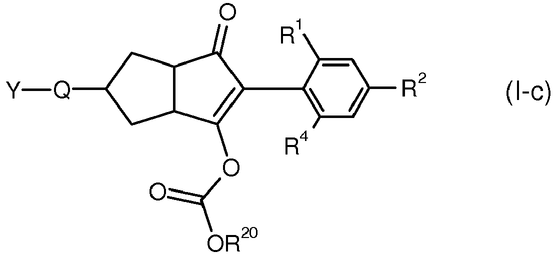

| **Bsp.- Nr.** | **R¹** | **R²** | **R⁴** | **R²⁰** | **Y-Q** | **Fp. [°C] oder ¹H-NMR (400MHz, CDCl₃, δ in ppm)** | **Bemerkung** |
|---|---|---|---|---|---|---|---|
| I-c-2 | C₂H₅ | CH₃ | C₂H₅ | C₂H₅ | 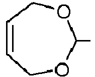 | δ = 1.05 (mc, 6H), 1.24 (t, 3H), 3.15 und 3.24 (mc, 1H), 4.00 (mc, 1H), 4.18 (mc, 4H), 4.33-4.43 (m, 2H), 4.55 und 4.58 (je d, ∑ 1H), 5.70 (s. 2H), 6.90 (s, 2H) | syn-/anti-Gemisch |
| I-c-3 | C₂H₅ | CH₃ | C₂H₅ | C₂H₅ | 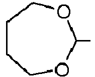 | δ = 1.07 (mc, 6H), 1.25 (t, 3H), 3.61 und 3.88 (je mc, je 2H), 4.00 (mc, 1H), 4.19 (mc, 2H) 4.48 und 4.51 (je d, ∑ 1H), 6.90 (s, 2H) | syn-/anti-Gemisch |
| I-c-4 | C₂H₅ | CH₃ | C₂H₅ | C₂H₅ | 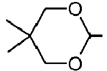 | δ = 0.70 (s, 3H), 1.08 (mc, 6H), 1.12 (s, 3H), 1.24 (t, 3H), 3.22 (mc, 1H), 3.40 und 3.58 (je d, je 2H), 4.18 (mc, 2H), 4-30 (d, 1H), 6.90 (mc, 2H) | syn-/anti-Gemisch |
| I-c-5 | C₂H₅ | CH₃ | C₂H₅ | C₂H₅ | *ⁱ*PrO-N=CH- | δ = 1.05-1.12 (m, 3H), 1.18-1.28 (m, 6H), 3.00 (mc, 1H), 4.00-4.32 (m. 4H), 6.58 und 7.31 (je d, ∑ 1H), | E-/Z-Gemisch syn-/anti-Gemisch |
| I-c-6 | C₂H₅ | CH₃ | C₂H₅ | C₂H₅ | Cl₂C=CHCH₂O-N=CH- | δ = 1.10 (mc, 6H), 1.25 (mc, 3H), 3.00 (mc, 1H), 4.05 (mc, 1H), 4.20 (mc, 2H), 4.60 und 4.66 (je d, ∑ 2H), 6.10 (mc, 1H), 6.63 und 7.45 (j d, ∑ 1H) | E-/Z-Gemisch syn-/anti-Gemisch |
| I-c-7 | C₂H₅ | CH₃ | C₂H₅ | C₂H₅ | C₃H₅CH₂O-N=CH- | δ = 0.25 und 0.53 (je mc, je 2H), 1.10 (mc, 6H), 1.24 (mc, 3H), 3.28 (mc, 1H), 3.33 und 3.39 (je d, ∑ 2H), 4.19 (mc, 2H), 6.60 und 7.38 (je d, ∑ 1H), 6.90 (mc, 2H) | E-/Z-Gemisch syn-/anti-Gemisch |
| I-c-8 | C₂H₅ | CH₃ | C₂H₅ | C₂H₅ | CH₂=CHCH₂O-N=CH- | δ = 1.10 (mc, 6H), 1.21 (mc, 3H), 4.05 (mc, 1H), 4.20 (mc, 2H), 4.50 und 4.50 (je d, ∑ 1H), 5.90-6.02 (m, 1H), 6.61 und 7.48 (je d, ∑ 1H) | E-/Z-Gemisch syn-/anti-Gemisch |
| I-c-9 | C₂H₅ | CH₃ | C₂H₅ | CH₃ | 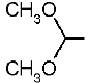 | δ = 1.05-1.10 (m, 6H), 2.31 (s, 3H), 3.15-3.28 (m, 1H), 3.35 (mc, 6H), 3.79 (s, 3H), 4.03 (mc, 1H), 4.18 und 4.20 (je d, ∑ 1H), | syn-/anti-Gemisch |
| I-c-10 | C₂H₅ | CH₃ | C₂H₅ | iPr | 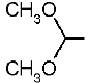 | δ = 1.05-1.12 (m, 6H), 1.21-1.27 (m, 6H), 3.30-3.35 (m, 6H), 4.02 (mc, 1H), 4.18 und 4,21 (je d, ∑ 1H), 4.82 (hept, 1H), 6.90 (mc, 2H) | syn-/anti-Gemisch |
| I-c-11 | C₂H₅ | CH₃ | C₂H₅ | C₂H₅ | 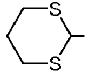 | δ = 1.25 (mc, 3H), 2.80-2.92 (m, 4H), 3.12-3.26 (m, 1H), 3.97-4.21 (m, 4H), 6.90 (s, 2H) | syn-/anti-Gemisch |
| I-c-12 | C₂H₅ | CH₃ | C₂H₅ | C₂H₅ | 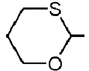 | δ = 1.08 (mc, 6H), 1.24 (mc, 3H), 2.75, 3.01, 3.58 (je mc, je 1H), 4.10-4.22 (m, 3H), 4.69 (mc, 1H), 6.89 (s, 2H) | syn-/anti-Gemisch |
| I-c-13 | C₂H₅ | CH₃ | C₂H₅ | C₂H₅ | 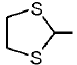 | δ = 1.24 (mc, 3H), 3.25 (mc, 5H), 4.00 (mc, 1H), 4.18 (mc, 2H), 4.48 und 4.50 (je d, ∑ 1H), 6.90 (mc, 2H) | syn-/anti-Gemisch |
| I-c-14 | C₂H₅ | CH₃ | C₂H₅ | iPr | CH₃O-N=CH- | δ = 1.09 (mc, 6H), 1.23 (mc, 6H), 1.48-1.64 (m, 2H), 3.28 (mc, 1H), 3.81 und 3.82 (je s, ∑ 3H), 4.05 (mc, 1H), 4.81 (mc, 1H), 6.60 unf 7.31 (je d, ∑ 1H), 6.90 (mc, 2H) | E-/Z-Gemisch syn-/anti-Gemisch |
| I-c-15 | C₂H₅ | CH₃ | C₂H₅ | C₂H₅ | CH₃(C=O)OCH₂- | δ = 1.08 (mc, 6H), 1.25 (mc, 3H), 1.31-1.45 (m, 2H), 2.05 und 2.06 (je s, ∑ 3H), 2.30 (s, 3H), 3.18-3.30 (m, 1H), 4.03 (mc, 3H), 4.20 (mc, 1H), 6.9 (mc, 2H) | syn-/anti-Gemisch |
| I-c-16 | C₂H₅ | CH₃ | C₂H₅ | C₂H₅ | -CH₂CO₂C₂H₅ | δ = 1.08 (mc, 6H), 1.22-1.35 (m, 6H), 2.28-2.45 (m, 8H), 2.30 (s, 3H), 2.85 (mc, 1H), 3.26 (mc, 1H), 4.03 (q, 1H), 4.14 (q, 2H), 4.18 (mc, 1H), 6.91 (mc, 2H) | syn-/anti-Gemisch |
| I-c-17 | C₂H₅ | CH₃ | C₂H₅ | C₂H₅ | CH₃C=O- | δ = 1.06 (mc, 6H), 1.23 (mc, 3H), 2.00 (mc, 2H), 2.20 (s, 3H), 2.31 (s, 3H), 2.82 (mc, 1H), 3.22 (mc, 1H), 4.03 (mc, 1H), 4.18 (mc, 1H), 6.92 (mc, 2H) | syn-/anti-Gemisch |
| I-c-18 | C₂H₅ | CH₃ | C₂H₅ | C₂H₅ | CH₃C(=NOCH₃)- | δ = 1.09 (mc, 6H), 1.25 (t, 3H), 1.81 (s, 3H) 1.93 (mc, 2H), 2.09 (mc, 1H), 2.59 (mc, 1H), 3.20 (mc, 1H), 3.81 (s, 3H), 1.71 (mc, 2H), 2.31 (s, 3H), 3.20 (mc, 1H), 3.81 (s, 3H), 4.03 (mc, 1H), 4.18 (mc, 2H) | E-/Z-Gemisch syn-/anti-Gemisch |
| I-c-19 | C₂H₅ | CH₃ | C₂H₅ | C₂H₅ | -CH₂CH₂CO₂CH₃ | δ = 1.08 (mc, 6H), 1.23 (mc, 3H), 1.71 (mc, 2H), 2.31 (s, 3H), 3.12-3.25 (m, 1H), 3.66 und 3.67 (je s, ∑ 3H), 3.97 (mc, 1H), 4.19 (mc, 2H), 6.90 (mc, 2H) | syn-/anti-Gemisch |
| I-c-20 | C₂H₅ | CH₃ | C₂H₅ | iPr | -CH₂CH₂CO₂CH₃ | δ = 1.07 (mc, 6H), 1.09 (mc, 3H), 1.70 (mc, 2H), 2.31 (s, 3H), 2.57 (hept, 1H), 3.10-3.25 (m, 1H), 3.67 und 3.68 (je s, ∑ 3H), 3.88-3.95 (m, 1H), 6.90 (mc, 2H) | syn-/anti-Gemisch |
| I-c-21 | C₂H₅ | CH₃ | C₂H₅ | C₂H₅ | -CH₂CH₂CN | δ = 1.08 (mc, 6H), 1.22 (mc, 3H), 2.33, 1.78 (mc, 2H), (s, 3H), 3.12-3.30 (m, 1H), 4.00 (mc, 1H), 4.18 (mc, 2H), 6.88 (mc, 2H) | syn-/anti-Gemisch |
| I-c-22 | C₂H₅ | CH₃ | C₂H₅ | C₂H₅ | -CH=CHCN | δ = 1.08 (mc, 6H), 1.24 (mc, 3H), 1.65-1.80 (m, 2H), 2.31 (s, 3H), 3.20-3.29 (m, 1H), 4.05 (mc, 1H), 4.18 (mc, 2H), 5.45 (J=13 Hz, 1H), 6.77 (dd, J= 13 und 5 Hz, 1H), 6.90 (mc, 2H) | syn-/anti-Gemisch E-Isomer |
| I-c-23 | C₂H₅ | CH₃ | C₂H₅ | C₂H₅ | CH₃(C=O)CH₂CH₂- | δ = 1.08 (mc, 6H), 1.25 (mc, 3H), 2.23 und 2.24 (je s, ∑ 3H), 2.31 (s, 3H), 3.10-3.23 (m, 1H), 3.95 (mc, 1H), 4.19 (mc, 2H), 6.90 (mc, 2H) | syn-/anti-Gemisch |
| I-c-24 | C₂H₅ | CH₃ | C₂H₅ | C₂H₅ | CH₃(C=NOCH₃)CH₂CH₂- | δ = 1.07 (mc, 6H), 1.26 (mc, 3H), 1.80 und 1.83 (je s, ∑ 3H), 3.22-3.25 (m, 1H), 3.30 und 3.32 (je s, ∑ 3H), 3.98 (mc, 1H), 4.20 (mc, 2H), 6.90 (mc, 2H) | E-/Z-Gemisch syn-/anti-Gemisch |
| I-c-25 | C₂H₅ | CH₃ | C₂H₅ | C₂H₅ | C₆H₅CH₂NH(C=O)OCH₂- | δ = 1.07 (mc, 6H), 1.25 (mc, 3H), 2.52 (mc, 1H), 3.15-3.29 (m, 1H), 4.03 (mc, 1H), 4.11 (mc, 2H), 4.19 (mc, 2H), 4.38 (mc, 2H), 6.90 (mc, 2H), 7.08 (mc, 1H), 7.24-7.38 (m, 5H) | syn-/anti-Gemisch |
| I-c-26 | C₂H₅ | CH₃ | C₂H₅ | C₂H₅ | C₆H₅NH(C=O)OCH₂- | δ = 1.08 (mc, 6H), 1.25 (t, 3H), 2.60 (mc, 1H), 3.18-3.31 (m, 1H), 4.05 (mc, 1H), 4.11-4.22 (m, 4H), 6.89 (mc, 2H), 7.08 (mc, 1H), 7.25-7.40 (m, 4H) | syn-/anti-Gemisch |
| I-c-27 | C₂H₅ | CH₃ | C₂H₅ | C₂H₅ | H₂N(C=O)- | 72-73 °C | syn-/anti-Gemisch |
| I-c-28 | C₂H₅ | CH₃ | C₂H₅ | C₂H₅ | H₂N(C=O)OCH₂- | δ = 1.09 (mc, 6H), 1.26 (t, 3H), 2.55 (mc, 1H), 3.15-3.30 (m, 1H), 4.05 (mc, 1H), 4.07 (d, 2H), 4.20 (mc, 2H), 4.60 (s, br, 2H), 6.90 (mc, 2H) | syn-/anti-Gemisch |
| I-c-29 | C₂H₅ | CH₃ | C₂H₅ | C₂H₅ | (CH₃)₂N(C=O)- | δ = 1.10 (mc, 6H), 1.24 (t, 3H), 2.32 (s, 3H), 2.95 (s, 3H), 3.06 (s, 3H), 3.25 (mc, 2H), 4.00-4.25 (m, 3H), 6.90 (mc, 2H) | syn-/anti-Gemisch |
| I-c-30 | C₂H₅ | CH₃ | C₂H₅ | C₂H₅ | CH₂=CHCH₂-SCH₂- | δ = 1.09 (mc, 6H), 1.25 (t, 3H), 2.32 (s, 3H), 3.12 (d, 2H), 3.22 (mc, 1H), 4.00 (mc, 1H), 4.20 (mc, 2H), 4.90 (mc, 2H), 3.72-3.83 (m, 1H), 6.90 (mc, 2H) | syn-/anti-Gemisch |

### Beispiel 1

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

### Nachauflauf, 80g/ha

| Beispiel | ALOMY | AVEFA | ECHCG | LOLMU | SETVI | POLCO | VERPE |
|---|---|---|---|---|---|---|---|
| I-a-4 | 50 | 90 | 100 | 60 | 100 | | |
| I-a-5 | 100 | 100 | 100 | 100 | 100 | | |
| I-a-7 | 100 | 70 | 100 | 100 | 80 | | |
| I-a-9 | 100 | 90 | 100 | 100 | 100 | | |
| I-a-10 | 100 | 100 | 100 | 100 | 100 | | |
| I-a-12 | 100 | 100 | 100 | 100 | 100 | | |
| I-a-13 | 100 | 100 | 100 | 100 | 100 | | |
| I-a-22 | 100 | 100 | 100 | 100 | 100 | | |
| I-a-23 | 70 | 60 | 100 | 90 | 100 | | |
| I-a-30 | | | 90 | 90 | 90 | | |
| I-a-31 | 90 | 80 | 90 | 90 | 100 | | |
| I-a-32 | | | 80 | 90 | 90 | | |
| I-a-33 | 100 | 100 | 100 | 100 | 90 | | |
| I-a-34 | 100 | | 100 | 100 | 90 | | |
| I-a-37 | 100 | 100 | 100 | 100 | 100 | | 80 |
| I-a-38 | | 90 | 90 | 90 | 90 | | |
| I-a-39 | 100 | | 100 | 100 | 100 | | |
| I-a-40 | 100 | 90 | | 100 | 90 | 100 | |
| I-a-41 | 100 | 100 | 100 | 100 | 100 | | |
| I-a-42 | 90 | 100 | 100 | 100 | 100 | | |
| I-a-60 | 90 | 90 | 100 | 90 | 90 | | |
| I-a-61 | | | 90 | 90 | 90 | | |
| I-a-79 | 90 | 100 | 100 | 100 | 90 | | 100 |
| I-a-81 | 80 | | 80 | 80 | 90 | | |
| I-b-1 | 100 | 100 | 100 | 100 | 100 | | |
| I-b-2 | | | 100 | | 100 | | |
| I-b-3 | 100 | | 100 | 100 | | | |
| I-b-23 | 80 | 90 | 100 | 90 | 90 | | |
| I-b-4 | 100 | 90 | 100 | 100 | 100 | | |
| I-b-5 | 100 | 100 | 100 | 100 | 100 | | |
| I-c-14 | 100 | 100 | 100 | 100 | 100 | | |
| I-c-15 | | | 90 | 90 | 80 | | |
| I-c-16 | | | 80 | | 80 | | |
| I-c-17 | 90 | 90 | 90 | 100 | 80 | | |
| I-c-18 | 100 | 80 | 90 | 100 | | | |
| I-c-21 | | 100 | 100 | 100 | 90 | | |
| I-c-22 | 80 | 100 | 100 | | 100 | | 90 |
| I-c-24 | 90 | 80 | 100 | 100 | 100 | | |

### Nachauflauf, 100 g/ha

| Beispiel | APESV | AVEFA | PHAMI | POAAN | SETVI |
|---|---|---|---|---|---|
| I-c-1 | 100 | 80 | 99 | 85 | 97 |

### Vorauflauf, 320 g/ha

| Beispiel | ALOMY | AVEFA | ECHCG | LOLMU | SETVI | STEME | VERPE | VIOTR |
|---|---|---|---|---|---|---|---|---|
| I-a-4 | | | 100 | | 90 | | | |
| I-a-5 | 100 | 90 | | 100 | 90 | | | |
| I-a-7 | | 80 | 100 | 100 | | | | |
| I-a-9 | 100 | 80 | 100 | 100 | 90 | | | |
| I-a-10 | 100 | 90 | 100 | 100 | 100 | | | 90 |
| I-a-12 | 100 | 80 | 100 | 100 | | | | |
| I-a-13 | | 80 | | 100 | | 100 | 90 | 100 |
| I-a-22 | 100 | 90 | 100 | 100 | 100 | | | |
| I-a-23 | | | 100 | | 90 | | | |
| I-b-1 | 100 | 90 | 100 | 100 | 100 | | | 90 |
| I-b-2 | 80 | 80 | 100 | 100 | 90 | | | |
| I-b-3 | 100 | 90 | 100 | 100 | 100 | | | 90 |
| I-b-4 | 100 | 90 | 100 | 100 | 100 | | | 90 |
| I-b-5 | 100 | 100 | 100 | 100 | 90 | | | |

| | | | | | | | | |
|---|---|---|---|---|---|---|---|---|
| LOLMU = Lolium multiflorum ALOMY = Alopecurus myosuroides AVEFA = Avena fatua SETVI = Setaria viridis ECHCG = Echinochloa crus-galli STEME = Stellaria media APESV = Apera spica-venti PHAMI = Phalaris minor POAAN = Poa annua VERPE = Veronica persica VIOTR = Viola tricolor POLCO = Polygonum convolvulus | | | | | | | | |

### Beispiel 2

### Tetranychus-Test, OP-resistent (TETRUR Spritzbehandlung)

| | | |
|---|---|---|
| Lösungsmittel: | 78,0 | GewichtsteileAceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 80% bei einer Aufwandmenge von 100 g/ha: I-c-19, I-c-21

Bei diesem Test zeigen z. B. die folgenden Verbindungen der Herstellungsbeispiele eine Wirkung von 90% bei einer Aufwandmenge von 100 g/ha:
I-a-4, I-a-5, I-a-6, I-a-13, I-a-14, 1-a-15, I-a-20, I-a-30, I-a-57, I-a-40, I-a-47, I-a-71, I-a-72, I-a-74, I-b-7, I-b-23, I-b-24, I-b-27, I-c-2, I-c-15, I-c-16, I-c-25, I-c-29

Bei diesem Test zeigen z. B. die folgenden Verbindungen der Herstellungsbeispiele eine Wirkung von 100% bei einer Aufwandmenge von 100 g/ha:
I-a-8, I-a-32, I-b-6, I-b-8, I-b-9, I-b-10, I-c-5, I-c-14, I-c-30

### Beispiel 3

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele Wirkung von 100 % bei einer Aufwandmenge von 500 g/ha: I-a-44, I-a-47

### Beispiel 4

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele Wirkung von 100 % bei einer Aufwandmenge von 500 g/ha : I-a-59, I-c-22

## Patentansprüche

1. Verbindungen der Formel (I) 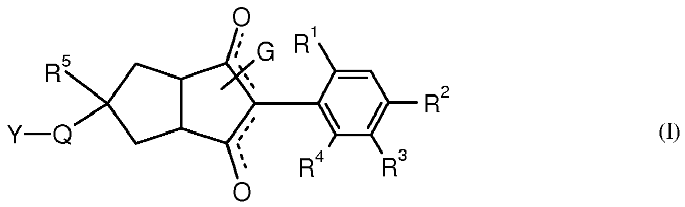 wobei folgende isomere Formen gebildet werden können 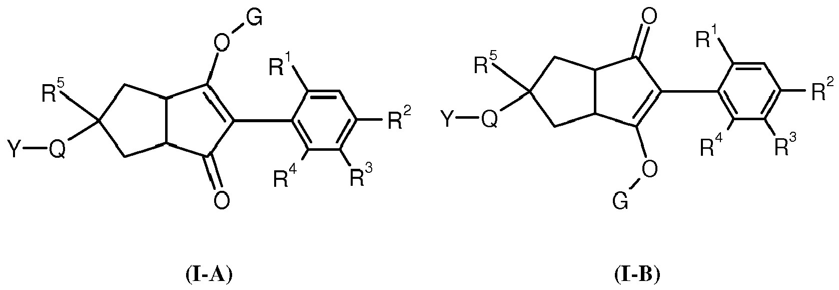 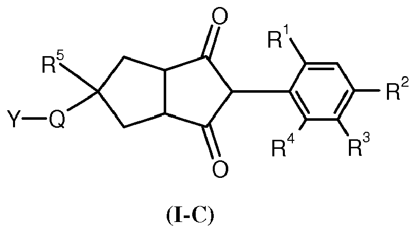 in welcher
wenn
Q, Y und R⁵ gemeinsam eine der Gruppen CHCN=, CH(CO₂C₁-C₆-Alkyl)=, 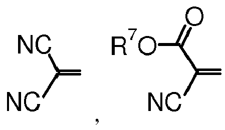 oder 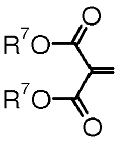 bilden, dann steht
G für Wasserstoff, Methyl, Ethyl oder Benzyl (a) oder für eine der Gruppen 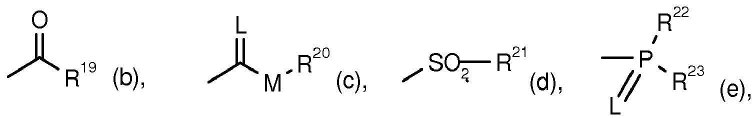
E (f) oder 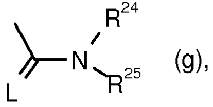
E für ein Metallionenäquivalent, ein tertiäres Sulfoniumion oder ein Ammoniumion,
L für Sauerstoff oder Schwefel,
M für Sauerstoff oder Schwefel,
R¹ für Wasserstoff, Halogen, Cyano, Nitro, C₁-C₆-Alkyl, Halogen-C₁-C₆-alkyl, C₃-C₆-Cycloalkyl, Halogen-C₃-C₆-cycloalkyl, C₂-C₆-Alkenyl, Halogen-C₂-C₆-alkenyl, C₂-C₆-Alkinyl, Halogen-C₂-C₆-alkinyl, C₁-C₆-Alkoxy, Halogen-C₁-C₆-alkoxy, C₃-C₆-Cycloalkoxy, Halogen-C₃-C₆-cycloalkoxy, C₂-C₆-Alkinyloxy, Halogen-C₂-C₆-alkinyloxy, C₂-C₆-Alkenyloxy, Halogen-C₂-C₆-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl oder Halogen-C₁-C₃-alkylsulfonyl,
R² und R³ unabhängig voneinander, gleich oder verschieden für Wasserstoff, Halogen, Cyano, Nitro, C₁-C₆-Alkyl, Halogen-C₁-C₆-alkyl, C₂-C₆-Alkenyl, Halogen-C₂-C₆-alkenyl, C₂-C₆-Alkinyl, Halogen-C₂-C₆-alkinyl, C₁-C₆-Alkoxy, Halogen-C₁-C₆-alkoxy, C₃-C₆-Cycloalkyl, C₃-C₆-Cycloalkoxy, Halogen-C₃-C₆-cycloalkoxy, C₂-C₆-Alkinyloxy, Halogen-C₂-C₆-alkinyloxy, C₂-C₆-Alkenyloxy, Halogen-C₂-C₆-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl, Halogen-C₁-C₃-alkylsulfonyl, Phenyl oder gegebenenfalls ein- oder mehrfach, gleich oder verschieden durch Halogen, C₁-C₄-Alkyl, Halogen-C₁-C₄-alkyl, C₃-C₆-Cycloalkyl, Halogen-C₃-C₆-cycloalkyl, C₁-C₆-Alkoxy, Halogen-C₁-C₆-alkoxy, Nitro oder Cyano substituiertes Phenyl,
R⁴ für Wasserstoff, Halogen, Cyano, Nitro, C₁-C₆-Alkyl, Halogen-C₁-C₆-alkyl, C₃-C₆-Cycloalkyl, Halogen-C₃-C₆-cycloalkyl, C₁-C₆-Alkoxy, Halogen-C₁-C₆-alkoxy, C₃-C₆-Cycloalkoxy, oder Halogen-C₃-C₆-cycloalkoxy,
R⁵ für Wasserstoff oder C₁-C₄-Alkyl,
R⁷ für Wasserstoff, für jeweils gegebenenfalls substituiertes C₁-C₆-Alkyl, C₃-C₆-Cycloalkyl, C₂-C₆-Alkenyl oder für ein Kation E,
R¹⁹ für gegebenenfalls durch Halogen substituiertes C₁-C₆-Alkyl, C₂-C₆-Alkenyl, C₁-C₄-Alkoxy-C₁-C₄-alkyl, C₁-C₃-Alkylthio-C₁-C₄-alkyl, Poly-C₁-C₄-alkoxy-C₁-C₄-alkyl oder gegebenenfalls ein- oder mehrfach, gleich oder verschieden durch Halogen, C₁-C₄-Alkyl oder C₁-C₄-Alkoxy substituiertes C₃-C₆-Cycloalkyl, das gegebenenfalls durch Sauerstoff oder Schwefel im Ring unterbrochen sein kann, jeweils gegebenenfalls mit Halogen oder C₁-C₄-Alkyl substituiertes Phenyl, Phenyl-C₁-C₄-alkyl, Hetaryl, Phenoxy-C₁-C₄-alkyl oder Hetaryloxy-C₁-C₄-alkyl,
R²⁰ für jeweils gegebenenfalls ein- oder mehrfach, gleich oder verschieden durch Halogen substituiertes C₁-C₆-Alkyl, C₂-C₆-Alkenyl, C₁-C₄-Alkoxy-C₁-C₄-alkyl, Poly-C₁-C₄-alkoxy C₁-C₄-alkyl oder für C₃-C₆-Cycloalkyl, das gegebenenfalls durch Sauerstoff oder Schwefel im Ring unterbrochen sein kann oder für Benzyl,
R²¹, R²² und R²³ unabhängig voneinander, gleich oder verschieden für jeweils gegebenenfalls ein- oder mehrfach, gleich oder verschieden durch Halogen substituiertes C₁-C₆-Alkyl, C₁-C₆-Alkoxy, C₁-C₆-Alkylamino, C₁-C₆-Dialkylamino, C₁-C₃Alkylthio, C₂-C₄-Alkenylthio, C₃-C₆-Cycloalkylthio oder für Phenyl, Benzyl, Phenoxy oder Phenylthio,
R²⁴ und R²⁵ unabhängig voneinander, gleich oder verschieden für Wasserstoff, jeweils gegebenenfalls ein-oder mehrfach, gleich oder verschieden durch Halogen substituiertes C₁-C₆-Alkyl, C₃-C₆-Cycloalkyl, C₂-C₆-Alkenyl, C₁-C₆-Alkoxy, C₁-C₆-AlkoxyC₁-C₄-alkyl, für gegebenenfalls ein-oder mehrfach, gleich oder verschieden durch Halogen oder C₁-C₄-Alkyl substituiertes Phenyl oder Benzyl oder
R²⁴ und R²⁵ bilden gemeinsam mit dem benachbarten Stickstoffatom eine Morpholino- Piperidino- oder Pyrrolidinogruppe,
oder wenn
Q, Y und R⁵ gemeinsam für die Gruppe CH₂= stehen, dann steht
G für Methyl, Ethyl oder Benzyl (a).

2. Verbindungen der Formel (I) gemäß Anspruch 1, in welcher
wenn
Q, Y und R⁵ gemeinsam eine der Gruppen CHCN=, CH(CO₂C₁-C₆-Alkyl)=, 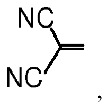 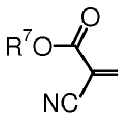 oder 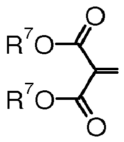 bilden, dann steht
G Wasserstoff, Methyl, Ethyl oder Benzyl (a) oder für eine der Gruppen 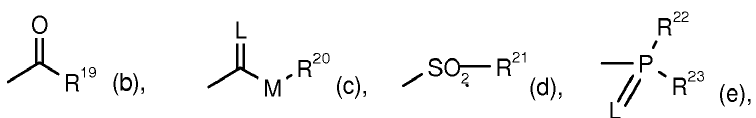
E (f) oder 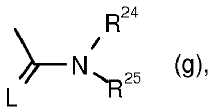
E für ein Metallionenäquivalent, ein tertiäres Sulfoniumion oder ein Ammoniumion,
L für Sauerstoff oder Schwefel,
M für Sauerstoff oder Schwefel,
R¹ für Wasserstoff, Halogen, C₁-C₄-Alkyl, Halogen-C₁-C₄-alkyl, C₃-C₆-Cycloalkyl, Halogen-C₃-C₆-cycloalkyl, C₂-C₄-Alkenyl, Halogen-C₂-C₄-alkenyl, C₂-C₄-Alkinyl, Halogen-C₂-C₄-alkinyl, C₁-C₄-Alkoxy, Halogen-C₁-C₄-alkoxy, C₂-C₄-Alkinyloxy, Halogen-C₂-C₄-alkinyloxy, C₂-C₄-Alkenyloxy, Halogen-C₂-C₄-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl oder Halogen-C₁-C₃-alkylsulfonyl,
R² und R³ unabhängig voneinander, gleich oder verschieden für Wasserstoff, Halogen, C₁-C₄-Alkyl, Halogen-C₁-C₄-alkyl, C₂-C₄-Alkenyl, Halogen-C₂-C₄-alkenyl, C₂-C₄-Alkinyl, Halogen-C₂-C₄-alkinyl, C₁-C₄-Alkoxy, Halogen-C₁-C₄-alkoxy, C₃-C₆-Cycloalkyl, C₃-C₆-Cycloalkoxy, Halogen-C₃-C₆-cycloalkoxy, C₂-C₄-Alkinyloxy, Halogen-C₂-C₄-alkinyloxy, C₂-C₄-Alkenyloxy, Halogen-C₂-C₄-alkenyloxy, C₁-C₃-Alkylthio, C₁-C₃-Alkylsulfinyl, C₁-C₃-Alkylsulfonyl, Halogen-C₁-C₃-alkylthio, Halogen-C₁-C₃-alkylsulfinyl, Halogen-C₁-C₃-alkylsulfonyl, Phenyl oder gegebenenfalls ein- oder mehrfach, gleich oder verschieden durch Halogen, C₁-C₄-Alkyl, Halogen-C₁-C₄-alkyl substituiertes Phenyl,
R⁴ für Wasserstoff, Halogen, C₁-C₄-Alkyl, Halogen-C₁-C₄-alkyl, C₃-C₆-Cycloalkyl, Halogen-C₃-C₆-cycloalkyl, C₁-C₄-Alkoxy, Halogen-C₁-C₄-alkoxy, C₃-C₄-Cycloalkoxy, oder Halogen-C₃-C₆-cycloalkoxy,
R⁵ für Wasserstoff oder C₁-C₄-Alkyl,
R⁷ für Wasserstoff, für jeweils gegebenenfalls ein-oder mehrfach, gleich oder verschieden durch Halogen oder C₁-C₄-Alkoxy substituiertes C₁-C₄-Alkyl, C₃-C₆-Cycloalkyl, C₂-C₄-Alkenyl oder für ein Kation E,
R¹⁹ für gegebenenfalls durch Halogen substituiertes C₁-C₆-Alkyl, C₂-C₆-Alkenyl, C₁-C₄-Alkoxy-C₁-C₄-alkyl, C₁-C₃-Alkylthio-C₁-C₄-alkyl, Poly-C₁-C₄-alkoxy-C₁-C₄-alkyl oder gegebenenfalls ein- oder mehrfach, gleich oder verschieden durch Halogen, C₁-C₄-Alkyl oder C₁-C₄-Alkoxy substituiertes C₃-C₆-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind, jeweils gegebenenfalls mit Halogen oder C₁-C₄-Alkyl substituiertes Phenyl, Phenyl-C₁-C₄-alkyl, Hetaryl, Phenoxy-C₁-C₄-alkyl oder HetaryloxyC₁-C₄-alkyl,
R²⁰ für jeweils gegebenenfalls ein- oder mehrfach, gleich oder verschieden durch Halogen substituiertes C₁-C₆-Alkyl, C₂-C₆-Alkenyl, C₁-C₄-Alkoxy C₁-C₄-alkyl, Poly-C₁-C₄-alkoxy C₁-C₄-alkyl oder für C₃-C₆-Cycloalkyl, in welchem gegebenenfalls ein oder mehrere nicht direkt benachbarte Ringglieder durch Sauerstoff und/oder Schwefel ersetzt sind, oder für Benzyl,
R²¹, R²² und R²³ unabhängig voneinander, gleich oder verschieden für jeweils gegebenenfalls ein- oder mehrfach, gleich oder verschieden durch Halogen substituiertes C₁-C₄-Alkyl, C₁-C₄-Alkoxy, C₁-C₄-Alkylamino, C₁-C₄-Dialkylamino, C₁-C₃-Alkylthio, C₂-C₄-Alkenylthio, C₃-C₆-Cycloalkylthio oder für jeweils gegebenenfalls ein-oder mehrfach, gleich oder verschieden durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl, Benzyl, Phenoxy oder Phenylthio,
R²⁴ und R²⁵ unabhängig voneinander, gleich oder verschieden für Wasserstoff, jeweils gegebenenfalls ein-oder mehrfach, gleich oder verschieden durch Halogen substituiertes C₁-C₆-Alkyl, C₃-C₆-Cycloalkyl, C₂-C₆-Alkenyl, C₁-C₆-Alkoxy, C₁-C₆-AlkoxyC₁-C₄-alkyl, für gegebenenfalls ein- oder mehrfach, gleich oder verschieden durch Halogen oder C₁-C₄-Alkyl substituiertes Phenyl oder Benzyl oder
R²⁴ und R²⁵ bilden gemeinsam mit dem benachbarten Stickstoffatom eine Morpholino- Piperidino- oder Pyrrolidinogruppe bilden,
oder wenn
Q, Y und R⁵ gemeinsam für die Gruppen CH₂= stehen,
dann steht
G für Methyl, Ethyl oder Benzyl (a).

3. Verbindungen der Formel (I) gemäß Anspruch 1, in welcher
wenn
Q, Y und R⁵ gemeinsam eine der Gruppen 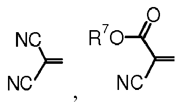 oder 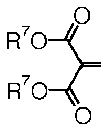 bilden, dann steht
G für Wasserstoff, Ethyl oder Benzyl oder für eine der Gruppen 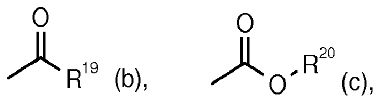
R¹ für Methyl, Ethyl, Methoxy, Ethoxy, Halogen oder Cyclopropyl,
R² für Methyl, Ethyl oder 4-Chlorphenyl,
R³ für Wasserstoff, Methyl, Ethyl oder Cyclopropyl,
R⁴ für Wasserstoff, Methyl oder Ethyl,
R⁵ für Wasserstoff,
R⁷ für Wasserstoff, Methyl, Ethyl, iso-Propyl oder n-Propyl,
R¹⁹ für C₁-C₄-Alkyl,
R²⁰ für Metyhl, Ethyl oder iso-Propyl,
oder wenn
Q, Y und R⁵ gemeinsam für die Gruppe CH₂= stehen,
dann steht
G für Methyl, Ethyl oder Benzyl (a).

4. Verbindungen der Formel (I) gemäß Anspruch 1, in welcher
wenn
Q, Y und R⁵ gemeinsam eine der Gruppen 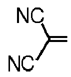 oder 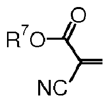 bilden, dann steht
G Wasserstoff, Ethyl, Benzyl (a) oder für eine der Gruppen 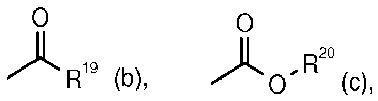
R¹ für Methyl oder Ethyl steht,
R² für Methyl steht,
R³ für Wasserstoff steht,
R⁴ für Methyl oder Ethyl steht,
R⁵ für Wasserstoff steht,
R⁷ für Wasserstoff, Methyl oder Ethyl steht,
R¹⁹ für Ethyl, tert.-Butyl oder iso-Propyl steht,
R²⁰ für Methyl, Ethyl oder iso-Propyl steht.

5. Verbindungen der Formel (I) gemäß Anspruch 1, in welcher
Q, Y und R⁵ gemeinsam für die Gruppe CH₂= stehen,
G für Benzyl (a) steht,
R¹ für Ethyl steht,
R² für Methyl steht,
R³ für Wasserstoff steht,
R⁴ für Ethyl steht.

6. Verfahren zur Herstellung von Verbindungen der Formel (I) gemäß Anspruch 1, **dadurch gekennzeichnet, dass** man zum Erhalt von
(A) Verbindungen der Formel (I-a) 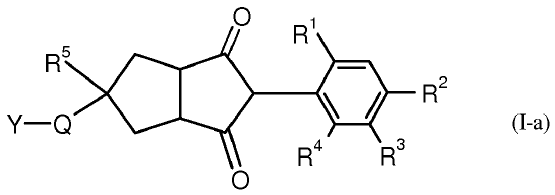 in welcher
Q, Y, R¹, R², R³, R⁴ und R⁵ die oben angegebene Bedeutung haben ,
Verbindungen der Formel (II) 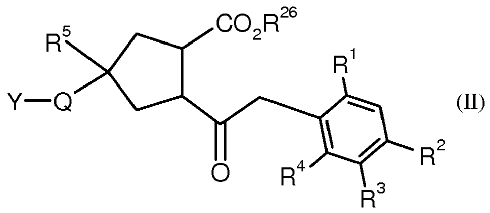 in welcher
Q, Y, R¹, R², R³, R⁴ und R⁵ die oben angegebene Bedeutung haben, und
R²⁶ für Alkyl steht,
gegebenenfalls in Gegenwart eines Verdünnungsmittels in Gegenwart einer Base intramolekular cyclisiert,
(B) Verbindungen der oben gezeigten Formel (I-b), in welcher G, Q, Y, R¹, R², R³, R⁴ und R⁵ die oben angegebene Bedeutung haben, Verbindungen der oben gezeigten Formel (I-a), in welcher Q, Y, R¹, R², R³, R⁴ und R⁵ die oben angegebene Bedeutung haben, jeweils
(α) mit Säurehalogeniden der Formel (III) 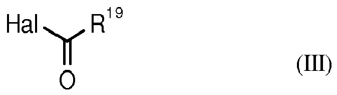 in welcher
R¹⁹ die oben angegebene Bedeutung hat und
Hal für Halogen steht
oder
(ß) mit Carbonsäureanhydriden der Formel (IV)
R¹⁹-CO-O-CO-R¹⁹ (IV)
in welcher
R¹⁹ die oben angegebene Bedeutung hat,
gegebenenfalls in Gegenwart eines Verdünnungsmittels und gegebenenfalls in Gegenwart eines Säurebindemittels umsetzt,
(C) Verbindungen der oben gezeigten Formel (I-c), in welcher Q, Y, R¹, R², R³, R⁴ und R⁵ die oben angegebene Bedeutung haben haben und L für Sauerstoff steht, Verbindungen der oben gezeigten Formel (I-a), in welcher Q, Y, R¹, R², R³, R⁴ und R⁵ die oben angegebene Bedeutung haben, jeweils
mit Chlorameisensäureestern oder Chlorameisensäurethioestern der Formel (V)
R²⁰-M-CO-Cl (V)
in welcher
R²⁰ und M die oben angegebenen Bedeutungen haben,
gegebenenfalls in Gegenwart eines Verdünnungsmittels und gegebenenfalls in Gegenwart eines Säurebindemittels umsetzt;
(D) Verbindungen der oben gezeigten Formel (I-c), in welcher Q, Y, R¹, R², R³, R⁴ und R⁵ die oben angegebene Bedeutung haben haben und L für Schwefel steht, Verbindungen der oben gezeigten Formel (I-a), in welchen Q, Y, R¹, R², R³, R⁴ und R⁵ die oben angegebene Bedeutung haben, jeweils
mit Chlormonothioameisensäureestern oder Chlordithioameisensäureestern der Formel (VI) 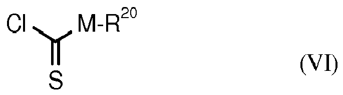 in welcher
M und R²⁰ die oben angegebenen Bedeutungen haben,
gegebenenfalls in Gegenwart eines Verdünnungsmittels und gegebenenfalls in Gegenwart eines Säurebindemittels umsetzt,
(E) Verbindungen der oben gezeigten Formel (I-d), in welcher Q, Y, R¹, R², R³, R⁴ und R⁵ die oben angegebene Bedeutung haben, Verbindungen der oben gezeigten Formel (I-a), in welcher Q, Y, R¹, R², R³, R⁴ und R⁵ die oben angegebene Bedeutung haben, jeweils mit Sulfonsäurechloriden der Formel (VII)
R²¹-SO₂-Cl (VII)
in welcher
R²¹ die oben angegebene Bedeutung hat,
gegebenenfalls in Gegenwart eines Verdünnungsmittels und gegebenenfalls in Gegenwart eines Säurebindemittels umsetzt,
(F) Verbindungen der oben gezeigten Formel (I-e), in welcher Q, Y, R¹, R², R³, R⁴ und R⁵ die oben angegebene Bedeutung haben, Verbindungen der oben gezeigten Formel (I-a), in welchen Q, Y, R¹, R², R³, R⁴ und R⁵ die oben angegebene Bedeutung haben, jeweils
mit Phosphorverbindungen der Formel (VIII) 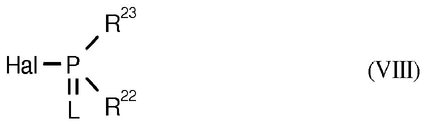 in welcher
L, R²² und R²³ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(G) Verbindungen der oben gezeigten Formel (I-f), in welcher Q, Y, R¹, R², R³, R⁴ und R⁵ die oben angegebene Bedeutung haben, Verbindungen der Formel (I-a), in welcher Q, Y, R¹, R², R³, R⁴ und R⁵ die oben angegebene Bedeutung haben, jeweils
mit Metallverbindungen oder Aminen der Formeln (IX) oder (X) 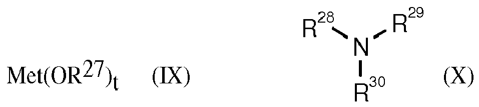 in welchen
Met für ein ein- oder zweiwertiges Metall,
t für die Zahl 1 oder 2 und
R²⁷, R²⁸, R²⁹ und R³⁰ unabhängig voneinander für Wasserstoff oder Alkyl stehen,
gegebenenfalls in Gegenwart eines Verdünnungsmittels umsetzt,
(H) Verbindungen der oben gezeigten Formel (I-g), in welcher Q, Y, R¹, R², R³, R⁴ R⁵ und R²⁴ die oben angegebene Bedeutung haben und R²⁵ für Wasserstoff steht, Verbindungen der oben gezeigten Formel (I-a), in welcher Q, Y, R¹, R², R³, R⁴ und R⁵ die oben angegebene Bedeutung haben, jeweils
(α) mit Isocyanaten oder Isothiocyanaten der Formel (XI)
R²⁴-N=C=L (XI)
in welcher
R²⁴ und L die oben angegebenen Bedeutungen haben,
egebenenfalls in Gegenwart eines Verdünnungsmittels und gegebenenfalls in Gegenwart eines Katalysators umsetzt oder
(ß) mit Carbamidsäurechloriden oder Thiocarbamidsäurechloriden der Formel (XII) 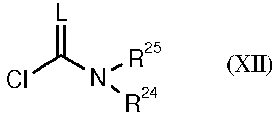 in welcher
L, R²⁴ und R²⁵ die oben angegebenen Bedeutungen haben,
gegebenenfalls in Gegenwart eines Verdünnungsmittels und gegebenenfalls in Gegenwart eines Säurebindemittels, umsetzt.

7. Mittel zur Bekämpfung von Schädlingen und/oder unerwünschten Pflanzenwuchs, **gekennzeichnet durch** einen Gehalt an mindestens einer Verbindung der Formel (I) gemäß Anspruch 1.

8. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge, unerwünschtem Pflanzenwuchs und/oder ihren Lebensraum einwirken lässt.

9. Verwendung von Verbindungen der Formel (I) gemäß Anspruch 1 zur Bekämpfung von tierischen Schädlingen und/oder unerwünschtem Pflanzenbewuchs.

10. Verfahren zur Herstellung von Mitteln zur Bekämpfung von Schädlingen und/oder unerwünschtem Pflanzenbewuchs, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen und/oder unerwünschtem Pflanzenwuchs.

12. Mittel enthaltend einen wirksamen Gehalt an einer Wirkstoffkombination umfassend als Komponenten
(a') mindestens eine Verbindung der Formel (I) gemäß Anspruch, in welcher R¹, R², R³, R⁴, R⁵, Y, Q und G die oben angegebene Bedeutung haben
und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14, S15, S16.

13. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

14. Verwendung eines Mittels gemäß Anspruch 12 zum Bekämpfen von unerwünschten Pflanzenwuchs.

15. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

16. Verbindungen der Formel (II) 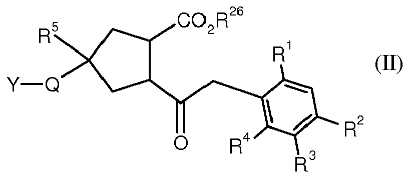 in welcher
Q, Y, R¹, R², R³, R⁴ und R⁵ die in Anspruch 1 angegebene Bedeutungen haben, und
R²⁶ für Alkyl steht.

17. Verbindungen der Formel (XIII) 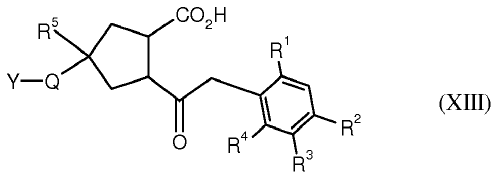 in welcher
Q, Y, R¹, R², R³, R⁴ und R⁵ die in Anspruch 1 angegebene Bedeutungen haben.

18. Verbindungen der Formel (XIV) 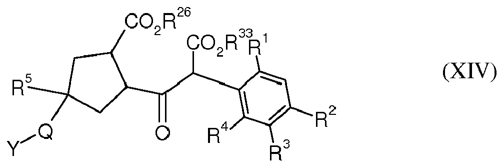 in welcher Q, Y, R¹, R², R³, R⁴ und R⁵ die in Anspruch 1 angegebene Bedeutungen haben,
R²⁶ für Alkyl oder Wasserstoff steht,
R³³ für Alkyl steht.
